(19) **Europäisches Patentamt European Patent Office Office européen des brevets**

(11) **EP 4 671 277 A1**

(12) **EUROPEAN PATENT APPLICATION**
published in accordance with Art. 153(4) EPC

(43) Date of publication:
**31.12.2025 Bulletin 2026/01**

(21) Application number: **24759779.2**

(22) Date of filing: **23.02.2024**

(51) International Patent Classification (IPC):
*C07K 16/28* (2006.01)    *C07K 16/40* (2006.01)
*C07K 16/46* (2006.01)    *A61P 35/00* (2006.01)

(52) Cooperative Patent Classification (CPC):
**A61P 35/00; C07K 16/28; C07K 16/40; C07K 16/46**

(86) International application number:
**PCT/CN2024/078357**

(87) International publication number:
**WO 2024/175105 (29.08.2024 Gazette 2024/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.02.2023 CN 202310174524**

(71) Applicant: **Shanghai Mabgen Biotech Ltd.**
**Shanghai 201206 (CN)**

(72) Inventors:
• **WU, Hui**
 **Shanghai 201206 (CN)**
• **YAN, Yijie**
 **Shanghai 201206 (CN)**
• **ZHAO, Zhilong**
 **Shanghai 201206 (CN)**
• **LI, Lei**
 **Shanghai 201206 (CN)**

(74) Representative: **Regimbeau**
 **20, rue de Chazelles**
 **75847 Paris Cedex 17 (FR)**

Remarks:
The complete document including Reference Table(s) and the Sequence Listing(s) can be downloaded from the EPO website

(54) **PHARMACEUTICAL COMPOSITION OF FAP/CD40 BINDING MOLECULE AND PHARMACEUTICAL USE THEREOF**

(57) A pharmaceutical composition of a FAP/CD40 binding molecule and the pharmaceutical use thereof. Specifically, the pharmaceutical composition contains a FAP/CD40 binding molecule and a buffer agent. The pharmaceutical composition has a good biological activity and stability.

EP 4 671 277 A1

## Description

The present disclosure claims priority to Chinese Patent Application No. 202310174524.1, filed on Feb. 24, 2023, which is incorporated herein by reference in its entirety.

## TECHNICAL FIELD

The present disclosure relates to the field of pharmaceutical formulations, in particular to a pharmaceutical composition comprising a FAP/CD40-binding molecule and pharmaceutical use thereof.

## BACKGROUND

CD40 (TNFRSF5), a transmembrane phosphorylated glycoprotein, is a member of the tumor necrosis factor receptor superfamily (TNFRS). CD40 is expressed in many types of cells, including B cells, follicular dendritic cells (DCs), epithelial cells, monocytes, macrophages, smooth muscle cells, and tumor cells. Its ligand CD40L is mainly expressed in activated T cells, activated B cells, platelets, smooth muscle cells, etc. CD40L binding multimerizes CD40, generating downstream activation, growth, and differentiation signals. CD40 signaling activates a variety of downstream signaling pathways, such as NF-κB, MAPK, and STAT3 (Pype S, et al. J Biol Chem. 2000 Jun. 16; 275(24):1858693), and these pathways regulate gene expression by regulating the activator protein c-Jun, ATF2, and Rel transcription factors. Binding of CD40 to CD40L induces resting B cell proliferation, immunoglobulin switching, and antibody secretion, and plays important roles in the development of germinal centers in tissues and B cell survival. All of these are essential for humoral immune responses (Kehry M R. J Immunol 1996; 156: 2345-2348). CD40L binds to the CD40 on DCs, inducing DC maturation, which is manifested in up-regulated expression of the B7 family of costimulatory factors (CD80 and CD86) and increased secretion of proinflammatory cytokines such as interleukin 12 (IL-12). The interaction between CD40 and CD40L provides a costimulatory signal for T cell activation and promotes DCs' presentation of antigens to T cells.

With the clinical success of immune checkpoint inhibitor (ICI) therapies targeting CD40, CTLA-4, and PD-L1 in tumor treatment, immunotherapy has become the most anticipated research field among third-generation tumor therapies. Anti-tumor immunotherapy based on the mechanism that DC cells' antigen presentation to and activation of T cells are increased by activating CD40 has been shown to be clinically effective. However, there is systemic activation of CD40, which can lead to some target-specific toxicities such as cytokine storms, hepatotoxicity, hematotoxicity, and venous thrombosis caused by peripheral CD40 activation. These toxicities limit the therapeutic windows of CD40 agonist antibodies. The clinical studies on Pfizer's CD40 agonists CP-870 and 893 and Medimmune's CD40L-Fc fusion protein MEDI5083 (a novel fusion protein from AstraZeneca that can activate the CD40 signaling pathway, consisting of 3 CD40L molecules connected in tandem and IgG4-Fc) have been terminated. Thus, in the research and development of second-generation CD40 agonist antibodies, researchers aim to mediate CD40 activation through tumor-associated antigens (TAAs), such that CD40 is specifically activated within tumors while peripheral CD40 activation is reduced, thereby increasing the therapeutic windows of CD40 agonist antibodies.

Fibroblast activation protein (FAP) α is a tumor-associated antigen. FAP is lowly expressed in normal tissues of healthy adults and is selectively expressed in 93% of tumor tissues, with 30% being high expression, such as colon cancer, pancreatic cancer, breast cancer, gastric cancer, prostate cancer, bladder cancer, and oral squamous cell carcinoma. WO2023025194 provides an anti-FAP antibody, an anti-CD40 antibody, and a bispecific antibody of the two. The bispecific antibody can mediate tumor-specific CD40 activation through FAP, has maturation-promoting and activating effects on APCs (e.g., dendritic cells (DCs)), can eliminate hepatotoxicity, peripheral hematotoxicity, and other peripheral toxicities, and has an excellent administration window and druggability.

Due to their great molecular weights and complex structures, antibody drugs are prone to degradation, aggregation, unwanted chemical modifications, etc., which make them unstable. It is particularly important to develop stable formulations of antibody drugs that make the antibodies more suitable for administration, keep the antibodies stable during storage and subsequent use, and make the antibodies produce better effects. For a novel FAP/CD40-binding molecule, there is still a need to develop a suitable pharmaceutical composition.

## SUMMARY

The present disclosure provides a pharmaceutical composition comprising a FAP/CD40-binding molecule, and the composition has excellent stability.

The present disclosure provides a pharmaceutical composition comprising a FAP/CD40-binding molecule and a buffer, wherein the buffer is selected from the group consisting of an acetate buffer, a succinate buffer, a histidine salt buffer, and a phosphate buffer. In some embodiments, the buffer is a histidine salt buffer. In some embodiments, the buffer is a histidine-hydrochloride buffer. In some embodiments, the buffer is a histidine-acetate buffer. In some embodiments, the buffer is a histidine-histidine hydrochloride buffer. In some embodiments, the buffer is a histidine-acetic acid buffer.

In some embodiments, the FAP/CD40-binding molecule comprises a first antigen-binding domain that specifically binds to FAP and a second antigen-binding domain that specifically binds to CD40, wherein the second antigen-binding domain that specifically binds to CD40 comprises (at least one) immunoglobulin single variable domain.

In some embodiments, the FAP/CD40-binding molecule comprises one immunoglobulin single variable domain that specifically binds to CD40. In some other embodiments, the FAP/CD40-binding molecule comprises 2, 3, 4, 5, 6, 7, 8, 9, 10, or more immunoglobulin single variable domains that specifically bind to CD40, and the immunoglobulin single variable domains may be identical or different.

In some embodiments, the FAP/CD40-binding molecule comprises at least one (e.g., 2, 3, or 4) antigen-binding domain that specifically binds to FAP.

In some embodiments, in the FAP/CD40-binding molecule, the immunoglobulin single variable domain that specifically binds to CD40 comprises three complementarity-determining regions CDR1, CDR2, and CDR3, wherein the amino acid sequences of the CDR1, CDR2, and CDR3 are set forth in SEQ ID NOs: 12, 13, and 14, respectively.

In some embodiments, in the aforementioned FAP/CD40-binding molecule, the immunoglobulin single variable domain that specifically binds to CD40 comprises or is set forth in any one of SEQ ID NOs: 11 and 16 to 19, or has at least 90%, at least 95%, at least 98%, or at least 99% sequence identity to any one of SEQ ID NOs: 23 and 35 to 38. In some embodiments, the FAP/CD40-binding molecule comprises a first antigen-binding domain that specifically binds to FAP and a second antigen-binding domain that specifically binds to CD40, wherein the first antigen-binding domain that specifically binds to FAP comprises a heavy chain variable region and a light chain variable region; the heavy chain variable region comprises a HCDR1, a HCDR2, and a HCDR3, and the light chain variable region comprises a LCDR1, a LCDR2, and a LCDR3, wherein:

1) the amino acid sequences of the HCDR1, HCDR2, and HCDR3 are set forth in SEQ ID NOs: 3, 4, and 5, respectively; the amino acid sequences of the LCDR1, LCDR2, and LCDR3 are set forth in SEQ ID NOs: 6, 7, and 8, respectively; or
2) the amino acid sequences of the HCDR1, HCDR2, and HCDR3 are set forth in SEQ ID NOs: 3, 4, and 5, respectively; the amino acid sequences of the LCDR1, LCDR2, and LCDR3 are set forth in SEQ ID NOs: 6, 32, and 8, respectively.

In some embodiments, in the aforementioned FAP/CD40-binding molecule, the heavy chain variable region of the first antigen-binding domain that specifically binds to FAP comprises the amino acid sequence set forth in SEQ ID NO: 1 or an amino acid sequence having at least 80% identity thereto, and the light chain variable region comprises the amino acid sequence set forth in SEQ ID NO: 2 or an amino acid sequence having at least 80% identity thereto.

In some embodiments, the FAP/CD40-binding molecule further comprises a human immunoglobulin Fc region (e.g., an Fc region of human IgG1 or IgG4).

In some embodiments, in the aforementioned FAP/CD40-binding molecule, the first antigen-binding domain that specifically binds to FAP comprises a heavy chain (HC) and a light chain (LC);
for example, the heavy chain is of the IgG1 or IgG4 isotype, and the light chain is of the Kappa isotype.

In some specific embodiments, the heavy chain is the amino acid sequence set forth in SEQ ID NO: 24 or an amino acid sequence having at least 80% identity thereto, and the light chain is the amino acid sequence set forth in SEQ ID NO: 25 or an amino acid sequence having at least 80% identity thereto.

In some embodiments, in the aforementioned FAP/CD40-binding molecule, the first antigen-binding domain that specifically binds to FAP comprises a heavy chain variable region and a light chain variable region, wherein:

the immunoglobulin single variable domain of the second antigen-binding domain that specifically binds to CD40 is located at the N-terminus of the heavy chain variable region of the first antigen-binding domain that specifically binds to FAP;
the immunoglobulin single variable domain of the second antigen-binding domain that specifically binds to CD40 is located at the C-terminus of the heavy chain variable region of the first antigen-binding domain that specifically binds to FAP;
the immunoglobulin single variable domain of the second antigen-binding domain that specifically binds to CD40 is located at the N-terminus of the light chain variable region of the first antigen-binding domain that specifically binds to FAP; and/or
the immunoglobulin single variable domain of the second antigen-binding domain that specifically binds to CD40 is located at the C-terminus of the light chain variable region of the first antigen-binding domain that specifically binds to FAP.

In some embodiments, the immunoglobulin single variable domain of the aforementioned second antigen-binding domain that specifically binds to CD40 is linked, either directly or by a linker, to the first antigen-binding domain that specifically binds to FAP; for example, the linker has an amino acid sequence represented by $(G_4S)_x$, wherein x is independently selected from the group consisting of integers of 1-20 (e.g., 1, 2, 3, 4, 5, 6, 7, 8, 9, or 10); for example, the linker is an amino acid sequence represented by $(G_4S)_2$, $(G_4S)_3$, or $(G_4S)_4$.

In some embodiments, in the aforementioned FAP/CD40-binding molecule, the second antigen-binding domain that specifically binds to CD40 is multivalent (for example, one of the aforementioned FAP/CD40-binding molecules comprises 1, 2, 3, 4, 5, 6, 7, 8, 9, or 10 second antigen-binding domains that specifically bind to CD40). In some specific embodiments, the second antigen-binding domain that specifically binds to CD40 is bivalent, tetravalent, or hexavalent. In some specific embodiments, the second antigen-binding domain that specifically binds to CD40 comprises 2, 3, 4, 5, or 6 said immunoglobulin single variable domains.

In some embodiments, the FAP/CD40-binding molecule comprises a first polypeptide chain and a second polypeptide chain, wherein the first polypeptide chain comprises the amino acid sequence set forth in any one of SEQ ID NOs: 21-23 or an amino acid sequence having at least 80% identity thereto, and the second polypeptide chain comprises the amino acid sequence set forth in SEQ ID NO: 20 or an amino acid sequence having at least 80% identity thereto. In some specific embodiments, the FAP/CD40-binding molecule comprises two first polypeptide chains and two second polypeptide chains; in some specific embodiments, the two first polypeptide chains are identical, and the two second polypeptide chains are identical.

In some embodiments, the aforementioned FAP/CD40-binding molecule is capable of inhibiting tumor growth (e.g., volume increase or tumor weight increase) and/or metastasis (e.g., multi-organ or multi-tissue metastasis or distant metastasis) by at least about 10%, e.g., at least about 20%, about 30%, about 40%, about 50%, about 60%, about 70%, about 80%, or about 90%.

In some embodiments, the aforementioned FAP/CD40-binding molecule is an anti-FAP/CD40 bispecific antibody or an antigen-binding fragment thereof, wherein the antigen-binding fragment includes, but is not limited to, Fab, Fv, sFv, Fab', F(ab')$_2$, linear antibodies, single-chain antibodies, scFv, sdAb, sdFv, nanobodies, peptibodies, domain antibodies, and multispecific antibodies (bispecific antibodies, diabodies, triabodies, tetrabodies, tandem di-scFv, and tandem tri-scFv), and is, for example, an scFv, Fv, Fab, or Fab' fragment.

In some embodiments, the anti-FAP/CD40 bispecific antibody comprises the immunoglobulin single variable domain in the aforementioned second antigen-binding domain that specifically binds to CD40, and the heavy chain variable region (VH) and the light chain variable region (VL) in the aforementioned first antigen-binding domain that specifically binds to FAP.

In some embodiments, in the anti-FAP/CD40 bispecific antibody:

the first antigen-binding domain that specifically binds to FAP is a first antibody, which comprises a heavy chain (HC) and a light chain (LC); and
the second antigen-binding domain that specifically binds to CD40 is a second antibody, which is a VHH and has the CDR1, CDR2, and CDR3 in the aforementioned CD40-binding molecule.

In some specific embodiments, the VHH, as a second antibody, is located at the N-terminus and/or the C-terminus of the heavy or light chain of the first antibody.

In some specific embodiments, the anti-FAP/CD40 bispecific antibody comprises 1 first antibody and 1, 2, 3, 4, 5, 6, 7, 8, 9, or 10 (e.g., 2, 4, or 6) VHH second antibodies; the first antibody comprises two HCs and two LCs, wherein the VH of one HC of the first antibody forms an antigen-binding site with the VL of one LC, and the VH of the other HC forms an antigen-binding site with the VL of the other LC.

In some specific embodiments, the first antibody of the anti-FAP/CD40 bispecific antibody or the antigen-binding fragment thereof may be linked with 1, 2, 3, 4, 5, 6, 7, or 8 VHH second antibodies, and the VHH second antibodies may be identical or different, may all be linked to the N-terminus of the heavy chain of the first antibody, or may all be linked to the C-terminus of the heavy chain of the first antibody, or may all be linked to the N-terminus of the light chain of the first antibody, or may all be linked to the C-terminus of the light chain of the first antibody, or may be linked to any combination of the N-terminus of the heavy chain, the C-terminus of the heavy chain, the N-terminus of the light chain, and the C-terminus of the light chain.

In some specific embodiments, the anti-FAP/CD40 bispecific antibody comprises a first polypeptide chain and a second polypeptide chain, wherein:

(i) the first polypeptide chain is, from N-terminus to C-terminus: [heavy chain of first antibody]-linker 1-[second antibody]; the second polypeptide chain is the light chain of the first antibody;
(ii) the first polypeptide chain is, from N-terminus to C-terminus: [heavy chain of first antibody]-linker 1-[second antibody]$_1$-linker 2-[second antibody]$_2$; the second polypeptide chain is the light chain of the first antibody;
(iii) the first polypeptide chain is, from N-terminus to C-terminus: [heavy chain of first antibody]-linker 1-[second antibody]₁-linker 2-[second antibody]₂-linker 3-[second antibody]₃; the second polypeptide chain is the light chain of the first antibody;

(iv) the first polypeptide chain is, from N-terminus to C-terminus: [second antibody]-linker 1-[heavy chain of first antibody]; the second polypeptide chain is the light chain of the first antibody;

(v) the first polypeptide chain is, from N-terminus to C-terminus: [second antibody]₂-linker 2-[second antibody]₁-linker 1-[heavy chain of first antibody]; the second polypeptide chain is the light chain of the first antibody;

(vi) the first polypeptide chain is, from N-terminus to C-terminus: [second antibody]₃-linker 3-[second antibody]₂-linker 2-[second antibody] i-linker 1-[heavy chain of first antibody]; the second polypeptide chain is the light chain of the first antibody;

(vii) the first polypeptide chain is, from N-terminus to C-terminus: [second antibody]₁-linker 1-[heavy chain of first antibody]-linker 2-[second antibody]₂; the second polypeptide chain is the light chain of the first antibody;

(viii) the first polypeptide chain is, from N-terminus to C-terminus: [second antibody]₁-linker 1-[second antibody]₂-linker 2-[heavy chain of first antibody]-linker 3-[second antibody]₃; the second polypeptide chain is the light chain of the first antibody;

(ix) the first polypeptide chain is, from N-terminus to C-terminus: [second antibody]₁-linker 1-[heavy chain of first antibody]-linker 2-[second antibody]₂-linker 3-[second antibody]₃; the second polypeptide chain is the light chain of the first antibody;

wherein the [second antibody]₁, [second antibody]₂, and [second antibody]₃ may be identical or different.

In some embodiments, the VHH second antibodies in the aforementioned anti-FAP/CD40 bispecific antibody are linked, either directly or by linkers, to the first antibody. The linkers are selected from the group consisting of amino acid sequences represented by $(G_mS_n)_x$, $(GGNGT)_x$, and $(YGNGT)_x$, wherein m and n are each independently selected from the group consisting of integers of 1-8 (e.g., 1, 2, 3, 4, 5, 6, 7, or 8), and x is independently selected from the group consisting of integers of 1-20 (e.g., 1, 2, 3, 4, 5, 6, 7, 8, 9, 10, 11, 12, 13, 14, 15, 16, 17, 18, 19, or 20). For example, the linkers are amino acid sequences represented by $G_4S$, $(G_4S)_2$, $(G_4S)_3$, $(G_4S)_4$, $(G_4S)_5$, or $(G_4S)_6$. In some embodiments, linker 1, linker 2, and linker 3 may be identical or different.

In some embodiments, the heavy chain of the first antibody of the anti-FAP/CD40 bispecific antibody comprises a heavy chain variable region (VH) and a heavy chain constant region (CH), and the light chain comprises a light chain variable region (VL) and a light chain constant region (CL). The first antibody may be a full-length antibody. In some embodiments, the heavy chain of the first antibody of the anti-FAP/CD40 bispecific antibody is of an IgG isotype (e.g., IgG1, IgG2, IgG3, or IgG4), e.g., the IgG1 isotype; and/or the light chain of the first antibody is of the Kappa isotype.

In some embodiments, the two HCs of the anti-FAP/CD40 bispecific antibody comprise identical CDRs and/or the two LCs comprise identical CDRs. In some specific embodiments, the two HCs of the first antibody comprise identical VH and/or the two LCs comprise identical VL. In some specific embodiments, the two HCs of the first antibody have identical amino acid sequences and/or the two LCs have identical amino acid sequences.

In some embodiments, two VHH second antibodies of the anti-FAP/CD40 bispecific antibody have identical or different amino acid sequences. For example, the two VHH second antibodies have identical amino acid sequences.

In some embodiments, the anti-FAP/CD40 bispecific antibody comprises two first polypeptide chains and two second polypeptide chains, wherein for each polypeptide chain: a) each of the first polypeptide chains independently comprises a VHH second antibody and the heavy chain (HC) of the first antibody, and b) each of the second polypeptide chains independently comprises the light chain (LC) of the first antibody, wherein the VHH is linked to the N-terminus and/or the C-terminus of the HC of the second antibody by a linker;

or, i) each of the first polypeptide chains independently comprises the heavy chain (HC) of the first antibody, and ii) each of the second polypeptide chains independently comprises a VHH second antibody and the light chain (LC) of the first antibody, wherein the VHH is linked, either directly or by a linker, to the N-terminus and/or the C-terminus of the LC of the first antibody.

In some specific embodiments, the anti-FAP/CD40 bispecific antibody comprises two identical first polypeptide chains and two identical second polypeptide chains.

In some embodiments, a mutation is introduced into the Fc region of the FAP/CD40-binding molecule or the anti-FAP/CD40 bispecific antibody. The mutation is, for example, a mutation that removes or reduces IgG Fc effector function, including but not limited to, N297A or D265A/N297A on IgG1, or L234A/L235A, L234A/L235A/P329G, L234E, L234F, L234E/L235F, or L234E/L235F/P329G on IgG1, V234A/G237A/P238S/H268A/V309L/A330S/P331S on IgG2, F234A/L235A on IgG4, S228P/F234A/L235A on IgG4, N297A on IgG2 or IgG4, V234A/G237A on IgG2, K214T/E233P/L234V/L235A/G236 deletion/A327G/P331A/D365E/L358M on IgG1, H268Q/V309L/A330S/P331S on IgG2, S267E/L328F on IgG1, L234F/L235E/D265A on IgG1, L234A/L235A/G237A/P238S/H268A/A330S/P331S on IgG1, and S228P/F234A/L235A/G237A/P238S on IgG4.

In the context of the mutations contained in the Fc region herein, "/" represents "and"; for example, "D265A/N297A" represents "D265A and N297A"; that is, the Fc comprises mutations D265A and N297A; the amino acid positions of the mutations are numbered according to the EU numbering scheme.

In some embodiments, the aforementioned FAP/CD40-binding molecule or anti-FAP/CD40 bispecific antibody has one or more of the following characteristics:

(a) binding to human FAP or an epitope thereof with a $K_D$ value of $\leq 10^{-7}$;
(b) binding to human CD40 or an epitope thereof with a $K_D$ value of $\leq 10^{-7}$;
(c) inducing the immune stimulation of CD40-expressing antigen-presenting cells (APCs);
(d) increasing APC (e.g., dendritic cell) activation, and/or promoting APC (e.g., dendritic cell) proliferation;
(e) stimulating tumor-specific T-cell responses;
(f) causing or promoting tumor cell apoptosis; and/or
(g) inhibiting tumor growth and/or metastasis.

In some embodiments, the FAP/CD40-binding molecule in the pharmaceutical composition is at a concentration of 0.01 mg/mL-500 mg/mL, such as 0.05 mg/mL-450 mg/mL, 0.05 mg/mL-400 mg/mL, 0.05 mg/mL-350 mg/mL, 0.05 mg/mL-300 mg/mL, 0.05 mg/mL-250 mg/mL, 0.05 mg/mL-200 mg/mL, 0.05 mg/mL-150 mg/mL, 0.05 mg/mL-140 mg/mL, 0.05 mg/mL-130 mg/mL, 0.05 mg/mL-120 mg/mL, 0.05 mg/mL-110 mg/mL, 0.05 mg/mL-100 mg/mL, 0.1 mg/mL-400 mg/mL, 0.1 mg/mL-350 mg/mL, 0.1 mg/mL-300 mg/mL, 0.1 mg/mL-250 mg/mL, 0.1 mg/mL-200 mg/mL, 0.1 mg/mL-150 mg/mL, 0.1 mg/mL-140 mg/mL, 0.1 mg/mL-130 mg/mL, 0.1 mg/mL-120 mg/mL, 0.1 mg/mL-110 mg/mL, 0.1 mg/mL-100 mg/mL, 0.5 mg/mL-350 mg/mL, 0.5 mg/mL-300 mg/mL, 0.5 mg/mL-250 mg/mL, 0.5 mg/mL-200 mg/mL, 0.5 mg/mL-150 mg/mL, 0.5 mg/mL-140 mg/mL, 0.5 mg/mL-130 mg/mL, 0.5 mg/mL-120 mg/mL, 0.5 mg/mL-110 mg/mL, 0.5 mg/mL-100 mg/mL, 1 mg/mL-300 mg/mL, 1 mg/mL-250 mg/mL, 1 mg/mL-200 mg/mL, 1 mg/mL-150 mg/mL, 1 mg/mL-140 mg/mL, 1 mg/mL-130 mg/mL, 1 mg/mL-120 mg/mL, 1 mg/mL-110 mg/mL, 1 mg/mL-100 mg/mL, 1 mg/mL-95 mg/mL, 1 mg/mL-90 mg/mL, 1 mg/mL-85 mg/mL, 1 mg/mL-80 mg/mL, 1 mg/mL-75 mg/mL, 1 mg/mL-70 mg/mL, 1 mg/mL-65 mg/mL, 1 mg/mL-60 mg/mL, 1 mg/mL-55 mg/mL, 1 mg/mL-50 mg/mL, 1 mg/mL-45 mg/mL, 1 mg/mL-40 mg/mL, 1 mg/mL-35 mg/mL, 1 mg/mL-30 mg/mL, 1 mg/mL-25 mg/mL, 1 mg/mL-20 mg/mL, 1 mg/mL-15 mg/mL, 5 mg/mL-90 mg/mL, 5 mg/mL-85 mg/mL, 5 mg/mL-80 mg/mL, 5 mg/mL-75 mg/mL, 5 mg/mL-70 mg/mL, 5 mg/mL-65 mg/mL, 5 mg/mL-60 mg/mL, 5 mg/mL-55 mg/mL, 5 mg/mL-50 mg/mL, 5 mg/mL-45 mg/mL, 5 mg/mL-40 mg/mL, 5 mg/mL-35 mg/mL, 5 mg/mL-30 mg/mL, 5 mg/mL-25 mg/mL, 5 mg/mL-20 mg/mL, 5 mg/mL-15 mg/mL, 8 mg/mL-12 mg/mL, 30 mg/mL-250 mg/mL, 30 mg/mL-200 mg/mL, 30 mg/mL-190 mg/mL, 30 mg/mL-180 mg/mL, 30 mg/mL-170 mg/mL, 30 mg/mL-160 mg/mL, 30 mg/mL-150 mg/mL, 30 mg/mL-140 mg/mL, 30 mg/mL-130 mg/mL, 30 mg/mL-120 mg/mL, 30 mg/mL-110 mg/mL, 30 mg/mL-100 mg/mL, 50 mg/mL-200 mg/mL, 50 mg/mL-190 mg/mL, 50 mg/mL-180 mg/mL, 50 mg/mL-170 mg/mL, 50 mg/mL-160 mg/mL, 50 mg/mL-150 mg/mL, 50 mg/mL-140 mg/mL, 50 mg/mL-130 mg/mL, 50 mg/mL-120 mg/mL, 50 mg/mL-110 mg/mL, 50 mg/mL-100 mg/mL, 70 mg/mL-180 mg/mL, 70 mg/mL-170 mg/mL, 70 mg/mL-160 mg/mL, 70 mg/mL-150 mg/mL, 70 mg/mL-140 mg/mL, 70 mg/mL-130 mg/mL, 70 mg/mL-120 mg/mL, 70 mg/mL-110 mg/mL, 70 mg/mL-100 mg/mL, 90 mg/mL-110 mg/mL, or 95 mg/mL-105 mg/mL. In some embodiments, the FAP/CD40-binding molecule in the pharmaceutical composition is at a concentration of 0.1 mg/mL-400 mg/mL. In some embodiments, the FAP/CD40-binding molecule in the pharmaceutical composition is at a concentration of 0.5 mg/mL-200 mg/mL. In some embodiments, the FAP/CD40-binding molecule in the pharmaceutical composition is at a concentration of 1 mg/mL-150 mg/mL. In some embodiments, the FAP/CD40-binding molecule in the pharmaceutical composition is at a concentration of 5 mg/mL-45 mg/mL. In some embodiments, the FAP/CD40-binding molecule in the pharmaceutical composition is at a concentration of 50 mg/mL-110 mg/mL. In some embodiments, the FAP/CD40-binding molecule in the pharmaceutical composition is at a concentration of about 1 mg/mL, about 5 mg/mL, about 10 mg/mL, about 15 mg/mL, about 20 mg/mL, about 25 mg/mL, about 30 mg/mL, about 40 mg/mL, about 45 mg/mL, about 50 mg/mL, about 55 mg/mL, about 60 mg/mL, about 70 mg/mL, about 75 mg/mL, about 80 mg/mL, about 85 mg/mL, about 90 mg/mL, about 95 mg/mL, or about 100 mg/mL. In some embodiments, the FAP/CD40-binding molecule in the pharmaceutical composition is at a concentration of about 5 mg/mL, about 10 mg/mL, about 20 mg/mL, about 25 mg/mL, about 50 mg/mL, about 80 mg/mL, or about 100 mg/mL. In some embodiments, the FAP/CD40-binding molecule in the pharmaceutical composition is at a concentration of about 5 mg/mL, about 10 mg/mL, about 20 mg/mL, or about 25 mg/mL. In some embodiments, the FAP/CD40-binding molecule in the pharmaceutical composition is at a concentration of about 50 mg/mL, about 80 mg/mL, or about 100 mg/mL. In some embodiments, the FAP/CD40-binding molecule in the pharmaceutical composition is at a concentration of at least 500 mg/mL. In some embodiments, the FAP/CD40-binding molecule in the pharmaceutical composition is at a concentration of at least 450 mg/mL, at least 400 mg/mL, at least 350 mg/mL, at least 300 mg/mL, at least 250 mg/mL, at least 200 mg/mL, at least 150 mg/mL, at least 100 mg/mL, at least 50 mg/mL, at least 10 mg/mL, at least 1 mg/mL, at least 0.1 mg/mL, or at least 0.01 mg/mL.

In some embodiments, the buffer in the pharmaceutical composition is at a concentration of 0.1 mM-50 mM, such as 0.1 mM-45 mM, 0.1 mM-40 mM, 0.1 mM-35 mM, 0.1 mM-30 mM, 0.1 mM-25 mM, 0.1 mM-20 mM, 0.1 mM-15 mM, 0.1 mM-10 mM, 0.5 mM-45 mM, 0.5 mM-40 mM, 0.5 mM-35 mM, 0.5 mM-30 mM, 0.5 mM-25 mM, 0.5 mM-20 mM, 0.5 mM-15 mM, 0.5 mM-10 mM, 1 mM-40 mM, 1 mM-35 mM, 1 mM-30 mM, 1 mM-25 mM, 1 mM-20 mM, 1 mM-15 mM, 1 mM-10 mM, 5 mM-35 mM, 5 mM-30 mM, 5 mM-25 mM, 5 mM-20 mM, 5 mM-15 mM, 5 mM-10 mM, 8 mM-30 mM, 8 mM-25 mM, 8 mM-20 mM, 8 mM-15 mM, or 8 mM-12 mM. In some embodiments, the buffer in the pharmaceutical composition is at a concentration of 0.5 mM-40 mM. In some embodiments, the buffer in the pharmaceutical composition is at a concentration of 1 mM-30 mM. In some embodiments, the buffer in the pharmaceutical composition is at a concentration of 5 mM-20 mM. In some embodiments, the buffer in the pharmaceutical composition is at a concentration of about 1 mM, about 2 mM, about 3 mM, about 4 mM, about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 21 mM, about 22 mM, about 23 mM, about 24 mM, about 25 mM, about 26 mM, about 27 mM, about 28 mM, about 29 mM, or about 30 mM. In some embodiments, the buffer in the pharmaceutical composition is at a concentration of about 10 mM. In some embodiments, the buffer in the pharmaceutical composition is at a concentration of at least 50 mM. In some embodiments, the buffer in the pharmaceutical composition is at a concentration of at least 10 mM.

In some embodiments, the pharmaceutical composition further comprises a surfactant. In some embodiments, the surfactant is one or more surfactants selected from the group consisting of a polysorbate and a poloxamer. In some embodiments, the polysorbate is one or more polysorbates selected from the group consisting of polysorbate 20 and polysorbate 80. In some embodiments, the poloxamer is poloxamer 188. In some embodiments, the surfactant is a polysorbate. In some embodiments, the surfactant is polysorbate 80.

In some embodiments, the surfactant in the pharmaceutical composition is at a concentration of 0.01 mg/mL-10 mg/mL, such as 0.01 mg/mL-9 mg/mL, 0.01 mg/mL-8 mg/mL, 0.01 mg/mL-7 mg/mL, 0.01 mg/mL-6 mg/mL, 0.01 mg/mL-5 mg/mL, 0.01 mg/mL-4 mg/mL, 0.01 mg/mL-3 mg/mL, 0.01 mg/mL-2 mg/mL, 0.01 mg/mL-1 mg/mL, 0.01 mg/mL-0.5 mg/mL, 0.05 mg/mL-8 mg/mL, 0.05 mg/mL-7 mg/mL, 0.05 mg/mL-6 mg/mL, 0.05 mg/mL-5 mg/mL, 0.05 mg/mL-4 mg/mL, 0.05 mg/mL-3 mg/mL, 0.05 mg/mL-2 mg/mL, 0.05 mg/mL-1 mg/mL, 0.05 mg/mL-0.5 mg/mL, 0.1 mg/mL-6 mg/mL, 0.1 mg/mL-5 mg/mL, 0.1 mg/mL-4 mg/mL, 0.1 mg/mL-3 mg/mL, 0.1 mg/mL-2 mg/mL, 0.1 mg/mL-1 mg/mL, 0.1 mg/mL-0.5 mg/mL, 0.2 mg/mL-5 mg/mL, 0.2 mg/mL-4.5 mg/mL, 0.2 mg/mL-4 mg/mL, 0.2 mg/mL-3.5 mg/mL, 0.2 mg/mL-3 mg/mL, 0.2 mg/mL-2.5 mg/mL, 0.2 mg/mL-2 mg/mL, 0.2 mg/mL-1.5 mg/mL, 0.2 mg/mL-1 mg/mL, 0.2 mg/mL-0.5 mg/mL, 0.3 mg/mL-4.5 mg/mL, 0.3 mg/mL-4 mg/mL, 0.3 mg/mL-3.5 mg/mL, 0.3 mg/mL-3 mg/mL, 0.3 mg/mL-2.5 mg/mL, 0.3 mg/mL-2 mg/mL, 0.3 mg/mL-1.5 mg/mL, 0.3 mg/mL-1 mg/mL, 0.3 mg/mL-0.5 mg/mL, 0.7 mg/mL-3.5 mg/mL, 0.7 mg/mL-3 mg/mL, 0.7 mg/mL-2.5 mg/mL, 0.7 mg/mL-2 mg/mL, 0.7 mg/mL-1.5 mg/mL, 0.7 mg/mL-1 mg/mL, or 0.7 mg/mL-0.9 mg/mL. In some embodiments, the surfactant in the pharmaceutical composition is at a concentration of 0.05 mg/mL-5 mg/mL. In some embodiments, the surfactant in the pharmaceutical composition is at a concentration of 0.1 mg/mL-3 mg/mL. In some embodiments, the surfactant in the pharmaceutical composition is at a concentration of 0.2 mg/mL-2 mg/mL. In some embodiments, the surfactant in the pharmaceutical composition is at a concentration of about 0.1 mg/mL, about 0.2 mg/mL, about 0.3 mg/mL, about 0.4 mg/mL, about 0.5 mg/mL, about 0.6 mg/mL, about 0.7 mg/mL, about 0.8 mg/mL, about 0.9 mg/mL, about 1 mg/mL, about 1.1 mg/mL, about 1.2 mg/mL, about 1.3 mg/mL, about 1.4 mg/mL, about 1.5 mg/mL, about 1.6 mg/mL, about 1.7 mg/mL, about 1.8 mg/mL, about 1.9 mg/mL, or about 2 mg/mL. In some embodiments, the surfactant in the pharmaceutical composition is at a concentration of about 0.2 mg/mL, about 0.4 mg/mL, about 0.6 mg/mL, or about 0.8 mg/mL. In some embodiments, the surfactant in the pharmaceutical composition is at a concentration of about 0.4 mg/mL, about 0.6 mg/mL, or about 0.8 mg/mL. In some embodiments, the surfactant in the pharmaceutical composition is at a concentration of about 0.4 mg/mL or about 0.6 mg/mL. In some embodiments, the surfactant in the pharmaceutical composition is at a concentration of about 0.4 mg/mL or about 0.8 mg/mL. In some embodiments, the surfactant in the pharmaceutical composition is at a concentration of at least 10 mg/mL. In some embodiments, the surfactant in the pharmaceutical composition is at a concentration of at least 0.4 mg/mL, at least 0.6 mg/mL, or at least 0.8 mg/mL.

In some embodiments, the pharmaceutical composition further comprises a sugar. In some embodiments, the sugar is one or more sugars selected from the group consisting of sucrose, glucose, trehalose, and maltose. In some embodiments, the sugar is sucrose.

In some embodiments, the sugar in the pharmaceutical composition is at a concentration of 1 mg/mL-200 mg/mL, such as 1 mg/mL-150 mg/mL, 1 mg/mL-140 mg/mL, 1 mg/mL-130 mg/mL, 1 mg/mL-120 mg/mL, 1 mg/mL-110 mg/mL, 1 mg/mL-100 mg/mL, 1 mg/mL-95 mg/mL, 1 mg/mL-90 mg/mL, 1 mg/mL-85 mg/mL, 1 mg/mL-80 mg/mL, 10 mg/mL-150 mg/mL, 10 mg/mL-140 mg/mL, 10 mg/mL-130 mg/mL, 10 mg/mL-120 mg/mL, 10 mg/mL-110 mg/mL, 10 mg/mL-100 mg/mL, 10 mg/mL-95 mg/mL, 10 mg/mL-90 mg/mL, 10 mg/mL-85 mg/mL, 10 mg/mL-80 mg/mL, 20 mg/mL-130 mg/mL, 20 mg/mL-120 mg/mL, 20 mg/mL-110 mg/mL, 20 mg/mL-100 mg/mL, 20 mg/mL-95 mg/mL, 20 mg/mL-90 mg/mL, 20 mg/mL-85 mg/mL, 20 mg/mL-80 mg/mL, 30 mg/mL-120 mg/mL, 30 mg/mL-110 mg/mL, 30 mg/mL-100 mg/mL, 30 mg/mL-95 mg/mL, 30 mg/mL-90 mg/mL, 30 mg/mL-85 mg/mL, 30 mg/mL-80 mg/mL, 50 mg/mL-110 mg/mL, 50 mg/mL-100 mg/mL, 50 mg/mL-95 mg/mL, 50 mg/mL-90 mg/mL, 50 mg/mL-85 mg/mL, 50 mg/mL-80 mg/mL, 60 mg/mL-100 mg/mL, 60 mg/mL-95 mg/mL, 60 mg/mL-90 mg/mL, 60 mg/mL-85 mg/mL, 60 mg/mL-80 mg/mL, 70 mg/mL-95 mg/mL, 70 mg/mL-90 mg/mL, 70 mg/mL-85 mg/mL, or 70 mg/mL-80 mg/mL. In some embodiments, the sugar in the pharmaceutical composition is at a concentration of 10 mg/mL-150 mg/mL. In some embodiments, the sugar in the pharmaceutical composition is at a concentration of 30 mg/mL-120 mg/mL. In some embodiments, the sugar in the pharmaceutical composition is at a concentration of 50 mg/mL-100 mg/mL. In some embodiments, the sugar in the pharmaceutical composition is at a concentration of about 30 mg/mL, about 35 mg/mL, about 40 mg/mL, about 45 mg/mL, about 50 mg/mL, about 55 mg/mL, about 60 mg/mL, about 65 mg/mL, about 70 mg/mL, about 75 mg/mL, about 80 mg/mL, about 85 mg/mL, about 90 mg/mL, about 95 mg/mL, about 100 mg/mL, about 105 mg/mL, about 110 mg/mL, about 115 mg/mL, or about 120 mg/mL. In some embodiments, the sugar in the pharmaceutical composition is at a concentration of about 80 mg/mL. In some embodiments, the sugar in the pharmaceutical composition is at a concentration of at least 200 mg/mL. In some embodiments, the sugar in the pharmaceutical composition is at a concentration of at least 80 mg/mL.

In some embodiments, the pharmaceutical composition further optionally comprises one or more additional auxiliary materials selected from the group consisting of a polyol and a metal chelating agent. In some embodiments, the polyol is one or more polyols selected from the group consisting of glycerol, mannitol, and sorbitol. In some embodiments, the metal chelating agent is selected from the group consisting of ethylenediaminetetraacetic acid and a pharmaceutically acceptable salt thereof. In some embodiments, the polyol is mannitol. In some embodiments, the metal chelating agent is ethylenediaminetetraacetic acid.

In some embodiments, the additional auxiliary material in the pharmaceutical composition is at a concentration of 0.001% w/v-20% w/v, such as 0.005% w/v-20% w/v, 0.005% w/v-15% w/v, 0.005% w/v-10% w/v, 0.005% w/v-9% w/v, 0.005% w/v-8% w/v, 0.005% w/v-7% w/v, 0.005% w/v-6% w/v, 0.005% w/v-5% w/v, 0.005% w/v-4% w/v, 0.005% w/v-3% w/v, 0.005% w/v-2% w/v, 0.005% w/v-1% w/v, 0.005% w/v-0.1% w/v, 0.005% w/v-0.05% w/v, 0.008% w/v-15% w/v, 0.008% w/v-10% w/v, 0.008% w/v-9% w/v, 0.008% w/v-8% w/v, 0.008% w/v-7% w/v, 0.008% w/v-6% w/v, 0.008% w/v-5% w/v, 0.008% w/v-4% w/v, 0.008% w/v-3% w/v, 0.008% w/v-2% w/v, 0.008% w/v-1% w/v, 0.008% w/v-0.1% w/v, 0.008% w/v-0.05% w/v, 0.01% w/v-10% w/v, 0.01% w/v-9% w/v, 0.01% w/v-8% w/v, 0.01% w/v-7% w/v, 0.01% w/v-6% w/v, 0.01% w/v-5% w/v, 0.01% w/v-4% w/v, 0.01% w/v-3% w/v, 0.01% w/v-2% w/v, 0.01% w/v-1% w/v, 0.01% w/v-0.1% w/v, 0.01% w/v-0.05% w/v, 0.1% w/v-15% w/v, 0.1% w/v-10% w/v, 0.1% w/v-10% w/v, 0.1% w/v-9% w/v, 0.1% w/v-8% w/v, 0.1% w/v-7% w/v, 0.1% w/v-6% w/v, 0.1% w/v-5% w/v, 1% w/v-15% w/v, 1% w/v-10% w/v, 1% w/v-9% w/v, 1% w/v-8% w/v, 1% w/v-7% w/v, 1% w/v-6% w/v, 1% w/v-5% w/v, 0.001% w/v-5% w/v, 0.001% w/v-4% w/v, 0.001% w/v-3% w/v, 0.001% w/v-2% w/v, 0.001% w/v-1% w/v, 0.001% w/v-0.1% w/v, or 0.001% w/v-0.05% w/v. In some embodiments, the additional auxiliary material in the pharmaceutical composition is at a concentration of 0.005% w/v-15% w/v. In some embodiments, the additional auxiliary material in the pharmaceutical composition is at a concentration of 0.008% w/v-10% w/v. In some embodiments, the additional auxiliary material in the pharmaceutical composition is at a concentration of 0.01% w/v-8% w/v.

In some embodiments, mannitol in the pharmaceutical composition is at a concentration of 0.1% w/v-15% w/v, such as 0.5% w/v-12% w/v, 0.5% w/v-11% w/v, 0.5% w/v-10% w/v, 0.5% w/v-9% w/v, 0.5% w/v-8% w/v, 0.5% w/v-7% w/v, 0.5% w/v-6% w/v, 0.5% w/v-5% w/v, 1% w/v-10% w/v, 1% w/v-9% w/v, 1% w/v-8% w/v, 1% w/v-7% w/v, 1% w/v-6% w/v, or 1% w/v-5% w/v. In some embodiments, mannitol in the pharmaceutical composition is at a concentration of 0.5% w/v-12% w/v. In some embodiments, mannitol in the pharmaceutical composition is at a concentration of 1% w/v-10% w/v. In some embodiments, mannitol in the pharmaceutical composition is at a concentration of about 1% w/v, about 2% w/v, about 3% w/v, about 4% w/v, about 5% w/v, about 6% w/v, about 7% w/v, about 8% w/v, about 9% w/v, or about 10% w/v. In some embodiments, mannitol in the pharmaceutical composition is at a concentration of about 4% w/v. In some embodiments, mannitol in the pharmaceutical composition is at a concentration of at least 15% w/v. In some embodiments, mannitol in the pharmaceutical composition is at a concentration of at least 4% w/v.

In some embodiments, ethylenediaminetetraacetic acid in the pharmaceutical composition is at a concentration of 0.001% w/v-5% w/v, such as 0.001% w/v-4% w/v, 0.001% w/v-3% w/v, 0.001% w/v-2% w/v, 0.001% w/v-1% w/v, 0.001% w/v-0.1% w/v, 0.001% w/v-0.05% w/v, 0.005% w/v-4% w/v, 0.005% w/v-3% w/v, 0.005% w/v-2% w/v, 0.005% w/v-1% w/v, 0.005% w/v-0.1% w/v, or 0.005% w/v-0.05% w/v. In some embodiments, ethylenediaminetetraacetic acid in the pharmaceutical composition is at a concentration of 0.001% w/v-1% w/v. In some embodiments, ethylenediaminetetraacetic acid in the pharmaceutical composition is at a concentration of 0.005% w/v-0.1% w/v. In some embodiments, ethylenediaminetetraacetic acid in the pharmaceutical composition is at a concentration of about 0.005% w/v, about 0.006% w/v, about 0.007% w/v, about 0.008% w/v, about 0.009% w/v, about 0.01% w/v, about 0.02% w/v, about 0.03% w/v, about 0.04% w/v, about 0.05% w/v, about 0.06% w/v, about 0.07% w/v, about 0.08% w/v, about 0.09% w/v, or about 0.1% w/v. In some embodiments, ethylenediaminetetraacetic acid in the pharmaceutical composition is at a concentration of about 0.01% w/v. In some embodiments, ethylenediaminetetraacetic acid in the pharmaceutical composition is at a concentration of at least 5% w/v. In some embodiments, ethylenediaminetetraacetic acid in the pharmaceutical composition is at a concentration of at least 0.01% w/v.

In some embodiments, the buffer in the pharmaceutical composition has a pH of 3.5-9, such as 3.5-8.5, 3.5-8, 3.5-7.5, 3.5-7, 3.5-6.9, 3.5-6.8, 3.5-6.7, 3.5-6.6, 3.5-6.5, 3.5-6.4, 3.5-6.3, 3.5-6.2, 3.5-6.1, 3.5-6, 3.5-5.9, 3.5-5.8, 3.5-5.7, 3.5-5.6, 3.5-5.5, 4-5.4, 4-5.3, 4-5.2, 4-5.1, 4-5.0, 4-8.5, 4-8, 4-7.5, 4-7, 4-6.9, 4-6.8, 4-6.7, 4-6.6, 4-6.5, 4-6.4, 4-6.3, 4-6.2, 4-6.1, 4-6, 4-5.9, 4-5.8, 4-5.7, 4-5.6, 4-5.5, 4-5.4, 4-5.3, 4-5.2, 4-5.1, 4-5.0, 4.2-8, 4.2-7.5, 4.2-7, 4.2-6.9, 4.2-6.8, 4.2-6.7, 4.2-6.6, 4.2-6.5, 4.2-6.4, 4.2-6.3, 4.2-6.2, 4.2-6.1, 4.2-6, 4.2-5.9, 4.2-5.8, 4.2-5.7, 4.2-5.6, 4.2-5.5, 4.2-5.4, 4.2-5.3, 4.2-5.2, 4.2-5.1, 4.2-5.0, 4.5-8, 4.5-7.5, 4.5-7, 4.5-6.9, 4.5-6.8, 4.5-6.7, 4.5-6.6, 4.5-6.5, 4.5-6.4, 4.5-6.3, 4.5-6.2, 4.5-6.1, 4.5-6, 4.5-5.9, 4.5-5.8, 4.5-5.7, 4.5-5.6, 4.5-5.5, 4.5-5.4, 4.5-5.3, 4.5-5.2, 4.5-5.1, 4.5-5.0, 4.6-7.5, 4.6-7, 4.6-6.9, 4.6-6.8, 4.6-6.7, 4.6-6.6, 4.6-6.5, 4.6-6.4, 4.6-6.3, 4.6-6.2, 4.6-6.1, 4.6-6, 4.6-5.9, 4.6-5.8, 4.6-5.7, 4.6-5.6, 4.6-5.5, 4.6-5.4, 4.6-5.3, 4.6-5.2, 4.6-5.1, 4.6-5.0, 4.8-7, 4.8-6.9, 4.8-6.8, 4.8-6.7, 4.8-6.6, 4.8-6.5, 4.8-6.4, 4.8-6.3, 4.8-6.2, 4.8-6.1, 4.8-6, 4.8-5.9, 4.8-5.8, 4.8-5.7, 4.8-5.6, 4.8-5.5, 5-6.5, 5-6.4, 5-6.3, 5-6.2, 5-6.1, 5-6, 5-5.9, 5-5.8, 5-5.7, 5-5.6, or 5-5.5. In some embodiments, the buffer in the pharmaceutical composition has a pH of 3.5-7. In some embodiments, the buffer in the pharmaceutical composition has a pH of 4.0-6.5. In some embodiments, the buffer in the pharmaceutical composition has a pH of 4.2-6.2. In some embodiments, the buffer in the pharmaceutical composition has a pH of 4.5-6.0. In some embodiments, the buffer in the pharmaceutical composition has a pH of about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, or about 6.5. In some embodiments, the buffer in the pharmaceutical composition has a pH of about 5.0, about 5.2, about 5.3, about 5.5, about 5.6, about 5.8, or about 6.0. In some embodiments, the buffer in the pharmaceutical composition has a pH of about 5.0, about 5.2, about 5.3, or about 5.5.

In some embodiments, the pharmaceutical composition further comprises a pH adjuster, such as sodium hydroxide and/or hydrochloric acid.

In some embodiments, there is a difference of no more than ±0.5 between the pH of the pharmaceutical composition and the pH of the buffer that the pharmaceutical composition comprises. In some embodiments, the pharmaceutical composition has a pH of 3.5-9, such as 3.5-8.5, 3.5-8, 3.5-7.5, 3.5-7, 3.5-6.9, 3.5-6.8, 3.5-6.7, 3.5-6.6, 3.5-6.5, 3.5-6.4, 3.5-6.3, 3.5-6.2, 3.5-6.1, 3.5-6, 3.5-5.9, 3.5-5.8, 3.5-5.7, 3.5-5.6, 3.5-5.5, 4-5.4, 4-5.3, 4-5.2, 4-5.1, 4-5.0, 4-8.5, 4-8, 4-7.5, 4-7, 4-6.9, 4-6.8, 4-6.7, 4-6.6, 4-6.5, 4-6.4, 4-6.3, 4-6.2, 4-6.1, 4-6, 4-5.9, 4-5.8, 4-5.7, 4-5.6, 4-5.5, 4-5.4, 4-5.3, 4-5.2, 4-5.1, 4-5.0, 4.2-8, 4.2-7.5, 4.2-7, 4.2-6.9, 4.2-6.8, 4.2-6.7, 4.2-6.6, 4.2-6.5, 4.2-6.4, 4.2-6.3, 4.2-6.2, 4.2-6.1, 4.2-6, 4.2-5.9, 4.2-5.8, 4.2-5.7, 4.2-5.6, 4.2-5.5, 4.2-5.4, 4.2-5.3, 4.2-5.2, 4.2-5.1, 4.2-5.0, 4.5-8, 4.5-7.5, 4.5-7, 4.5-6.9, 4.5-6.8, 4.5-6.7, 4.5-6.6, 4.5-6.5, 4.5-6.4, 4.5-6.3, 4.5-6.2, 4.5-6.1, 4.5-6, 4.5-5.9, 4.5-5.8, 4.5-5.7, 4.5-5.6, 4.5-5.5, 4.5-5.4, 4.5-5.3, 4.5-5.2, 4.5-5.1, 4.5-5.0, 4.6-7.5, 4.6-7, 4.6-6.9, 4.6-6.8, 4.6-6.7, 4.6-6.6, 4.6-6.5, 4.6-6.4, 4.6-6.3, 4.6-6.2, 4.6-6.1, 4.6-6, 4.6-5.9, 4.6-5.8, 4.6-5.7, 4.6-5.6, 4.6-5.5, 4.6-5.4, 4.6-5.3, 4.6-5.2, 4.6-5.1, 4.6-5.0, 4.8-7, 4.8-6.9, 4.8-6.8, 4.8-6.7, 4.8-6.6, 4.8-6.5, 4.8-6.4, 4.8-6.3, 4.8-6.2, 4.8-6.1, 4.8-6, 4.8-5.9, 4.8-5.8, 4.8-5.7, 4.8-5.6, 4.8-5.5, 5-6.5, 5-6.4, 5-6.3, 5-6.2, 5-6.1, 5-6, 5-5.9, 5-5.8, 5-5.7, 5-5.6, or 5-5.5. In some embodiments, the pharmaceutical composition has a pH of 3.5-7. In some embodiments, the pharmaceutical composition has a pH of 4.0-6.5. In some embodiments, the pharmaceutical composition has a pH of 4.2-6.2. In some embodiments, the pharmaceutical composition has a pH of 4.5-6.0. In some embodiments, the pharmaceutical composition has a pH of about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, or about 6.5. In some embodiments, the pharmaceutical composition has a pH of about 5.0, about 5.2, about 5.3, about 5.5, about 5.6, about 5.8, or about 6.0. In some embodiments, the buffer in the pharmaceutical composition has a pH of about 5.0, about 5.2, about 5.3, or about 5.5.

In some embodiments, the present disclosure provides a pharmaceutical composition comprising a FAP/CD40-binding molecule (e.g., Ab10-A297V3-4 comprising heavy and light chain sequences of SEQ ID NOs: 22 and 20, respectively), which comprises any one of the following groups 1)-4):

1) a FAP/CD40-binding molecule;
a histidine salt buffer, such as a histidine hydrochloride buffer, a histidine acetate buffer, a histidine-histidine hydrochloride buffer, or a histidine-acetic acid buffer;
2) a FAP/CD40-binding molecule;

a histidine salt buffer;
a polysorbate, such as polysorbate 80;
sucrose;
wherein optionally, the composition further comprises mannitol or ethylenediaminetetraacetic acid;

3) a FAP/CD40-binding molecule;

a histidine hydrochloride buffer, such as a histidine-histidine hydrochloride buffer;
a polysorbate, such as polysorbate 80;
sucrose;
wherein optionally, the composition further comprises mannitol or ethylenediaminetetraacetic acid;

4) a FAP/CD40-binding molecule;

a histidine acetate buffer, such as a histidine-acetic acid buffer;
a polysorbate, such as polysorbate 80;

sucrose;
wherein optionally, the composition further comprises mannitol or ethylenediaminetetraacetic acid;

In some embodiments, the present disclosure provides a pharmaceutical composition comprising a FAP/CD40-binding molecule (e.g., Ab10-A297V3-4 comprising heavy and light chain sequences of SEQ ID NOs: 22 and 20, respectively), which is any one of the following groups 1)-4):

1) a FAP/CD40-binding molecule; a histidine salt buffer; a polysorbate; sucrose and water for injection;
2) a FAP/CD40-binding molecule; a histidine hydrochloride buffer; a polysorbate; sucrose and water for injection;
3) a FAP/CD40-binding molecule; a histidine-histidine hydrochloride buffer; a polysorbate; sucrose and water for injection;
4) a FAP/CD40-binding molecule; a histidine-acetic acid buffer; a polysorbate; sucrose and water for injection;
5) the pharmaceutical composition according to any one of 1)-4) described above, wherein the polysorbate is polysorbate 80.

In some embodiments, the present disclosure provides a pharmaceutical composition comprising a FAP/CD40-binding molecule (e.g., Ab10-A297V3-4 comprising heavy and light chain sequences of SEQ ID NOs: 22 and 20, respectively), which comprises any one of the following groups 1)-6):

1) 0.01 mg/mL-500 mg/mL FAP/CD40-binding molecule;

0.1 mM-50 mM histidine hydrochloride buffer, such as a histidine hydrochloride buffer, a histidine acetate buffer, a histidine-histidine hydrochloride buffer, or a histidine-acetic acid buffer;
0.01 mg/mL-10 mg/mL polysorbate;
1 mg/mL-200 mg/mL sucrose;
wherein optionally, the pharmaceutical composition further comprises 0.1% w/v-15% w/v mannitol or 0.001% w/v-5% w/v ethylenediaminetetraacetic acid;
and the pharmaceutical composition has a pH of 3.5-7;

2) 0.1 mg/mL-400 mg/mL FAP/CD40-binding molecule;

0.5 mM-40 mM histidine hydrochloride buffer, such as a histidine hydrochloride buffer, a histidine acetate buffer, a histidine-histidine hydrochloride buffer, or a histidine-acetic acid buffer;
0.05 mg/mL-5 mg/mL polysorbate;
10 mg/mL-150 mg/mL sucrose;
wherein optionally, the pharmaceutical composition further comprises 0.5% w/v-12% w/v mannitol or 0.001% w/v-1% w/v ethylenediaminetetraacetic acid;
and the pharmaceutical composition has a pH of 4-6.5;

3) 0.5 mg/mL-200 mg/mL FAP/CD40-binding molecule;

1 mM-30 mM histidine hydrochloride buffer, such as a histidine hydrochloride buffer, a histidine acetate buffer, a histidine-histidine hydrochloride buffer, or a histidine-acetic acid buffer;
0.1 mg/mL-3 mg/mL polysorbate;
30 mg/mL-120 mg/mL sucrose;
wherein optionally, the pharmaceutical composition further comprises 1% w/v-10% w/v mannitol or 0.005% w/v-0.1% w/v ethylenediaminetetraacetic acid;
and the pharmaceutical composition has a pH of 4.2-6.2;

4) 1 mg/mL-150 mg/mL FAP/CD40-binding molecule;

5 mM-20 mM histidine hydrochloride buffer, such as a histidine hydrochloride buffer, a histidine acetate buffer, a histidine-histidine hydrochloride buffer, or a histidine-acetic acid buffer;
0.2 mg/mL-2 mg/mL polysorbate;
50 mg/mL-100 mg/mL sucrose;
wherein optionally, the pharmaceutical composition further comprises 1% w/v-10% w/v mannitol or 0.005% w/v-0.1% w/v ethylenediaminetetraacetic acid;
and the pharmaceutical composition has a pH of 4.5-6;

5) 1 mg/mL-40 mg/mL FAP/CD40-binding molecule;

    5 mM-20 mM histidine hydrochloride buffer, such as a histidine hydrochloride buffer;
    0.2 mg/mL-2 mg/mL polysorbate;
    50 mg/mL-100 mg/mL sucrose;
    wherein optionally, the pharmaceutical composition further comprises 1% w/v-10% w/v mannitol or 0.005% w/v-0.1% w/v ethylenediaminetetraacetic acid;
    and the pharmaceutical composition has a pH of 4.5-6;

6) 50 mg/mL-150 mg/mL FAP/CD40-binding molecule;

    5 mM-20 mM histidine acetate buffer, such as a histidine-acetic acid buffer;
    0.2 mg/mL-2 mg/mL polysorbate;
    50 mg/mL-100 mg/mL sucrose;
    wherein optionally, the pharmaceutical composition further comprises 1% w/v-10% w/v mannitol or 0.005% w/v-0.1% w/v ethylenediaminetetraacetic acid;
    and the pharmaceutical composition has a pH of 4.5-6;

7) the pharmaceutical composition according to any one of 1)-6) described above, wherein the pharmaceutical composition further comprises water for injection;
8) the pharmaceutical composition according to any one of 1)-6) described above, wherein the polysorbate is polysorbate 80.

In some embodiments, the present disclosure provides a pharmaceutical composition comprising a FAP/CD40-binding molecule (e.g., Ab10-A297V3-4 comprising heavy and light chain sequences of SEQ ID NOs: 22 and 20, respectively), which comprises or is any one of the following groups 1)-25):

1) 1-150 mg/mL FAP/CD40-binding molecule;
about 10 mM histidine hydrochloride buffer or histidine acetate buffer, such as a histidine-histidine hydrochloride buffer or a histidine-acetic acid buffer;

    0.2 mg/mL-2 mg/mL polysorbate 80;
    about 80 mg/mL sucrose;
    wherein the pharmaceutical composition has a pH of 4.5-6;

2) about 5 mg/mL, about 10 mg/mL, about 20 mg/mL, about 25 mg/mL, about 50 mg/mL, about 80 mg/mL, or about 100 mg/mL FAP/CD40-binding molecule, such as about 5 mg/mL, about 10 mg/mL, about 20 mg/mL, or about 25 mg/mL FAP/CD40-binding molecule;

    about 10 mM histidine-hydrochloride buffer, such as a histidine-histidine hydrochloride buffer;
    about 0.4 mg/mL polysorbate 80;
    about 80 mg/mL sucrose;
    wherein the pharmaceutical composition has a pH of about 5.5;

3) about 5 mg/mL, about 10 mg/mL, about 20 mg/mL, about 25 mg/mL, about 50 mg/mL, about 80 mg/mL, or about 100 mg/mL FAP/CD40-binding molecule, such as about 5 mg/mL, about 10 mg/mL, about 20 mg/mL, or about 25 mg/mL FAP/CD40-binding molecule;

    about 10 mM histidine-hydrochloride buffer, such as a histidine-histidine hydrochloride buffer;
    about 0.4 mg/mL polysorbate 80;
    about 80 mg/mL sucrose;
    wherein the pharmaceutical composition has a pH of about 5.3;

4) about 5 mg/mL, about 10 mg/mL, about 20 mg/mL, about 25 mg/mL, about 50 mg/mL, about 80 mg/mL, or about 100 mg/mL FAP/CD40-binding molecule, such as about 5 mg/mL, about 10 mg/mL, about 20 mg/mL, or about 25 mg/mL FAP/CD40-binding molecule;

    about 10 mM histidine-hydrochloride buffer, such as a histidine-histidine hydrochloride buffer;

about 0.4 mg/mL polysorbate 80;
about 80 mg/mL sucrose;
wherein the pharmaceutical composition has a pH of about 5.2;

5) about 5 mg/mL, about 10 mg/mL, about 20 mg/mL, about 25 mg/mL, about 50 mg/mL, about 80 mg/mL, or about 100 mg/mL FAP/CD40-binding molecule, such as about 5 mg/mL, about 10 mg/mL, about 20 mg/mL, or about 25 mg/mL FAP/CD40-binding molecule;

about 10 mM histidine-hydrochloride buffer, such as a histidine-histidine hydrochloride buffer;
about 0.4 mg/mL polysorbate 80;
about 80 mg/mL sucrose;
wherein the pharmaceutical composition has a pH of about 5;

6) about 5 mg/mL, about 10 mg/mL, about 20 mg/mL, about 25 mg/mL, about 50 mg/mL, about 80 mg/mL, or about 100 mg/mL FAP/CD40-binding molecule, such as about 5 mg/mL, about 10 mg/mL, about 20 mg/mL, or about 25 mg/mL FAP/CD40-binding molecule;

about 10 mM histidine-hydrochloride buffer, such as a histidine-histidine hydrochloride buffer;
about 0.6 mg/mL polysorbate 80;
about 80 mg/mL sucrose;
wherein the pharmaceutical composition has a pH of about 5.5;

7) about 5 mg/mL, about 10 mg/mL, about 20 mg/mL, about 25 mg/mL, about 50 mg/mL, about 80 mg/mL, or about 100 mg/mL FAP/CD40-binding molecule, such as about 5 mg/mL, about 10 mg/mL, about 20 mg/mL, or about 25 mg/mL FAP/CD40-binding molecule;

about 10 mM histidine-hydrochloride buffer, such as a histidine-histidine hydrochloride buffer;
about 0.6 mg/mL polysorbate 80;
about 80 mg/mL sucrose;
wherein the pharmaceutical composition has a pH of about 5.3;

8) about 5 mg/mL, about 10 mg/mL, about 20 mg/mL, about 25 mg/mL, about 50 mg/mL, about 80 mg/mL, or about 100 mg/mL FAP/CD40-binding molecule, such as about 5 mg/mL, about 10 mg/mL, about 20 mg/mL, or about 25 mg/mL FAP/CD40-binding molecule;

about 10 mM histidine-hydrochloride buffer, such as a histidine-histidine hydrochloride buffer;
about 0.6 mg/mL polysorbate 80;
about 80 mg/mL sucrose;
wherein the pharmaceutical composition has a pH of about 5.2;

9) about 5 mg/mL, about 10 mg/mL, about 20 mg/mL, about 25 mg/mL, about 50 mg/mL, about 80 mg/mL, or about 100 mg/mL FAP/CD40-binding molecule, such as about 5 mg/mL, about 10 mg/mL, about 20 mg/mL, or about 25 mg/mL FAP/CD40-binding molecule;

about 10 mM histidine-hydrochloride buffer, such as a histidine-histidine hydrochloride buffer;
about 0.6 mg/mL polysorbate 80;
about 80 mg/mL sucrose;
wherein the pharmaceutical composition has a pH of about 5;

10) about 5 mg/mL, about 10 mg/mL, about 20 mg/mL, about 25 mg/mL, about 50 mg/mL, about 80 mg/mL, or about 100 mg/mL FAP/CD40-binding molecule;

about 10 mM histidine-acetic acid buffer;
about 0.4 mg/mL polysorbate 80;
about 80 mg/mL sucrose;
wherein the pharmaceutical composition has a pH of about 5;

11) about 5 mg/mL, about 10 mg/mL, about 20 mg/mL, about 25 mg/mL, about 50 mg/mL, about 80 mg/mL, or about 100 mg/mL FAP/CD40-binding molecule;

about 10 mM histidine-acetic acid buffer;
about 0.4 mg/mL polysorbate 80;
about 80 mg/mL sucrose;
wherein the pharmaceutical composition has a pH of about 5.2;

12) about 5 mg/mL, about 10 mg/mL, about 20 mg/mL, about 25 mg/mL, about 50 mg/mL, about 80 mg/mL, or about 100 mg/mL FAP/CD40-binding molecule;

about 10 mM histidine-acetic acid buffer;
about 0.4 mg/mL polysorbate 80;
about 80 mg/mL sucrose;
wherein the pharmaceutical composition has a pH of about 5.3;

13) about 5 mg/mL, about 10 mg/mL, about 20 mg/mL, about 25 mg/mL, about 50 mg/mL, about 80 mg/mL, or about 100 mg/mL FAP/CD40-binding molecule;

about 10 mM histidine-acetic acid buffer;
about 0.4 mg/mL polysorbate 80;
about 80 mg/mL sucrose;
wherein the pharmaceutical composition has a pH of about 5.5;

14) about 5 mg/mL, about 10 mg/mL, about 20 mg/mL, about 25 mg/mL, about 50 mg/mL, about 80 mg/mL, or about 100 mg/mL FAP/CD40-binding molecule;

about 10 mM histidine-acetic acid buffer;
about 0.6 mg/mL polysorbate 80;
about 80 mg/mL sucrose;
wherein the pharmaceutical composition has a pH of about 5;

15) about 5 mg/mL, about 10 mg/mL, about 20 mg/mL, about 25 mg/mL, about 50 mg/mL, about 80 mg/mL, or about 100 mg/mL FAP/CD40-binding molecule;

about 10 mM histidine-acetic acid buffer;
about 0.6 mg/mL polysorbate 80;
about 80 mg/mL sucrose;
wherein the pharmaceutical composition has a pH of about 5.2;

16) about 5 mg/mL, about 10 mg/mL, about 20 mg/mL, about 25 mg/mL, about 50 mg/mL, about 80 mg/mL, or about 100 mg/mL FAP/CD40-binding molecule;

about 10 mM histidine-acetic acid buffer;
about 0.6 mg/mL polysorbate 80;
about 80 mg/mL sucrose;
wherein the pharmaceutical composition has a pH of about 5.3;

17) about 5 mg/mL, about 10 mg/mL, about 20 mg/mL, about 25 mg/mL, about 50 mg/mL, about 80 mg/mL, or about 100 mg/mL FAP/CD40-binding molecule;

about 10 mM histidine-acetic acid buffer;
about 0.6 mg/mL polysorbate 80;
about 80 mg/mL sucrose;
wherein the pharmaceutical composition has a pH of about 5.5;

18) about 5 mg/mL, about 10 mg/mL, about 20 mg/mL, about 25 mg/mL, about 50 mg/mL, about 80 mg/mL, or about 100 mg/mL FAP/CD40-binding molecule;

about 10 mM histidine-acetic acid buffer;
about 0.8 mg/mL polysorbate 80;
about 80 mg/mL sucrose;
wherein the pharmaceutical composition has a pH of about 5;

19) about 5 mg/mL, about 10 mg/mL, about 20 mg/mL, about 25 mg/mL, about 50 mg/mL, about 80 mg/mL, or about 100 mg/mL FAP/CD40-binding molecule;

about 10 mM histidine-acetic acid buffer;
about 0.8 mg/mL polysorbate 80;
about 80 mg/mL sucrose;
wherein the pharmaceutical composition has a pH of about 5.2;

20) about 5 mg/mL, about 10 mg/mL, about 20 mg/mL, about 25 mg/mL, about 50 mg/mL, about 80 mg/mL, or about 100 mg/mL FAP/CD40-binding molecule;

about 10 mM histidine-acetic acid buffer;
about 0.8 mg/mL polysorbate 80;
about 80 mg/mL sucrose;
wherein the pharmaceutical composition has a pH of about 5.3;

21) about 5 mg/mL, about 10 mg/mL, about 20 mg/mL, about 25 mg/mL, about 50 mg/mL, about 80 mg/mL, or about 100 mg/mL FAP/CD40-binding molecule;

about 10 mM histidine-acetic acid buffer;
about 0.8 mg/mL polysorbate 80;
about 80 mg/mL sucrose;
wherein the pharmaceutical composition has a pH of about 5.5;

22) 1-45 mg/mL FAP/CD40-binding molecule, such as about 5 mg/mL, about 10 mg/mL, about 20 mg/mL, or about 25 mg/mL FAP/CD40-binding molecule;

about 10 mM histidine-hydrochloride buffer, such as a histidine-histidine hydrochloride buffer;
0.2 mg/mL-2 mg/mL polysorbate 80;
about 80 mg/mL sucrose;
wherein the pharmaceutical composition has a pH of 4.5-6;

23) 50-120 mg/mL FAP/CD40-binding molecule, such as about 50 mg/mL, about 80 mg/mL, or about 100 mg/mL FAP/CD40-binding molecule;

about 10 mM histidine acetate buffer, such as a histidine-acetic acid buffer;
0.2 mg/mL-2 mg/mL polysorbate 80;
about 80 mg/mL sucrose;
wherein the pharmaceutical composition has a pH of 4.5-6;

24) the pharmaceutical compositions of 1)-23), wherein the pharmaceutical compositions further comprise 4% w/v mannitol or 0.01% w/v ethylenediaminetetraacetic acid;
25) the pharmaceutical compositions of 1)-24) with a final volume of 1 mL, the volume being brought to 1 mL by using water for injection when required.

In another aspect, the present disclosure provides a pharmaceutical composition comprising a FAP/CD40-binding molecule at a low concentration (e.g., 1-45 mg/mL, about 5 mg/mL, about 10 mg/mL, about 20 mg/mL, or about 25 mg/mL) or a preparation method therefor, and the pharmaceutical composition is obtained by diluting a pharmaceutical composition comprising a FAP/CD40-binding molecule at a high concentration (e.g., 50-120 mg/mL, about 50 mg/mL, about 80 mg/mL, or about 100 mg/mL) with a diluent. In some embodiments, the diluent includes, but is not limited to, water, normal saline, and a glucose solution.

The pharmaceutical composition of the present disclosure already has sufficient stability for being prepared into a drug and can be stable after long-term storage.

In some embodiments, the pharmaceutical composition remains stable at 2-8 °C for at least 3 months, at least 6 months, at least 12 months, at least 18 months, at least 24 months, or at least 36 months. In some embodiments, the pharmaceutical composition remains stable at 25 °C for at least 3 months, at least 6 months, at least 12 months, at least 18 months, or at least 24 months. In some embodiments, the pharmaceutical composition remains stable at 40 °C for at least 7 days, at least 14 days, at least 28 days, at least 1 month, at least 3 months, at least 6 months, at least 12 months, at least 18 months, or at least 24 months.

The present disclosure provides a method for preparing the aforementioned pharmaceutical composition, which comprises the step of dissolving the FAP/CD40-binding molecule.

The pharmaceutical composition of the present disclosure may be further prepared into a freeze-dried formulation for convenience of drug delivery.

In certain embodiments, provided is the pharmaceutical composition according to any one of the above, wherein the pharmaceutical composition is a liquid formulation. In some embodiments, the solvent of the liquid formulation is water, normal saline, or a glucose solution.

The present disclosure also provides a freeze-dried formulation, wherein the freeze-dried formulation is capable of forming the pharmaceutical composition according to any one of the above upon reconstitution.

The present disclosure also provides a freeze-dried formulation, wherein the freeze-dried formulation is obtained by lyophilizing the pharmaceutical composition according to any one of the above.

The present disclosure provides a reconstituted solution, wherein the reconstituted solution is prepared by reconstituting the aforementioned freeze-dried formulation. In certain embodiments, the reconstituted solution is selected from the group consisting of, but not limited to, water for injection, normal saline, or a glucose solution.

The present disclosure also provides an article of manufacture, which comprises a container, wherein the container contains the aforementioned pharmaceutical composition, the aforementioned freeze-dried formulation, or the aforementioned reconstituted solution. In certain embodiments, the container is a tubular injection vial made of neutral borosilicate glass. In certain embodiments, the article of manufacture comprises a package insert.

The present disclosure also provides the pharmaceutical composition, the freeze-dried formulation, or the reconstituted solution of the freeze-dried formulation as a medicament for use in treating or ameliorating a disease or disorder.

Method for Treating Disease and Pharmaceutical Use

In some embodiments, the present disclosure provides a method for preventing and/or treating a cancer or tumor, which comprises administering to a patient or subject a prophylactically and/or therapeutically effective amount of the aforementioned pharmaceutical composition, freeze-dried formulation, or reconstituted solution of the freeze-dried formulation to inhibit tumor cell growth in the patient or subject. In some specific embodiments, the cancer is preferably, but is not limited to, a cancer that is responsive to immunotherapy.

In the above method or use, non-limiting examples of the cancer or tumor include lung cancer, ovarian cancer, colon cancer, rectal cancer, melanoma (e.g., metastatic malignant melanoma), renal cancer, bladder cancer, breast cancer, liver cancer, lymphoma, hematologic malignancies, head and neck cancer, glioma, gastric cancer, nasopharyngeal carcinoma, laryngeal cancer, cervical cancer, uterine body tumors, and osteosarcoma. Examples of other cancers that can be treated using the method of the present disclosure include: bone cancer, pancreatic cancer, skin cancer, prostate cancer, skin or intraocular malignant melanoma, uterine cancer, anal cancer, testicular cancer, fallopian tube cancer, endometrial cancer, vaginal cancer, vulvar cancer, Hodgkin's disease, non-Hodgkin's lymphoma, esophageal cancer, small intestine cancer, endocrine system cancer, thyroid cancer, parathyroid cancer, adrenal cancer, soft tissue sarcoma, urethral cancer, penile cancer, chronic or acute leukemia, including acute myeloid leukemia, chronic myeloid leukemia, acute lymphocytic leukemia, and chronic lymphocytic leukemia, childhood solid tumors, lymphocytic lymphoma, bladder cancer, renal or ureter cancer, renal pelvis cancer, central nervous system (CNS) tumor, primary CNS lymphoma, tumor angiogenesis, spinal tumor, brainstem neuroglioma, pituitary adenoma, Kaposi's sarcoma, epidermal carcinoma, squamous cell carcinoma, T cell lymphoma, and environmentally induced cancers, including asbestos-induced cancers, and combinations of the cancers. In some embodiments, the tumor or cancer described above is metastatic and/or advanced.

In addition, the present disclosure also provides a method for preventing and/or treating an infectious disease in a subject or patient, which comprises administering to the subject or patient the aforementioned pharmaceutical composition, freeze-dried formulation, or reconstituted solution of the freeze-dried formulation, such that the infectious disease in the subject is prevented and/or treated. In a way similar to the use for the cancer or tumor described above, individual use or use in combination with vaccines may be performed to stimulate immune responses to pathogens, toxins, and autoantigens. Examples of pathogens to which the treatment method can be particularly applied include pathogens for which no effective vaccine is currently available, or pathogens for which conventional vaccines are not fully effective. The pathogens include, but are not limited to, HIV, hepatitis viruses (A, B, and C), influenza viruses, herpes viruses, giardia, malaria, leishmania, *Staphylococcus aureus,* and *Pseudomonas aeruginosa.*

The pharmaceutical composition comprising the FAP/CD40-binding molecule of the present disclosure may be used for treating a patient in need of such treatment by parenteral administration. For the parenteral routes of administration, subcutaneous injection, intramuscular injection, or intravenous injection may be selected.

## BRIEF DESCRIPTION OF THE DRAWINGS

FIG. 1 shows the binding of the anti-FAP antibodies Ab9, Ab10, Ab14, and Ab15 to cell surface human FAP, with 28H1 as a positive control and the IgG1 isotype as a negative control.

FIG. 2 shows the binding of the anti-FAP antibodies Ab9, Ab10, Ab14, and Ab15 to cell surface mouse FAP, with 28H1 as a positive control and the IgG1 isotype as a negative control.

FIG. 3 shows the binding of the anti-CD40 antibodies A12 and A297 to Raji cell surface human CD40, with 9E5-SELFNS as a positive control and IgG1 as a negative control.

FIG. 4 shows the binding of the anti-CD40 antibodies A12 and A297 to HEK293 cell surface human CD40, with 9E5-SELFNS as a positive control and IgG1 as a negative control; the ordinate represents the agonistic activity percentage relative to 200 nM 9E5-SELFNS.

FIG. 5 shows the affinities of the humanized anti-CD40 antibodies A297_V1, A297_V2, A297_V3, and A297_V4 for HEK293 cells overexpressing human CD40, as measured by flow cytometry, with 9E5-SELFNS as a positive control and hIgG as a negative control.

FIG. 6 shows the Fc$\gamma$RIIb-mediated activation of HEK-Blue™CD40L cells by the humanized anti-CD40 antibodies A297_V1, A297_V2, A297_V3, and A297_V4, with 9E5-SELFNS as a positive control and hIgG as a negative control.

FIG. 7 shows a schematic diagram of the structures of anti-FAP/CD40 bispecific antibodies.

FIG. 8A shows the affinities of the anti-FAP/CD40 bispecific antibodies Ab10-A12V2-2, Ab10-A12V2-4, Ab10-A297V3-2, and Ab10-A297V3-4 for a stably transfected CHOK1/FAP strain highly expressing the human FAP antigen, as measured by flow cytometry. FIGs. 8B and 8C show the affinities of the aforementioned anti-FAP/CD40 bispecific antibodies for stably transfected CHOK1/FAP strains highly expressing the murine FAP antigen and the cynomolgus monkey FAP antigen, respectively, as measured by flow cytometry.

FIG. 9A shows the affinities of the anti-FAP/CD40 bispecific antibodies Ab10-A12V2-2, Ab10-A12V2-4, Ab10-A297V3-2, and Ab10-A297V3-4 for a stably transfected HEK-BlueTMCD40L strain highly expressing the human CD40 antigen, as measured by flow cytometry. FIG. 9B shows the affinities of the aforementioned anti-FAP/CD40 bispecific antibodies for HEK293 cells highly expressing the cynomolgus monkey CD40 antigen, as measured by flow cytometry.

FIG. 10 shows the affinities of the anti-FAP/CD40 bispecific antibodies Ab10-A297V3-2 and Ab10-A297V3-4 for the CD40 on immature human DCs, as measured by flow cytometry.

FIG. 11 shows the DC maturation-promoting effect of the anti-FAP/CD40 bispecific antibodies Ab10-A12V2-2, Ab10-A12V2-4, Ab10-A297V3-2, and Ab10-A297V3-4 of a concentration gradient in the absence of crosslinking of stably transfected CHOK1/FAP cells, with LPC, hIgG1, and the vehicle as controls.

FIG. 12 shows the DC maturation-promoting effect of the anti-FAP/CD40 bispecific antibodies Ab10-A12V2-2, Ab10-A12V2-4, Ab10-A297V3-2, and Ab10-A297V3-4 of a concentration gradient in the presence of crosslinking of stably transfected CHOK1/FAP cells, with LPC, hIgG1, and the vehicle as controls.

FIG. 13A shows tumor growth curves of mice after single-dose injections of the antibody Ab10-A297V3-2 or Ab10-A297V3-4 into mFAP-MC38 tumor-bearing hCD40 humanized mice. FIG. 13B shows the corresponding peripheral blood B cell activation in the mice. FIG. 13C shows curves of the corresponding changes in the body weight of the mice. FIG. 13D shows the corresponding changes in the platelet counts of the mice. FIG. 13E shows the corresponding influence on liver function in the mice.

FIG. 14A shows tumor growth curves of mice after multiple-dose injections of the antibody Ab10-A297V3-2 or Ab10-A297V3-4 into mFAP-MC38 tumor-bearing hCD40 humanized mice. FIG. 14B shows the corresponding peripheral blood B cell activation in the mice. FIG. 14C shows curves of the corresponding changes in the body weight of the mice. FIG. 14D shows the corresponding changes in the platelet counts of the mice. FIG. 14E shows the corresponding influence on liver function in the mice.

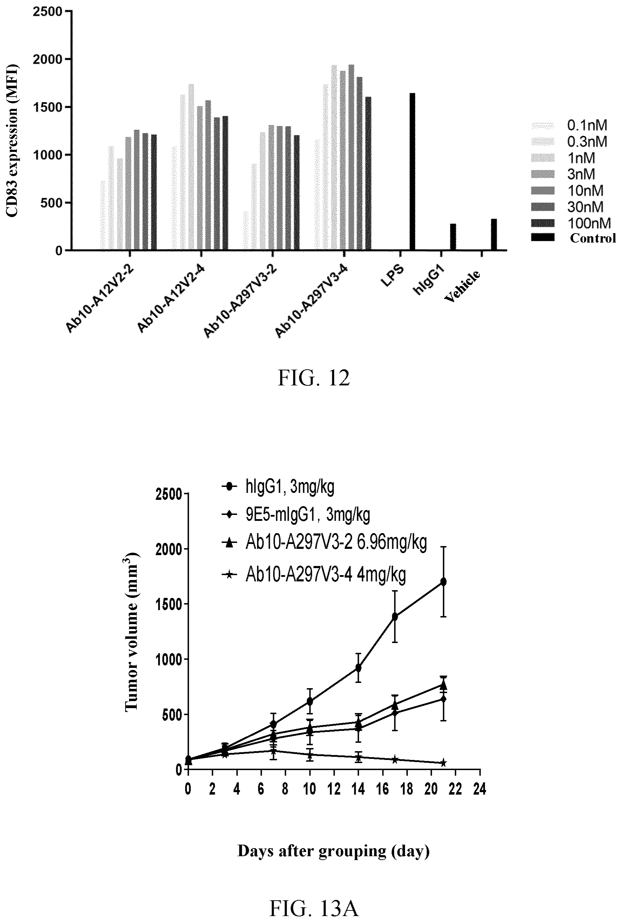

In FIGs. 13A to 14E, the P values were calculated relative to the control hIgG1, unless otherwise indicated. The statistical method Student' t-test was used. ns stands for no significant difference. * represents $p < 0.05$, ** represents $p < 0.01$, *** represents $p < 0.001$, and **** represents $p < 0.0001$.

## DETAILED DESCRIPTION

I. Terminology

In order to facilitate the understanding of the present disclosure, certain technical and scientific terms are specifically defined below. Unless otherwise explicitly defined herein, all other technical and scientific terms used herein have the meanings generally understood by those of ordinary skill in the art to which the present disclosure belongs. The three-letter and single-letter codes for amino acids used in the present disclosure are as described in J. biol. chem., 243, p3558 (1968).

"CD40" and "CD40 antigen" refer to an approximately 48 kD glycoprotein expressed on the surface of normal and neoplastic B cells, which acts as a receptor for signals involved in cellular proliferation and differentiation (Ledbetter et al., 1987, J. Immunol. 138:788-785). A cell that endogenously expresses CD40 is any cell characterized by the surface expression of CD40, including but not limited to, normal and neoplastic B cells, interdigitating cells, basal epithelial cells, carcinoma cells, macrophages, endothelial cells, follicular dendritic cells, tonsil cells, and bone marrow-derived plasma cells. In the present disclosure, "CD40" refers to any native CD40 derived from any vertebrate, including mammals such as primates (e.g., humans) and rodents (e.g., mice and rats), unless otherwise indicated. A cDNA molecule encoding CD40 has been isolated from a library prepared from the Burkitt lymphoma cell line Raji (Stamenkovic et al., 1989, EMBO J. 8:1403). Sequence information can be found in Table 8 of the present disclosure. In the present disclosure, "CD40" encompasses full-length unprocessed CD40 as well as any form of CD40 that results from processing in a cell, and further encompasses naturally occurring variants of CD40, such as splice variants or allelic variants. In one embodiment, the CD40-binding molecule of the present disclosure is capable of specifically binding to human, mouse, and/or cynomolgus monkey CD40.

"Fibroblast activation protein (FAP)" and "FAP antigen", also known as prolyl endopeptidase FAP or seprase (EC 3.4.21), refer to any natural FAP derived from any vertebrate, including mammals, such as primates (e.g., humans), non-human primates (e.g., cynomolgus monkeys), and rodents (e.g., mice and rats), unless otherwise indicated. In the present disclosure, "FAP" encompasses full-length unprocessed FAP as well as any form of FAP that results from processing in a cell, and further encompasses naturally occurring variants of FAP, such as splice variants or allelic variants. In one embodiment, the FAP-binding molecule of the present disclosure is capable of specifically binding to human, mouse, and/or cynomolgus monkey FAP. The amino acid sequence of human FAP is shown under UniProt (www.uniprot.org) accession No. Q12884 (version 149, SEQ ID NO: 2), or NCBI (www.ncbi.nlm.nih.gov/) RefSeq NP_004451.2, or GeneBank accession No. AAC51668. The extracellular domain (ECD) of human FAP extends from amino acid position 26 to 760. Amino acid sequences such as the ECD of His-tagged human FAP are shown in Table 2 of the present disclosure. The amino acid sequence of mouse FAP is shown under UniProt accession No. P97321 (version 126, SEQ ID NO: 143), or NCBI RefSeq NP_032012.1. The extracellular domain (ECD) of mouse FAP extends from amino acid position 26 to 761. In some embodiments, the FAP-binding molecule of the present disclosure binds to the extracellular domain of FAP.

"Antibody" is used in the broadest sense and encompasses a variety of antibody structures, including, but not limited to monoclonal antibodies, polyclonal antibodies, monospecific antibodies, multispecific antibodies (e.g., bispecific antibodies), full-length antibodies, and antibody fragments (or antigen-binding fragments, or antigen-binding moieties) so long as they exhibit the desired antigen-binding activity. An antibody may refer to an immunoglobulin that is a four-peptide-chain structure formed by linking two identical heavy chains and two identical light chains by interchain disulfide bonds. The heavy chain constant regions of immunoglobulins differ in their amino acid composition and arrangement, and thus in their antigenicity. Accordingly, immunoglobulins can be divided into five classes, otherwise called isotypes of immunoglobulins, namely IgM, IgD, IgG, IgA, and IgE, with their corresponding heavy chains being μ chain, δ chain, γ chain, α chain, and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3, and IgG4. Light chains are divided into κ or λ chains according to differences in the constant regions. Each of the five classes of Ig may have a κ chain or λ chain. In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (CDRs) and 4 framework regions (FRs) with relatively conservative sequences. The 3 hypervariable regions determine the specificity of the antibody and thus are also known as complementarity-determining regions (CDRs). Each of the light chain variable regions (VLs) and the heavy chain variable regions (VHs) consists of 3 CDRs and 4 FRs arranged from the amino-terminus to the carboxyl-terminus as follows: FR1, CDR1, FR2, CDR2, FR3, CDR3, and FR4. The 3 CDRs of the light chain refer to LCDR1, LCDR2, and LCDR3, and the 3 CDRs of the heavy chain refer to HCDR1, HCDR2, and HCDR3.

"Bispecific antibody" encompasses antibodies (including antibodies or antigen-binding fragments thereof, such as single-chain antibodies) capable of specifically binding to two different antigens or at least two different antigenic epitopes of the same antigen. Typical structural models of bispecific antibodies include bispecific antibodies such as KiH, CrossMAb, Triomab quadroma, FcΔAdp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP-DART, CODV-Fab-TL, HLE-BiTE, F(ab)2-CrossMAb, IgG-(scFv)2, Bs4Ab, DVD-Ig, Tetravalent-DART-Fc, (scFv)4-Fc, CODV-Ig, mAb2, and F(ab)4-CrossMAb (see Aran F. Labrijn et al., Nature Reviews Drug Discovery, volume 18, pages 585-608 (2019); Chen S1 et al., J Immunol Res., Feb. 11, 2019; 2019: 4516041).

For the determination or definition of CDRs, the deterministic depiction of CDRs and identification of residues comprising antigen-binding sites of an antibody can be accomplished by resolving the structure of the antibody and/or resolving the structure of the antibody-ligand complex. This can be accomplished by any one of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition.

The Kabat numbering scheme is a standard for numbering residues in antibodies and is generally used to identify CDRs (see, e.g., Johnson & Wu, 2000, Nucleic Acids Res., 28: 214-8). The Chothia numbering scheme is similar to the Kabat numbering scheme, except that it takes into account the positions of certain structural loop regions (see, e.g., Chothia et al., 1986, J. Mol. Biol., 196: 901-17; Chothia et al., 1989, Nature, 342: 877-83). The AbM numbering scheme adopts a computer program integration suite for modeling antibody structures manufactured by Oxford Molecular Group (see, e.g., Martin et al., 1989, Proc Natl Acad Sci (USA), 86: 9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3: 194-198). The contact definition is based on the analysis of the available complex crystal structures (see, e.g., MacCallum et al., 1996, J. Mol. Biol., 5: 732-45). In the conformational definition, the positions of the CDRs can be identified as residues that contribute enthalpy to the antigen binding (see, e.g., Makabe et al., 2008, Journal of Biological Chemistry, 283: 1156-1166). In addition, other CDR boundary definitions may not strictly follow one of the methods described above, but still overlap with at least a portion of the Kabat CDRs, although they may be shortened or lengthened based on predictions or experimental results that a particular residue or a particular group of residues do not significantly affect the antigen binding. As used in the present disclosure, a CDR may refer to a CDR defined by any method known in the art, including combinations of methods. The correspondence between the various numbering schemes is well known to those skilled in the art, and examples are shown in Table 1 below.

Table 1. The relationships between CDR numbering schemes

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

The CDR amino acid residues of the VL and VH regions of the antibody of the present disclosure accord with the known Kabat numbering scheme in terms of number and positions.

"Domain" of a polypeptide or protein refers to a folded protein structure that is capable of maintaining its tertiary structure independently of the rest of the protein. In general, a domain is responsible for a single functional property of a protein, and in many cases may be added, deleted, or transferred to other proteins without loss of functions of the rest of the protein and/or the domain of the protein.

"Immunoglobulin domain" refers to a globular region of an antibody chain (e.g., a chain of a conventional antibody with a four-peptide-chain structure or a heavy-chain antibody) or a polypeptide essentially consisting of such globular regions. The immunoglobulin domain is characterized in that it retains the immunoglobulin fold characteristic of an antibody molecule, and it consists of a 2-layer sandwich of about 7 antiparallel β-strands arranged in two β-sheets, optionally stabilized by a conserved disulfide bond.

"Immunoglobulin variable domain" refers to an immunoglobulin domain essentially consisting of four "framework regions" referred to in the art and hereinafter as "framework region 1" or "FR1", "framework region 2" or "FR2", "framework region 3" or "FR3", and "framework region 4" or "FR4", where the framework regions are spaced apart by three "complementarity-determining regions" or "CDRs" referred to in the art and hereinafter as "complementarity-determining region 1" or "CDR1", "complementarity-determining region 2" or "CDR2", and "complementarity-determining region 3" or "CDR3". Thus, the general structure or sequence of an immunoglobulin variable domain can be expressed as follows: FR1-CDR1-FR2-CDR2-FR3-CDR3-FR4. Immunoglobulin variable domains possess specificity for an antigen by virtue of having an antigen-binding site.

"Antibody framework (FR)" refers to a portion of a variable domain, which serves as a framework for the antigen-binding loops (CDRs) of the variable domain.

"Immunoglobulin single variable domain" is generally used to refer to an immunoglobulin variable domain (which may be a heavy or light chain domain, including a VH, VHH, or VL domain) that can form a functional antigen-binding site without interacting with other variable domains (e.g., without VH/VL interactions as are required between the VH and VL domains of conventional four-chain monoclonal antibodies). Examples of "immunoglobulin single variable domains" include nanobodies (including VHHs, humanized VHHs, and/or camelized VHs, e.g., camelized human VHs), IgNARs, domains, (single-domain) antibodies as VH domains or derived from VH domains (such as dAbs™) and (single-domain) antibodies as VL domains or derived from VL domains (such as dAbs™). Immunoglobulin single variable domains based on and/or derived from heavy chain variable domains (such as VH or VHH domains) are generally preferred. A specific example of an immunoglobulin single variable domain is a "VHH domain" (or "VHH" for short) as defined below.

"VHH domain", also known as heavy-chain single-domain antibody, VHH, $V_HH$ domain, VHH antibody fragment, VHH antibody, or nanobody, is a variable domain of an antigen-binding immunoglobulin known as a "heavy-chain antibody" (i.e., "an antibody devoid of light chains") (Hamers-Casterman C, Atarhouch T, Muyldermans S, Robinson G, Hamers C, Songa EB, Bendahman N, Hamers R., "Naturally occurring antibodies devoid of light chains"; Nature 363, 446-448 (1993)). The term "VHH domain" is used to distinguish the variable domain from the heavy chain variable domain (which is referred to in the present disclosure as a "VH domain") and the light chain variable domain (which is referred to in the present disclosure as a "VL domain") present in conventional antibodies with a four-peptide-chain structure. VHH domains specifically bind to an epitope without the need for an additional antigen-binding domain (as opposed to the VH or VL domain in conventional antibodies with a four-peptide-chain structure, in which case the epitope is recognized by the VL domain together with the VH domain). A VHH domain is a small, stable, and efficient antigen recognition unit formed by a single immunoglobulin domain. The terms "heavy-chain single-domain antibody", "VHH domain", "VHH", "$V_HH$ domain", "VHH antibody fragment", "VHH antibody", "Nanobody®", and "Nanobody® domain" ("Nanobody" is a trademark of Ablynx N.V., Ghent, Belgium) are used interchangeably. "VHH domains" include, but are not limited to, natural antibodies produced by camelids, antibodies produced by camelids and then humanized, or antibodies obtained by screening with phage display techniques. The total number of amino acid residues in a VHH domain will usually be in the range of 110 to 120, often between 112 and 115. However, it should be noted that smaller and longer sequences may also be suitable for the purposes described in the present disclosure. Methods for obtaining VHHs that bind to a particular antigen or epitope have been previously disclosed in the following documents: R. van der Linden et al., Journal of Immunological Methods, 240 (2000) 185-195; Li et al., J Biol Chem., 287 (2012) 13713-13721; Deffar et al., African Journal of Biotechnology Vol. 8 (12), pp.2645-2652, 17 June, 2009, and WO94/04678.

As is well known in the art for VH domains and for VHH domains, the total number of amino acid residues in each of the CDRs may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, in general, the numbering according to Kabat may or may not correspond to the actual numbering of the amino acid residues in the actual sequence. Other numbering systems or numbering schemes include Chothia, IMGT, and AbM.

"Humanized antibody", also known as CDR-grafted antibody, refers to an antibody produced by grafting non-human CDR sequences into the framework of variable regions of a human antibody. Chimeric antibodies, due to their significant non-human protein content, can induce a strong immune response. This issue can be overcome by using humanized antibodies. To avoid the decrease in activity caused by the decrease in immunogenicity, the variable regions of a fully human antibody can be subjected to minimum reverse mutation to maintain activity. Examples of "humanization" include "humanization" of VHH domains derived from camelidae by replacing one or more amino acid residues in the amino acid sequence of the original VHH sequence with one or more amino acid residues present at the corresponding positions in a VH domain of a human conventional antibody with a four-peptide-chain structure (also referred to in the present disclosure as "sequence optimization"; in addition to humanization, "sequence optimization" may also encompass other modifications to the sequence by one or more mutations providing improved properties of the VHH, such as removal of potential post-translational modification sites). The humanized VHH domain may contain one or more fully human framework region sequences. Additionally, in order to avoid the decrease in activity caused by the decrease in immunogenicity, the framework sequence in the human antibody variable region can be subjected to minimum reverse mutation or back mutation to maintain activity.

"Fully human antibody" includes antibodies having variable and constant regions derived from human germline immunoglobulin sequences. The fully human antibody of the present disclosure may include amino acid residues not encoded by human germline immunoglobulin sequences (e.g., mutations introduced by random or site-specific mutagenesis *in vitro* or by somatic mutations *in vivo*). "Fully human antibody" does not include "humanized antibodies".

Typically, the CD40-binding molecule or the FAP-binding molecule of the present disclosure will bind to the antigen to be bound (i.e., CD40 or FAP) with a dissociation constant ($K_D$) of preferably $10^{-7}$ to $10^{-10}$ mol/L (M), more preferably $10^{-8}$ to $10^{-10}$ mol/L, and even more preferably $10^{-9}$ to $10^{-10}$ or less, and/or with an association constant (KA) of at least $10^{-7}$ M, preferably at least $10^{-8}$ M, more preferably at least $10^{-9}$ M, and more preferably at least $10^{-10}$ M, as measured in a Biacore, KinExA, or Fortibio assay. Any $K_D$ value greater than $10^{-4}$ M is generally considered to indicate non-specific binding. Specific binding of an antigen-binding protein to an antigen or epitope can be measured in any suitable manner known, including, for example, surface plasmon resonance (SPR) assay, Scatchard assay, and/or competitive binding assay (e.g., radioimmunoassay (RIA), enzyme immunoassay (EIA), and sandwich competitive assay) described in the present disclosure.

"Compete", when used in a case where antigen-binding proteins (e.g., neutralizing antigen-binding proteins or neutralizing antibodies) compete for the same epitope, refers to the competition between the antigen-binding proteins, which is measured by the following assays in which a test antigen-binding protein (e.g., an antibody or an immunologically functional fragment thereof) prevents or inhibits (e.g., reduces) specific binding of a reference antigen-binding protein (e.g., a ligand or a reference antibody) to a common antigen (e.g., CD40 or a fragment thereof). Numerous types of competitive binding assays are available for determining whether an antigen-binding protein competes with another, such as solid phase direct or indirect radioimmunoassay (RIA), solid phase direct or indirect enzyme immunoassay (EIA), and sandwich competition assay (see, e.g., Stahli et al., 1983, Methods in Enzymology 9: 242-253); solid phase direct biotin-avidin EIA (see, e.g., Kirkland et al., 1986, J. Immunol. 137: 3614-3619), solid phase direct labeled assay, and solid phase direct labeled sandwich assay (see, e.g., Harlow and Lane, 1988, Antibodies: A Laboratory Manual, Cold Spring Harbor Press); solid phase direct labeled RIA with I-125 label (see, e.g., Morel et al., 1988, Molec. Immunol. 25: 7-15); solid phase direct biotin-avidin EIA (see, e.g., Cheung, et al., 1990, Virology 176: 546-552); and direct labeled RIA (Moldenhauer et al., 1990, Scand. J. Immunol. 32: 77-82). Generally, the assay relates to the use of a purified antigen (which is on a solid surface or cell surface) that can bind to an unlabeled detection antigen-binding protein and a labeled reference antigen-binding protein. Competitive inhibition is measured by measuring the amount of label bound to the solid surface or the cell in the presence of the test antigen-binding protein. Generally, the test antigen-binding protein exists in an excessive amount. Antigen-binding proteins identified by competitive assays (competitive antigen-binding proteins) include: antigen-binding proteins that bind to the same epitope as the reference antigen-binding protein; and antigen-binding proteins that bind to an epitope that is sufficiently close to the epitope to which the reference antigen-binding protein binds, where the two epitopes spatially hinder each other from binding. Generally, when the competitive antigen-binding protein exists in an excessive amount, the specific binding of the reference antigen-binding protein to the common antigen will be inhibited (e.g., reduced) by at least 40%-45%, 45%-50%, 50%-55%, 55%-60%, 60%-65%, 65%-70%, 70%-75%, or 75% or more. In certain instances, the binding is inhibited by at least 80%-85%, 85%-90%, 90%-95%, 95%-97%, or 97% or more.

Antibodies can be competitively screened for binding to the same epitope using conventional techniques known to those skilled in the art. For example, competition and cross-competition studies can be performed to obtain antibodies that compete or cross-compete with one another for binding to an antigen. A high-throughput method for obtaining antibodies that bind to the same epitope based on their cross-competition is described in International Patent Publication No. WO03/48731. Therefore, antibodies that compete for binding to the same epitope on CD40 or FAP with the antibody molecules of the present disclosure can be obtained by conventional techniques known to those skilled in the art.

"CD40-binding molecule" refers to any protein capable of specifically binding to CD40 or any molecule comprising the protein. The CD40-binding molecule may include antibodies against CD40 as defined in the present disclosure or conjugates thereof. The CD40-binding molecule further encompasses immunoglobulin superfamily antibodies (IgSF) or CDR-grafted molecules. The "CD40-binding molecule" of the present disclosure may comprise at least one immunoglobulin single variable domain (such as a VHH) that binds to CD40. In some embodiments, the "CD40-binding molecule" may comprise 2, 3, 4, or more immunoglobulin single variable domains (such as VHHs) that bind to CD40. The CD40-binding molecule of the present disclosure may further comprise, in addition to the immunoglobulin single variable domain of CD40, a linker and/or a moiety with effector function, such as a half-life extending moiety (e.g., an immunoglobulin single variable domain that binds to serum albumin), and/or a fusion partner (such as serum albumin) and/or a conjugated polymer (such as PEG) and/or an Fc region. In some embodiments, the "CD40-binding molecule" of the present disclosure further encompasses bispecific/multispecific antibodies comprising immunoglobulins that bind to different antigens (e.g., a first antibody that binds to a first antigen (e.g., CD40) and a second antibody that binds to a second antigen (e.g., FAP), optionally a third antibody that binds to a third antigen, and further optionally a fourth antibody that binds to a fourth antigen).

"FAP-binding molecule" refers to any protein capable of specifically binding to FAP or any molecule comprising the protein. The FAP-binding molecule may include antibodies against FAP as defined in the present disclosure or conjugates thereof.

"FAP/CD40-binding molecule" refers to any protein capable of specifically binding to CD40 and FAP or any molecule comprising the protein. The FAP/CD40-binding molecule may include antibodies against CD40 and FAP as defined in the present disclosure or conjugates thereof.

"Bind to CD40" or "bind CD40" refers to being able to interact with CD40 or an epitope thereof, where the CD40 or the epitope thereof may be derived from humans. "Bind to FAP" or "bind FAP" refers to being able to interact with FAP or an epitope thereof, where the FAP or the epitope thereof may be derived from humans. "Antigen-binding site" in the present disclosure refers to a discontinuous three-dimensional spatial site on an antigen that is recognized by the antibody of the present disclosure.

"Antigen" refers to a molecule used for immunization of an immunocompetent vertebrate to produce an antibody that recognizes the antigen or to screen an expression library (e.g., particularly phage, yeast, or ribosome display library). In the present disclosure, the antigen is defined in a broader sense, and includes a target molecule that is specifically recognized by the antibody, and a portion or a mimic of a molecule used in an immunization process for producing the antibody or in library screening for selecting the antibody. For example, for the antibody that binds to human CD40 of the present disclosure, monomers and multimers (e.g., dimers, trimers, etc.) of human CD40, and truncated variants and other variants of human CD40 are all referred to as antigens.

"Epitope" refers to a site on an antigen to which an immunoglobulin or an antibody binds. An epitope may be formed from contiguous amino acids, or non-contiguous amino acids juxtaposed by tertiary folding of the protein. An epitope formed from contiguous amino acids is generally retained after exposure to a denaturing solvent, while an epitope formed by tertiary folding is generally lost after a denaturing solvent treatment. An epitope generally comprises, for example, at least 3-15 amino acids in a distinctive spatial conformation. Methods for determining what epitope is bound by a given antibody are well known in the art and include an immunoblotting assay, an immunoprecipitation assay, and the like. Methods for determining the spatial conformation of an epitope include techniques in the art and techniques described in the present disclosure, such as X-ray crystallography and two-dimensional nuclear magnetic resonance.

"Specific binding" or "selective binding" refers to the binding of an antibody to an epitope on a predetermined antigen. For example, an antibody binds to a predetermined antigen or epitope thereof with an equilibrium dissociation constant ($K_D$) of about less than $10^{-7}$ M or even less and with an affinity that is at least twice as high as its affinity for binding to a non-specific antigen other than the predetermined antigen or the epitope thereof (or non-specific antigens other than closely related antigens, e.g., BSA, etc.), when measured by surface plasmon resonance (SPR) techniques in an instrument using human CD40 or an epitope thereof as an analyte and the antibody as a ligand. "Antigen-recognizing antibody" is used interchangeably in the present disclosure with "specifically bound antibody".

"Binding affinity" or "affinity" is used in the present disclosure as a measure of the strength of a non-covalent interaction between two molecules (e.g., an antibody or a portion thereof and an antigen). The binding affinity between two molecules can be quantified by determining the dissociation constant (KD). KD can be determined by measuring the kinetics of complex formation and dissociation using, for example, the surface plasmon resonance (SPR) method (Biacore). The rate constants corresponding to the association and dissociation of a monovalent complex are referred to as the association rate constant ka (or kon) and the dissociation rate constant kd (or koff), respectively. $K_D$ is related to ka and kd by the equation $K_D = kd/ka$. The value of the dissociation constant can be determined directly by well-known methods and can be calculated by methods such as those described by Caceci et al. (1984, Byte 9: 340-362) even for complex mixtures. For example, $K_D$ can be determined by using a dual filtration nitrocellulose filter binding assay such as that disclosed by Wong & Lohman (1993, Proc. Natl. Acad. Sci. USA 90: 5428-5432). Other standard assays for evaluating the binding ability of an antibody to a target antigen are known in the art and include, for example, ELISA, western blot, RIA, and flow cytometry, as well as other assays exemplified elsewhere in the present disclosure. The binding kinetics and binding affinity of the antibody can also be evaluated by standard assays known in the art, such as surface plasmon resonance (SPR), e.g., by using the Biacore™ system or KinExA. The binding affinities associated with different molecular interactions, e.g., the binding affinities of different antibodies for a given antigen, can be compared by comparing the $K_D$ values of antibody/antigen complexes. Similarly, the specificity of an interaction can be evaluated by determining and comparing the $K_D$ value for the interaction of interest (e.g., a specific interaction between an antibody and an antigen) with the $K_D$ value for an interaction not of interest (e.g., a control antibody known not to bind to CD40).

"Conservative replacement" refers to replacement by another amino acid residue having similar properties to the original amino acid residue. For example, lysine, arginine, and histidine have similar properties in that they have basic side chains, and aspartic acid and glutamic acid have similar properties in that they have acidic side chains. Additionally, glycine, asparagine, glutamine, serine, threonine, tyrosine, cysteine, and tryptophan have similar properties in that they have uncharged polar side chains, and alanine, valine, leucine, threonine, isoleucine, proline, phenylalanine, and methionine have similar properties in that they have nonpolar side chains. In addition, tyrosine, phenylalanine, tryptophan, and histidine have similar properties in that they have aromatic side chains. Thus, it will be apparent to those skilled in the art that even when an amino acid residue in a group exhibiting similar properties as described above is replaced, it will not exhibit a particular change in properties.

"Homology", "identity", or "sequence identity" refers to sequence similarity between two polynucleotide sequences or between two polypeptides. When positions in two compared sequences are occupied by identical nucleotides or amino acid monomers, e.g., if the position of each of two DNA molecules is occupied by an identical nucleotide, the molecules are homologous at that position. The homology percentage between two sequences is a function of the number of matching or homologous positions shared by the two sequences divided by the number of positions compared × 100%. For example, if 6 out of 10 positions are matched or homologous when two sequences are optimally aligned, the two sequences are 60% homologous. In general, when two sequences are aligned, a comparison is performed to obtain the maximum homology percentage. "Inhibition" and "blocking" are used interchangeably and encompass both partial and complete inhibition/blocking. "Inhibition of growth" (e.g., involving cells) is intended to include any measurable reduction in cell growth.

"Agonistic activity", "agonist activity", or "agonism" refers to the function of a substance as an agonist. The binding of an agonist to a cell receptor initiates a reaction or activity that is similar to or the same as that initiated by the receptor's natural ligand. For example, a CD40 agonist or CD40 agonist antibody can induce any or all of the following responses: cell proliferation and/or differentiation; up-regulation of intercellular adhesion via such molecules as ICAM-1, E-selectin, and VCAM; secretion of pro-inflammatory cytokines such as IL-1, IL-6, IL-8, IL-12, and TNF; signal transduction through the CD40 receptor by such pathways as TRAF (e.g., TRAF2 and/or TRAF3), MAP kinases such as NIK (NF-κB inducing kinase), 1-κB kinases (IKKα/β), transcription factor NF-κB, Ras and the MEK/ERK pathway, the PI3K/Akt pathway, and the P38 MAPK pathway; transduction of anti-apoptotic signals by such molecules as XIAP, Mcl-1, and BCLx; B and/or T cell memory generation: B cell antibody production; B cell isotype switching; up-regulation of cell surface expression of MHC class II and CD80/86, and the like.

"CD40-related disease" or "CD40-related disorder" refers to a disorder in which cells expressing CD40 are modified or eliminated. These cells include CD40-expressing cells demonstrating abnormal proliferation or CD40-expressing cells that are associated with cancerous or malignant growth. More specific examples of cancers that demonstrate abnormal expression of the CD40 antigen include B lymphoblastoid cells, Burkitt's lymphoma, multiple myeloma, T cell lymphomas, Kaposi's sarcoma, osteosarcoma, epidermal and endothelial tumors, pancreatic cancer, lung cancer, breast cancer, ovarian cancer, colon cancer, prostate cancer, head and neck cancer, skin cancer (melanoma), bladder cancer, and renal cancer. Such disorders include, but are not limited to, leukemias, lymphomas (including B cell lymphoma and non-Hodgkin's lymphoma), multiple myeloma, Waldenstrom's macroglobulinemia; solid tumors, including sarcomas, such as osteosarcoma, Ewing sarcoma, malignant melanoma, adenocarcinoma (including ovarian adenocarcinoma), Kaposi's sarcoma/Kaposi's tumor, and squamous cell carcinoma.

"Proliferative disease" refers to a disorder associated with a certain degree of abnormal cell proliferation. In one embodiment, the proliferative disorder is a cancer.

"Cancer", "cancerous", "proliferative disorder", and "tumor" are not mutually exclusive when referred to in the present disclosure.

"Preventing a cancer" refers to delaying, inhibiting, or preventing the onset of the cancer in a subject in which the onset of oncogenesis or tumorigenesis has not been evidenced but a predisposition toward the cancer has been identified, as determined, for example, by genetic screening or otherwise. The term also encompasses treating a subject having a premalignant disorder to stop the progression of, or cause regression of, the premalignant disorder towards malignancy.

"Optional" or "optionally" means that the event or circumstance subsequently described may, but does not necessarily, occur, and this description includes instances where the event or circumstance occurs or does not occur. For example, "optionally comprising mannitol or ethylenediaminetetraacetic acid" means that it may, but does not necessarily, be present in the pharmaceutical composition.

The term "about" or "approximately" as used herein means that a numerical value is within an acceptable error range for the particular value determined by one of ordinary skill in the art, and the numerical value depends in part on how the value is measured or determined (i.e., the limits of the measurement system). For example, "about" may mean a standard deviation within 1 or more than 1 in each practice in the art. Alternatively, "about" or "substantially comprise" may mean a range of up to ±30%; for example, a pH of about 5.5 means a pH of 5.5±1.65. Furthermore, particularly for biological systems or processes, the term may mean up to an order of magnitude or up to 5-fold of a numerical value. Unless otherwise specified, when a specific value is provided in the present application and claims, the meaning of "about" or "substantially comprise" should be assumed to be within an acceptable error range for that specific value.

"Buffer" refers to a buffer that resists changes in pH by the action of its acid-base conjugate components. Examples of buffers that control the pH in an appropriate range include tris(hydroxymethyl)aminomethane (Tris), acetate, succinate, gluconate, histidine salt, oxalate, lactate, phosphate, citrate, tartrate, fumarate, glycylglycine, and other organic acid buffers.

"Acetate buffer" is a buffer comprising acetate ions. Examples of acetate buffers include acetic acid-sodium acetate, histidine-histidine acetate, acetic acid-potassium acetate, acetic acid-calcium acetate, acetic acid-magnesium acetate, and the like. The preferred acetate buffer is acetic acid-sodium acetate.

"Succinate buffer" is a buffer comprising succinate ions. Examples of succinate buffers include succinic acid-sodium succinate, succinic acid-potassium succinate, succinic acid-calcium succinate, and the like. The preferred succinate buffer is succinic acid-sodium succinate. Illustratively, the succinic acid-sodium succinate may be prepared from succinic acid and sodium hydroxide, or from succinic acid and sodium succinate.

"Histidine salt buffer" is a buffer comprising histidine ions. Examples of histidine salt buffers include a histidine-hydrochloride buffer, a histidine-acetate buffer, a histidine-phosphate buffer, a histidine-sulfate buffer, and the like, and the histidine-hydrochloride buffer or histidine-acetate buffer is preferred. The histidine-acetate buffer is prepared from histidine and acetic acid, and the histidine-hydrochloride buffer is prepared from histidine and histidine hydrochloride, or histidine and hydrochloric acid.

"Phosphate buffer" is a buffer comprising phosphate ions. Examples of phosphate buffers include disodium hydrogen phosphate-sodium dihydrogen phosphate, disodium hydrogen phosphate-potassium dihydrogen phosphate, disodium hydrogen phosphate-citric acid, and the like. The preferred phosphate buffer is disodium hydrogen phosphate-sodium dihydrogen phosphate.

"Pharmaceutical composition" refers to a mixture comprising one or more of the antibodies described herein and other chemical components, and the other components are, for example, physiological/pharmaceutically acceptable carriers and excipients. The purpose of the pharmaceutical composition is to maintain the stability of the active ingredient and promote the administration to an organism, which facilitates the absorption of the active ingredient, thereby exerting biological activity.

In the present disclosure, "pharmaceutical composition" and "formulation" are not mutually exclusive.

Unless otherwise specified, the solvent in the pharmaceutical composition described in the present disclosure in solution form is water.

"Freeze-dried formulation" refers to a formulation or a pharmaceutical composition obtained by lyophilizing a pharmaceutical composition or a formulation in liquid or solution form *in vacuo.*

The pharmaceutical composition described in the present disclosure can achieve a stable effect, that is, the FAP/CD40-binding molecule in the pharmaceutical composition substantially retains its physical and/or chemical stability and/or biological activity after storage; for example, the pharmaceutical composition substantially retains its physical and chemical stability as well as its biological activity after storage. The storage period is generally selected based on a predetermined shelf life of the pharmaceutical composition. There are a variety of analytical techniques currently available for measuring protein stability, and the stability after storage for a selected period of time at a selected temperature can be measured.

A stable pharmaceutical antibody formulation is one in which no significant change is observed under the following conditions: storage at refrigeration temperature (2-8 °C) for at least 3 months, at least 6 months, at least 1 year, at least 2 years, or at most 2 years. In addition, stable liquid formulations include liquid formulations that exhibit desirable features after storage at 25 °C for periods including 1 month, 3 months, and 6 months, or storage at 40 °C for periods including 1 month. Typical acceptable criteria for stability are as follows: typically, no more than about 10%, preferably no more than about 5%, of antibody monomer is degraded as measured by SEC-HPLC. The pharmaceutical antibody formulation is colorless, or clear to slightly opalescent, by visual analysis. The concentration, pH, and osmolality of the formulation have a change of no more than $\pm 10\%$. Typically, clippings of no more than about 10%, preferably no more than about 5%, are observed. Typically, aggregation of no more than about 10%, preferably no more than about 5%, is formed.

An antibody "retains its physical stability" in a pharmaceutical formulation if it shows no significant increase in aggregation, precipitation, and/or denaturation upon visual inspection of color and/or clarity, or as determined by UV light scattering, size exclusion chromatography (SEC), and dynamic light scattering (DLS). Changes in protein conformation can be evaluated by fluorescence spectroscopy (which determines the protein tertiary structure) and by FTIR spectroscopy (which determines the protein secondary structure).

An antibody "retains its chemical stability" in a pharmaceutical formulation if it shows no significant chemical change. Chemical stability can be evaluated by detecting and quantifying chemically changed proteins. Degradation processes that often change the chemical structure of proteins include hydrolysis or clipping (assessed by methods such as size exclusion chromatography and SDS-PAGE), oxidation (assessed by methods such as peptide mapping in combination with mass spectroscopy or MALDI/TOF/MS), deamidation (assessed by methods such as ion-exchange chromatography, capillary isoelectric focusing, peptide mapping, and isoaspartic acid determination), and isomerization (assessed by isoaspartic acid content determination, peptide mapping, etc.). An antibody "retains its biological activity" in a pharmaceutical formulation if the biological activity of the antibody at a given time is within a predetermined range of the biological activity exhibited during the preparation of the pharmaceutical formulation. The biological activity of an antibody can be determined, for example, by an antigen-binding assay.

"Administrating", "giving", and "treating", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs, or biological fluids. "Administering", "giving", and "treating" can refer to, for example, therapeutic, pharmacokinetic, diagnostic, research, and experimental methods. The treatment of cells comprises contacting a reagent with cells and contacting the reagent with a fluid, where the fluid is in contact with the cells. "Administering", "giving", and "treating" also refer to treating, e.g., cells by reagents, diagnosis, or binding compositions, or by another cell *in vitro* and *ex vivo.* "Treating", when applied to humans, veterinary, or research subjects, refers to therapeutic treatment, preventive or prophylactic measures, and research and diagnostic applications.

"Treating" or "treatment" refers to administering a therapeutic agent, such as a therapeutic agent comprising any one of the antibodies of the present disclosure or a pharmaceutical composition thereof, either internally or externally, to a subject who has had, is suspected of having, or is predisposed to having one or more proliferative diseases or symptoms thereof on which the therapeutic agent is known to have a therapeutic effect. Typically, the therapeutic agent is administered in an amount effective in alleviating one or more disease symptoms in the subject or population being treated, whether by inducing regression of such symptoms or inhibiting the development of such symptoms into any clinically measurable degree. The amount of therapeutic agent effective in alleviating any specific disease symptom (also referred to as the "therapeutically effective amount") may vary depending on a variety of factors such as the disease state, age, and weight of the subject, and the capability of the drug to produce the desired therapeutic effect in the subject. Whether a disease symptom has been alleviated can be evaluated by any clinical testing methods commonly used by doctors or other health care professionals to evaluate the severity or progression of the symptom. Although embodiments of the present

disclosure (e.g., treatment methods or products) may be ineffective in alleviating a disease symptom of interest in a certain subject, they shall alleviate the disease symptom of interest in a statistically significant number of subjects as determined by any statistical test method known in the art, such as the Student's t-test, Chi-square test, U-test by Mann and Whitney, Kruskal-Wallis test (H-test), Jonckheere-Terpstra test, and Wilcoxon test. "Effective amount" comprises an amount sufficient to ameliorate or prevent a symptom or sign of a medical disorder. An effective amount also means an amount sufficient to allow or facilitate diagnosis. The effective amount for a subject may vary depending on factors such as the disorder to be treated, the general health of the subject, the method, route, and dose of administration, and the severity of side effects. An effective amount may be the maximum dose or administration regimen to avoid significant side effects or toxic effects. The subject of the present disclosure may be an animal or a human subject. The "subject" or "patient" in the present disclosure means a mammal, particularly a primate, and especially a human.

**The equipment and methods used in the detection are shown below:**

**Appearance:**

A visual method was used. The sample vial was wiped clean, and the color, clarity, and visible foreign matter of the sample were observed on a clarity tester under a white background and a black background at an illumination intensity of 1000-1500 lx. Instrument for appearance examination: Jingtuo Instrument YB-2A clarity analyzer.

**SEC molecular exclusion chromatography:**

This is an analytical method that separates a solute based on the relative relationship between the pore size of the gel pores and the coil size of the polymer sample molecule. SEC monomer content percentage = A monomer/A total $\times$ 100% (A monomer represents the peak area of the main peak monomer in the sample, and A total represents the sum of all peak areas).

Instrument for SEC determination: Agilent 1260-Bio; chromatographic column: Waters, XBrige BEH200Å SEC (300 $\times$ 7.8 mm, 3.5 $\mu$m)

**NR-CE capillary gel electrophoresis:**

This is a type of electrophoresis in which a gel is moved into a capillary as a support medium and a method that achieves separation under a certain voltage based on the molecular weight of the sample.

Non-reduced CE purity percentage = A main peak/A total $\times$ 100% (A main peak represents the peak area of the main peak in the sample, and A total represents the sum of all peak areas).

Instrument for CE determination: Sciex model PA800 plus.

**icIEF imaged capillary isoelectric focusing electrophoresis:**

This is a technique for separation according to the difference in isoelectric points (pI) of proteins.

**icIEF** main peak content percentage = main peak area/total area $\times$ 100% (total area represents the sum of areas of acidic, main, and basic peaks).

Manufacturer of instrument for **icIEF** determination: Protein Simple, model: Muarice.

**Protein concentration determination:**

Instrument for protein concentration determination: ultraviolet-visible spectrophotometer; model: Nano Drop 2000; optical path length: 1 mm.

**Exemplary antibody pharmaceutical composition (formulation) preparation process**

Step 1: A FAP/CD40-binding antibody (e.g., Ab10-A297V3-4, with heavy and light chain sequences set forth in SEQ ID NOs: 22 and 20, respectively) was mixed with the following formulation amounts of auxiliary materials to prepare a formulation stock solution containing the FAP/CD40-binding molecule. The stock solution was filtered, subjected to in-process control sampling, and tested for sterility. The stock solution was filtered through a 0.22 μm filter, and the filtrate was collected.

Step 2: The filling amount was adjusted to 1.15 mL, and the filtrate was filled into 2 mL vials. Stoppers were applied, and in-process control samplings were performed at the beginning, in the middle, and at the end of filling to detect the difference in filling amount. Step 3: The capping machine was started, aluminum caps were put on, and capping was carried out.

Step 4: Visual inspection was performed to confirm that the products have no defects, such as inaccurate filling amount. Vial labels were printed and put on the vials; carton labels were printed, cartons were folded, packing was performed, and the carton labels were put on the cartons.

## II. Examples and Test Examples

The present disclosure is further illustrated in detail by the following examples. These examples are for illustrative purposes only and are not intended to limit the scope of the present disclosure.

Experimental methods without specific conditions indicated in the examples of the present disclosure were generally conducted under conventional conditions or conditions recommended by the manufacturer of the starting materials or commercial products. Reagents without specific origins indicated were commercially available conventional reagents.

## Examples

Methods for preparing and purifying the FAP/CD40-binding molecule in the present application are described in Patent Application No. WO2023025194, which is incorporated herein by reference in its entirety.

## Example 1. Screening for and Preparation of Anti-FAP Monoclonal Antibodies

1.1. Sequences and preparation of screening and detection antigens

Human fibroblast activation protein (FAP; GeneBank accession No. AAC51668) is a serine oligopeptidase with a molecular weight of about 170 kDa and is a homodimer. The sequences, sources, and uses of the recombinant FAP proteins used in the present disclosure are shown in Table 2.

Table 2. The sources of the amino acid sequences of the recombinant proteins

| Name | Start and end of amino acid sequence | Sino Biological Cat. No. | Use |
|---|---|---|---|
| h-FAP-biotin | Leu26-Asp760 (GeneBank accession No. AAC51668) | 10464-H07H-B | Phage display liquid-phase magnetic bead screening |
| h-FAP-His | | 10464-H07H | ELISA screening for positive clones |
| h-FAP-FITC | | 10464-H07H-F | CHO cell surface antibody display screening |

1.2. Screening for h-FAP-specific binding antibody fragments from human natural Fab phage display library

With the antigen h-FAP-biotin as the target molecule, antigen-specific phages were captured using avidin-coated magnetic beads, and phage enrichment was performed using a magnetic rack. The antigen-specific phages were eluted with a glycine solution (pH 2.2).

After two rounds of screening, 284 clones were randomly picked and screened by phage ELISA for positive clones, which included the following specific steps: The growing colonies in the dish were counted (284 colonies in total). The phages were inoculated onto a 96-well plate, each well of which contained 400 μL of culture medium (2YT + Amp + 0.2% glucose). The plate was shaken at 37 °C at 250 rpm for 6 h. The plate was coated with the antigen h-FAP-His at 100 ng/100 μL/well and incubated overnight at 4 °C. The next day, after the 96-well plate was washed and blocked, 100 μL of

overnight-incubated bacterial liquid was added to each well, and the plate was incubated at 37 °C for 1 h. After washing, a secondary antibody (an HRP-labeled anti-human IgG-Fab antibody) was added, and the plate was incubated at 37 °C for 40 min. After washing, the substrate solution was added, and the plate was incubated in a dark place for 30 min. Then the OD600 was measured using a microplate reader. A total of 122 positive clones obtained from two rounds of screening were sequenced, and 74 distinctive VHs (constituting a VH-enriched library), 48 distinctive κ light chains (constituting a KLC-enriched library), and 10 distinctive λ light chains (constituting an LLC-enriched library) were obtained.

1.3. Construction of CHO cell surface full-length antibody display library

1) A full-length antibody CHO cell display gene library was constructed, which included: The VH-enriched library, the KLC-enriched library, and the LLC-enriched library were subjected to PCR amplification with three sets of primers, respectively. The three enriched libraries were digested with enzymes and then inserted into corresponding component vectors to construct corresponding component libraries. The KLC, LLC, and VH component libraries were digested with enzymes. The KLC, LLC, and VH fragment libraries after the digestion were analyzed and purified by electrophoresis. The full-length antibody cell display vector was digested with an enzyme, and the fragments of the vector were purified. The purified fragments of the vector and the KLC, LLC, and VH fragment libraries were mixed and ligated. The ligation products were purified and transferred into *Escherichia coli* cells by electroporation. The cells were plated on a dish and cultured overnight at 37 °C, and the colonies were counted. The library capacity measured $3.6 \times 10E6$, which is more than 800 times the theoretical diversity ($74 \times 58 = 4292$). All colonies were collected, and the vector DNA was extracted to obtain a full-length antibody cell display gene library.

2) An h-FAP antigen-specific full-length antibody cell display library was constructed, which included: CHO cells were transformed with the vector DNA of the full-length antibody cell display gene library to construct a full-length antibody CHO cell display library. The cell library was screened under the pressure of hygromycin. A stably transformed cell library was obtained, and the cell library was double-stained with a PE-labeled mouse anti-human κ (or λ) light-chain antibody and an FITC-labeled h-FAP antigen and sorted by FACS for PE and FITC double-positive cells. Each well contained one cell. The κ light chain library sorting used 2 96-well plates, and the λ light chain library sorting used 1 96-well plate. The cells were cultured under the pressure of hygromycin.

3) FACS analysis was performed to identify single-cell clones displaying h-FAP antigen-specific antibodies, which included: The cells were cultured under the pressure of hygromycin for 14 days, and 153 stably transformed κ chain single-cell clones and 50 λ chain single-cell clones were obtained. The cells were digested with a 0.5 mM EDTA-PBS buffer solution, and the single-cell clones were double-stained with a PE-labeled mouse anti-human κ (or λ) light-chain antibody and an FITC-labeled h-FAP antigen. FACS analysis showed that 138 PE and FITC fluorescence double-positive single-cell clones were obtained.

4) Antibody genes were cloned, which included: The affinities of the positive clones were analyzed by FACS, and 45 clones were subjected to antibody gene PCR amplification. After centrifugation, the positive clones were collected, and the supernatant was discarded. The genomic DNA was extracted from the cells, and the VH and LC were amplified by PCR. The fragments obtained from the amplification were separated by electrophoresis and sequenced, and 6 distinctive VHs and 6 distinctive κ chains were identified. Their combinations can yield 12 clones with distinctive sequences. The VH and VL sequences of 1 of the clones are shown in Table 3.

Table 3. The amino acid sequences of the VH and VL (κ of KLC) of the h-FAP antibody

| No. | | Amino acid sequence |
|---|---|---|
| Ab10 | VH | QVQLQQSGVEVKKPGASVTVSCRASGYSFA<u>DHFIH</u>WVRQAPGQGFQW MG<u>WINPNRGVTHHAQDFQG</u>RVAMTRDMSTDTVYMELTSLRSDDTAVY YCAR<u>DASLTARPYYFYGFDV</u>WGQGTLVTVSS (SEQ ID NO:1) |
| | VL | DIQMTQSPSSVSASVGDRVTITC<u>RASQGISSWLA</u>WYQQKPGKAPKLLIY<u>A ASSLQS</u>GVPSRFSGSGSGTDFTLTISSLQPEDFATYYC<u>QQANSFPPA</u>FGQG |
| | | TKVEIK (SEQ ID NO:2) |
| Note: The CDRs defined by the Kabat numbering scheme are underlined. | | |

The present disclosure also provides the sequences of the full-length heavy and light chains of Ab10.

> Ab10 full-length heavy chain

QVQLQQSGVEVKKPGASVTVSCRASGYSFADHFIHWVRQAPGQGFQWMGWIN PNRGVTHHAQDFQGRVAMTRDMSTDTVYMELTSLRSDDTAVYYCARDASLTA RPYYFYGFDVWGQGTLVTVSS<u>ASTKGPSVFPLAPSSKSTSGGTAALGCLVKDYF PEPVTVSWNSGALTSGVHTFPAVLQSSGLYSLSSVVTVPSSSLGTQTYICNVNHK PSNTKVDKKVEPKSCDKTHTCPPCPAPE**AA**GGPSVFLFPPKPKDTLMISRTPEVT CVVVDVSHEDPEVKFNWYVDGVEVHNAKTKPREEQYNSTYRVVSVLTVLHQD WLNGKEYKCKVSNKALPAPIEKTISKAKGQPREPQVYTLPPSRDELTKNQVSLT CLVKGFYPSDIAVEWESNGQPENNYKTTPPVLDSDGSFFLYSKLTVDKSRWQQG NVFSCSVMHEALHNHYTQKSLSLSPGK</u> (SEQ ID NO: 24)

> Ab10 full-length light chain

DIQMTQSPSSVSASVGDRVTITCRASQGISSWLAWYQQKPGKAPKLLIYAASSLQ SGVPSRFSGSGSGTDFTLTISSLQPEDFATYYCQQANSFPPAFGQGTKVEIK<u>RTVA APSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC</u> (SEQ ID NO: 25)

Table 4. The CDRs of the anti-FAP antibody

| No. | CDRs of VH | | CDRs of VL | |
|---|---|---|---|---|
| Ab10 | HCDR1 | DHFIH (SEQ ID NO:3) | LCDR1 | RASQGISSWLA (SEQ ID NO:6) |
| | HCDR2 | WINPNRGVTHHAQDFQG (SEQ ID NO:4) | LCDR2 | AASSLQ (SEQ ID NO:7) |
| | | | | AASSLQS (SEQ ID NO:32) |
| | HCDR3 | DASLTARPYYFYGFDV (SEQ ID NO:5) | LCDR3 | QQANSFPPA (SEQ ID NO:8) |

1.4. Construction of CHO cell surface full-length antibody display library

1) A soluble antibody expression vector was constructed, which included: Positive VH and LC fragments with distinctive sequences were digested with enzymes and purified. The VH fragments were inserted into a soluble heavy chain expression vector (IgG1), and the LC fragments were inserted into a soluble light chain expression vector. Colonies were sent for sequencing, and DNA was extracted.
2) A soluble antibody was expressed and purified, which included: Expi293 cells were expanded by suspension culture. According to the light-heavy chain pair determined above, 18 µg of light chain expression vector and 12 µg of heavy chain expression vector were mixed. The vector DNA and PEI were mixed in a ratio by weight of 1:2.5, and the mixture was used to transform Expi293 cells. On day 6, the supernatant of the culture medium was collected, and the antibody was purified by the Protein-A method. SDS-PAGE denaturing gel electrophoresis analysis showed that the purity of the antibody reached above 90%. The antibody was stored at -80 °C.

1.5. ELISA binding assay

A plate was directly coated with a His-tagged FAP recombinant protein. After the antibody was added, a secondary antibody (an HRP-conjugated anti-human IgG antibody) and the HRP substrate TMB were added to determine the binding activity of the antibody to the antigen. The assay included: A 96-well microplate was coated with a 0.5 $\mu$g/mL solution of human FAP-His protein at 100 $\mu$L/well, and the plate was incubated overnight at 4 °C.After thorough washing, a blocking solution was added at 200 $\mu$L/well, and the plate was incubated at room temperature for 2 h.After thorough washing, the test anti-FAP antibody, diluted with a diluent, was added at 100 $\mu$L/well.The plate was incubated at room temperature for 1 h. After thorough washing, an HRP-labeled goat anti-human IgG secondary antibody diluted with a diluent in a ratio of 1:20000 was added at 100 $\mu$L/well.The plate was incubated at room temperature for 1 h. After thorough washing, TMB was added at 100 $\mu$L/well, and the plate was incubated in a dark place for 15 min.0.16 M sulfuric acid was added at 50 $\mu$L/well.The OD value at 450 nm was measured on a Thermo MultiSkanFc microplate reader, and the binding $EC_{50}$ value of the anti-FAP antibody was calculated. The result was shown in Table 5.

Table 5. The $EC_{50}$ value for the binding affinity of the anti-FAP antibody for the human FAP antigen

| No. | $EC_{50}$ (mg/mL) |
| --- | --- |
| Abl0 | 0.0920 |

1.6. Surface plasmon resonance (SPR) binding assay

A CM5 sensor chip was used, and HBS-EP + buffer solution (10 mM HEPES, 150 mM NaCl, 3 mM EDTA, 0.05% surfactant P20) was used as the mobile phase. A 30 $\mu$g/mL solution of an anti-human IgG (Fc) antibody was prepared in a 10 mM sodium acetate buffer (pH 5.0), and amino coupling immobilization was performed. The test antibody was diluted in the HBS-EP+ buffer solution and captured by the anti-human IgG (Fc) antibody on chip channels. h-FAP-His was dissolved in the HBS-EP+ buffer solution as an analyte, and the resulting solution was serially diluted 2-fold. The diluted antibody was allowed to flow through the experimental and reference channels at a flow rate of 30 $\mu$L/min, with 1 min of association and 15 min of disassociation. 10 mM glycine pH 1.5 was run as a regeneration buffer solution at a flow rate of 10 $\mu$L/min for 30 s. Data were analyzed. The results in Table 6 show that the affinity of Ab10 for antigen FAP was 3-6 times higher than that of the control anti-FAP antibody 28H1 (the sequences 219 and 233 of the patent US9266938B2).

Table 6. The SPR affinity Ka, Kd, and $K_D$ values of the anti-FAP antibody

| No. | ka (1/Ms) | kd (1/s) | $K_D$ (M) | Ratio of $K_D$ of 28H1 to $K_D$ of test antibody | Ratio of $K_d$ of 28H1 to $K_d$ of test antibody |
| --- | --- | --- | --- | --- | --- |
| Ab10 | 2.97E+05 | 7.28E-05 | 2.45E-10 | 5.3 | 3.50 |

The heavy and light chain variable region sequences of the control antibody 28H1 are as follows:

> VH of 28H1

EVQLLESGGGLVQPGGSLRLSCAASGFTFSSHAMSWVRQAPGKGLEWVSAIWA SGEQYYADSVKGRFTISRDNSKNTLYLQMNSLRAEDTAVYYCAKGWLGNFDY WGQGTLVTVSS (SEQ ID NO: 9)

> VL of 28H1

EIVLTQSPGTLSLSPGERATLSCRASQSVSRSYLAWYQQKPGQAPRLLIIGASTRA TGIPDRFSGSGSGTDFTLTISRLEPEDFAVYYCQQGQVIPPTFGQGTKVEIK   (SEQ ID NO: 10)

1.7. FACS binding assay

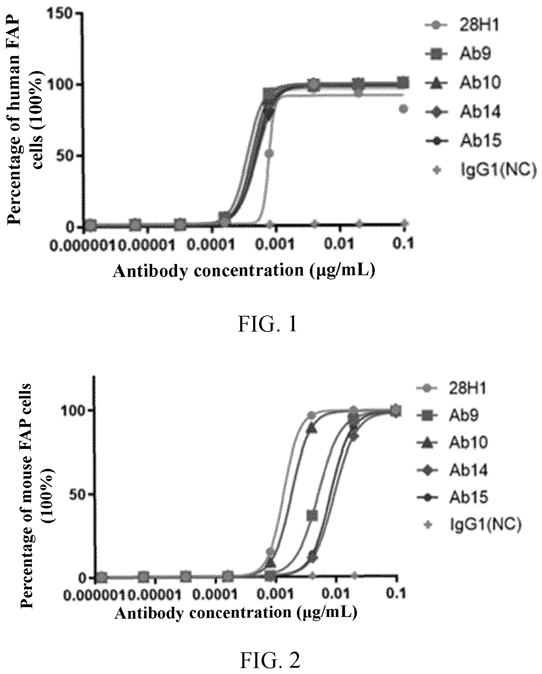

A FACS assay was used to assess the binding properties of anti-FAP antibodies on cells. The assay included: A CHO cell line overexpressing human FAP was constructed and plated (1E5/well). The test antibodies were added at 100 $\mu$L/well. The highest concentration was 100 nM and was 5-fold diluted to obtain 8 concentrations in total. The plate was incubated at 4 °C for 1 h. Anti-hIgG Alexa Fluor-647 was added as a secondary antibody in a ratio of 1:500. The plate was incubated at 4 °C for 30 min, and a FACS assay was performed. Antibodies capable of binding to FAP can mark cells. The relation between the percentage of the marked cells overexpressing human FAP among all cells and the antibody concentration is shown in FIG. 1. The $EC_{50}$ values for the binding of the antibodies to human FAP are shown in Table 7. The relation between the percentage of the marked cells overexpressing mouse FAP among all cells and the antibody concentration is shown in FIG. 2, where Ab9, Ab14, and are the other 3 anti-FAP antibodies obtained by screening in the present application.

Table 7. The $EC_{50}$ values for the binding affinities of the anti-FAP antibodies for the human FAP antigen

| Antibody No. | $EC_{50}$ (μg/mL) |
|---|---|
| 28H1 | 0.0007839 |
| Ab10 | 0.0004320 |
| Isotype (NC) | - |

**Example 2. Screening for and Preparation of Anti-CD40 Nanobodies (VHHs)**

2.1. Sequences and preparation of immunizing and screening antigens

A His-tagged human CD40 recombinant protein (h-CD40-His), a C-terminally biotinylated human CD40 recombinant protein (h-CD40-biotin), and a C-terminally His-tagged monkey CD40 recombinant protein (cyno-CD40-His) were selected. Their sequences and sources are shown in Table 8. The protein reagents can be used in the experiments of the following examples.

Table 8. The amino acid sequences and sources of the recombinant proteins

| Name | Start and end of amino acid sequence | Acrobiosystems Cat. No. |
|---|---|---|
| h-CD40-His | | CD0-H5228 |
| h-CD40-biotin | Glu21-Arg193 | CD0-H82E8 |
| cyno-CD40-His | | CD0-C52H6 |

2.2. Alpaca immunization process and titer determination

An alpaca was immunized with h-CD40-his once every two weeks, and a total of four immunizations were performed. In the first immunization, 0.5 mg of antigen and 1 mL of Freund's complete adjuvant (CFA) were well mixed and injected subcutaneously. In the following three immunizations, 0.25 mg of antigen and 1 mL of Freund's incomplete adjuvant (IFA) were well mixed and injected subcutaneously. Blank serum was collected before the immunizations. At one week after the third immunization and one week after the fourth immunization, 50 mL of peripheral blood was collected, and lymphocytes were separated. The serum titer was determined.

2.3. Titer determination and phage library construction

After the four immunizations of the alpaca, the serum titer was determined. After the titer was determined to be acceptable, PBMCs were separated, and total RNA was extracted. The purity was determined, and the RNA was reverse-transcribed into DNA. After two rounds of nested PCR, the purified vector and the VHH fragment of interest were digested with enzymes and ligated. Transfection was performed using electroporation, clones were selected, and phage libraries A and B were obtained.

2.4. Phage library affinity panning and ELISA identification

With the h-CD40 antigen as the target molecule, screening was performed by using a Gly-HCL acid elution method to elute specific phages. After two rounds of screening, 384 clones were randomly picked from the titer determination plates of the first round and the second round and screened by phage ELISA for positive clones, and the optical density at 450 nm was measured. According to the sequencing results, the sequences were subjected to sequence alignment and phylogenetic tree analysis, and 16 sequences were obtained by screening. Among them, the amino acid sequences of the better-functioning anti-CD40 antibody are shown in Table 9 and Table 10.

Table 9. The sequence of the anti-CD40 single-domain antibody

| No. | Amino acid sequence |
|---|---|
| A297 | QVQLVESGGGMVEPGGSLRLSCAASGFTFSRYGMKWVRQAPGKGPEWVSTINSHGDTTYYADSVKGRFTISRDNAQNTVYLQMNSLKPEDTALYYCLRIDYSDYSSRGQGTQVTVSS (SEQ ID NO:11) |
| Note: The CDRs defined by the Kabat numbering scheme are underlined. | |

Table 10. The CDRs of the anti-CD40 antibody

| No. | CDR sequences | |
|---|---|---|
| Ab297 | CDR1 | RYGMK (SEQ ID NO:12) |
| | CDR2 | TINSHGDTTYYADSVKG (SEQ ID NO:13) |
| | CDR3 | IDYSDYSS (SEQ ID NO:14) |

2.5. Expression and purification of anti-CD40 single-domain antibody-Fc fusion protein

The 2 VHHs above were separately ligated to human IgG1-Fc comprising the mutation N297A. The sequence of the resulting VHH-Fc fusion protein is shown in Table 11. Human IgG1-Fc is underlined, and the mutation N297A is in bold type.

9E5-SELFNS was used as a positive control CD40 agonist (see the sequences 58 and 59 of WO2020108611A1).

Table 11. The sequence of the fusion protein obtained from the anti-CD40 single-domain antibody and human IgG1-Fc

| No. | Amino acid sequence |
|---|---|
| A297-Fc | QVQLVESGGGMVEPGGSLRLSCAASGFTFSRYGMKWVRQAPGKGPEWVSTINSHGDTTYYADSVKGRFTISRDNAQNTVYLQMNSLKPEDTALYYCLRIDYSDYSSRGQGTQVTVSSEPKSADKTHTCPPCPAPELLGGPSVFLFPPKPKDTLMISRTPEVTCVVVDVSHEDPEVKFNWYVDGVEVHNAKTKPREEQY**A**STYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK(SEQ ID NO: 15) |

Plasmids were constructed and transiently transfected into cells, and antibodies were expressed and purified. Analysis showed that the antibody of interest was obtained.

2.6. FACS binding assay

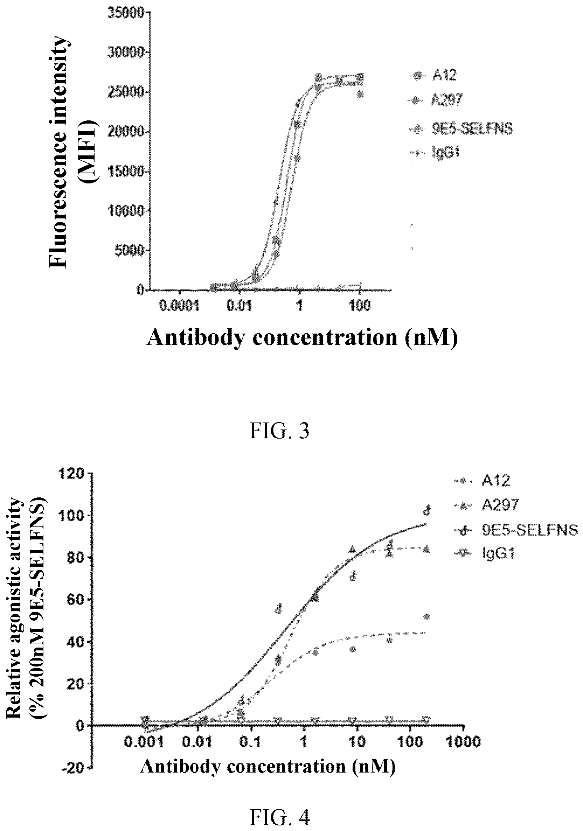

A FACS assay was used to assess the binding properties of anti-CD40 antibodies on cells. The assay included: Raji single cells were obtained and plated. The test antibodies were added at 100 μL/well. The highest concentration point was 100 nM. A 5-fold dilution was performed, yielding 8 concentration points in total. The plate was incubated at 4 °C for 1 h. Anti-hIgG Alexa Fluor-647 was added as a secondary antibody in a ratio of 1:500. The plate was incubated at 4 °C for 30 min. A FACS assay was performed. A CD40 agonist was used as a positive control. The $EC_{50}$ values of the antibodies are shown in FIG. 3 and Table 12. A12 was another anti-CD40 antibody obtained by screening in the present application.

Table 12. The $EC_{50}$ values for the binding affinities of the anti-CD40 antibodies for the human CD40 antigen

| No. | $EC_{50}$ (nM) |
|---|---|
| A297 | 0.5293 |
| 9E5-SELFNS | 0.1961 |

For a CD40 agonist antibody used in a FAP/CD40 bispecific antibody, its affinity $EC_{50}$ for CD40 should not be too high; otherwise, the bispecific antibody will bind preferentially to CD40. It is more desirable for the bispecific antibody to bind preferentially to FAP, thereby playing a role. Therefore, A297 has advantages that are more in line with the above characteristics. This is also reflected in Table 13.

2.7. Measurement of activity of anti-CD40 nanobodies

The activation of CD40 reporter gene cells by the anti-CD40 antibodies was measured, and the agonist activity of the CD40 antibodies was assessed according to $EC_{50}$. The measurement included: HEK-Blue™CD40L cells (purchased from Invivogen Cat# hkb-cd40, having been stably transfected with the human CD40 gene and the NF-$\kappa$B-mediated SEAP genome; the level of activation of the CD40 signaling pathway can be characterized by measuring the amount of SEAP secreted in the supernatant using the SEAP substrate QUANTI-Blue) were prepared. The culture medium was DMEM containing 10% FBS, 100 $\mu$g/mL Normocin, 100 $\mu$g/mL Zeocin, and 30 pg/mL Blasticidin. The cells were plated onto a 96-well plate at 5E4/well. The culture medium was DMEM containing 10% FBS and 100 $\mu$g/mL Normocin. The cells were cultured overnight. After cell adhesion, the test antibodies, serially diluted, were added at 100 $\mu$L/well, and the plate was incubated overnight at 37 °C. The cells were centrifuged. The cell supernatant was transferred to a new 96-well plate, and 180 $\mu$L of QUANTI-Blue substrate solution was added. The plate was incubated in a dark place for 15 min. The absorbance at 620 nm was measured using an Envision microplate reader, and the $EC_{50}$ values were calculated. A CD40 agonist (9E5-SELFNS) was used as a positive control. The $EC_{50}$ for the relation between the relative agonistic activity percentage (compared to 200 nM 9E5-SELFNS) and the antibody concentration is shown in FIG. 4 and Table 13. A12 was another anti-CD40 antibody obtained by screening in the present application.

Table 13. The agonistic activity $EC_{50}$ values of the anti-CD40 antibodies and their activating activity percentages (%) relative to the positive control

| No. | $EC_{50}$ (nM) | Maximum response (percentage relative to 200 nM 9E5-SELFNS) |
|---|---|---|
| A297 | 0.556 | 84.15 |
| 9E5-SELFNS | 0.466 | 102.55 |
| IgG1 | No activation | No activation |

2.8. Humanization of anti-CD40 nanobodies

According to the above results, A297 was humanized. On the basis of the typical VHH structures of the obtained nanobody A297, the VHH variable region sequences were compared with an antibody germline database to obtain highly homologous human germline templates. A preferred human germline template for the antibody A297 of the present disclosure was IGHV3-48*03. The CDRs were then grafted into human FRs, and the key amino acids that affect the structure and function of the antibody were back-mutated to restore the binding ability and activity. The humanized sequences are shown in Table 14.

Table 14. The sequences of the anti-CD40 humanized antibodies

| Name | | Amino acid sequence |
|---|---|---|
| A297 | V1 | QVQLVESGGGVVQPGGSLRLSCAASGFTFS<u>RYGMK</u>WVRQAPGKGLEWVS<u>TINSHGDTTYYADSVKG</u>RFTISRDNAKNSLYLQMNSLRAEDTALYYCAR<u>ID YSDYSS</u>RGQGTMVTVSS(SEQ ID NO: 16) |
| | V2 | QVQLVESGGGVVQPGGSLRLSCAASGFTFS<u>RYGMK</u>WVRQAPGKGLEWVS<u>TINSHGDTTYYADSVKG</u>RFTISRDNAKNSLYLQMNSLRAEDTALYYCLR<u>ID YSDYSS</u>RGQGTMVTVSS(SEQ ID NO: 17) |
| | V3 | EVQLVESGGGLVQPGGSLRLSCAASGFTFS<u>RYGMK</u>WVRQAPGKGLEWVS<u>TINSHGDTTYYADSVKG</u>RFTISRDNAKNSLYLQMNSLRAEDTAVYYCLR<u>ID YSDYSS</u>RGQGTLVTVSS(SEQ ID NO: 18) |

(continued)

| Name | | Amino acid sequence |
|---|---|---|
| | V4 | EVQLVESGGGLVQPGGSLRLSCAASGFTFSRYGMKWVRQAPGKGLEWVS TINSHGDSTYYADSVKGRFTISRDNSKNTLYLQMNSLRAEDTAVYYCLKID YSDYSSRGQGTLVTVSS(SEQ ID NO: 19) |

The above 4 VHHs were separately ligated to human IgG1-Fc comprising the mutations S267E/L328F (numbered according to EU). The sequences of the resulting VHH-Fc fusion proteins are shown in Table 15. Human IgG1-Fc is underlined, and the mutations S267E/L328F are in bold type.

Table 15. The sequences of the fusion proteins obtained from the humanized anti-CD40 single-domain antibodies and human IgG1-Fc (S267E/L328F)

| No. | Amino acid sequence |
|---|---|
| A297V1-Fc | QVQLVESGGGVVQPGGSLRLSCAASGFTFSRYGMKWVRQAPGKGLEWV STINSHGDTTYYADSVKGRFTISRDNAKNSLYLQMNSLRAEDTALYYCAR IDYSDYSSRGQGTMVTVSSEPKSADKTHTCPPCPAPELLGGPSVFLFPPKP KDTLMISRTPEVTCVVVDVEHEDPEVKFNWYVDGVEVHNAKTKPREEQ YNSTYRVVSVLTVLHQDWLNGKEYKCKVSSKAFPAPIEKTISKAKGQPRE PQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSP GK(SEQ ID NO: 26) |
| A297V2-Fc | QVQLVESGGGVVQPGGSLRLSCAASGFTFSRYGMKWVRQAPGKGLEWV STINSHGDTTYYADSVKGRFTISRDNAKNSLYLQMNSLRAEDTALYYCLRI DYSDYSSRGQGTMVTVSSEPKSADKTHTCPPCPAPELLGGPSVFLFPPKPK DTLMISRTPEVTCVVVDVEHEDPEVKFNWYVDGVEVHNAKTKPREEQY NSTYRVVSVLTVLHQDWLNGKEYKCKVSSKAFPAPIEKTISKAKGQPREP QVYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPG K(SEQ ID NO: 27) |
| A297V3-Fc | EVQLVESGGGLVQPGGSLRLSCAASGFTFSRYGMKWVRQAPGKGLEWVS TINSHGDTTYYADSVKGRFTISRDNAKNSLYLQMNSLRAEDTAVYYCLRI DYSDYSSRGQGTLVTVSSEPKSADKTHTCPPCPAPELLGGPSVFLFPPKPK DTLMISRTPEVTCVVVDVEHEDPEVKFNWYVDGVEVHNAKTKPREEQY NSTYRVVSVLTVLHQDWLNGKEYKCKVSSKAFPAPIEKTISKAKGQPREP QVYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPG K(SEQ ID NO: 28) |

(continued)

| No. | Amino acid sequence |
|---|---|
| A297V4-Fc | EVQLVESGGGLVQPGGSLRLSCAASGFTFSRYGMKWVRQAPGKGLEWVS TINSHGDSTYYADSVKGRFTISRDNSKNTLYLQMNSLRAEDTAVYYCLKI DYSDYSSRGQGTLVTVSSEPKSADKTHTCPPCPAPELLGGPSVFLFPPKPK DTLMISRTPEVTCVVVDVEHEDPEVKFNWYVDGVEVHNAKTKPREEQY NSTYRVVSVLTVLHQDWLNGKEYKCKVSSKAFPAPIEKTISKAKGQPREP QVYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPG K(SEQ ID NO: 29) |

The above 4 VHH-Fc were used to transiently transfect cells, and antibodies were expressed and purified. Analysis showed that the antibody proteins of interest were obtained.

2.9. Affinities of humanized CD40 antibodies for cell strain highly expressing CD40

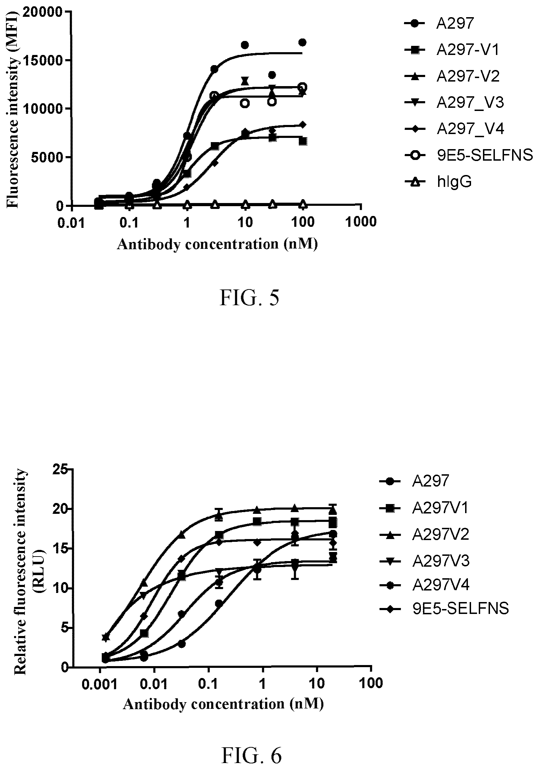

The binding abilities of the humanized antibodies to cells highly expressing the human antigen protein CD40 were determined by FACS. HEK293 cells were transiently transfected with CD40 plasmids (CD40 cDNA ORF Clone, Human, C-DYKDDDDK (Flag®) Tag; Sino Biological; HG10774-CF), yielding HEK293 cells highly expressing CD40. The resulting cells were resuspended in the flow cytometry staining buffer (PBS + 2% FBS), and the test antibodies that had been serially diluted to different concentrations were added. After 1 h of incubation on ice, the cells were washed with PBS and centrifuged at 400 g for 5 min. A goat anti-human Fc antibody labeled with the fluorescent group Alexa Fluor 647 was added as a secondary antibody. The cells were stained in an ice bath for 1 h and washed twice with PBS, and the fluorescent signals on the cell surface were then detected by FACS. The results are shown in FIG. 5, and the $EC_{50}$ values are shown in Table 16. The humanized antibodies of the present disclosure all have relatively high affinities for the cell strain highly expressing CD40.

Table 16. The $EC_{50}$ values for the affinities of the humanized anti-CD40 antibodies for the cell strain highly expressing human CD40

| Antibody | $EC_{50}$ (nM) | Emax MFI |
|---|---|---|
| A297 | 1.12 | 16728 |
| A297_V1 | 1.11 | 7300 |
| A297_V2 | 1.07 | 12764 |
| A297_V3 | 1.25 | 12827 |
| A297_V4 | 2.70 | 8255 |
| 9E5-SELFNS | 1.12 | 12120 |

2.10. Activation of HEK-Blue™ CD40L cells by humanized CD40 antibodies

The *in vitro* agonistic activity of the CD40 antibodies was assessed by assessing the activation of HEK-Blue™CD40L cells by the CD40 antibodies when crosslinked with FcγRIIb. The mutations S267E/L328F contained in the Fc of the humanized CD40 antibodies enhance the affinity of IgG1 Fc for FcγRIIb and thus enhance the crosslinking of the antibodies by FcγRIIb. The agonist activity of the CD40 antibodies when fully crosslinked was simulated by the FcγRIIb-mediated activation of HEK-Blue™ CD40L cells by the CD40 antibodies. HEK293 cells were transiently transfected with FcγRIIb plasmids (CD32B/Fcgr2b cDNA ORF Clone, Human, N-His tag; Sino Biological; HG10259-NH) to obtain HEK293 cells highly expressing FcγRIIb. HEK-Blue™CD40L cells were plated onto a 96-well cell culture plate at 5E4/well (the culture medium was DMEM, 10% FBS, 100 μg/mL Normocin), and the HEK293 cells expressing FcγRIIb were added at 5E4/well. The test antibodies, serially diluted, were added at 100 μL/well, and the plate was incubated overnight at 37 °C. The cells were centrifuged. 20 μL of the cell supernatant was transferred to a new 96-well plate, and 180 μL of QUANTI-Blue substrate solution was added. The plate was incubated in a dark place for 30 min. The absorbance at 620 nm was measured using an Envision microplate reader, and the EC$_{50}$ values and the Emax values (relative to the fluorescence intensity of the antibody-free group) were calculated. The *in vitro* cell agonist activity of the CD40 antibodies was assessed based on the EC$_{50}$ values.

The FcγRIIb-mediated activation of HEK-Blue™CD40L cells by the CD40 antibodies is shown in FIG. 6. The EC$_{50}$ values and the Emax values (relative fluorescence intensity) are shown in Table 17.

The results show that in the reporter gene cell system described above, the humanized CD40 antibodies all exhibited relatively strong agonist activity when crosslinked with FcγRIIb.

Table 17. The activation of HEK-Blue™CDA40L cells by the humanized anti-CD40 antibodies

| Antibody | FcγRIIb-mediated activation of HEK-Blue™CD40L cells | |
|---|---|---|
| | EC$_{50}$ (nM) | Emax (relative fluorescence intensity) |
| A297 | 0.243 | 17.34 |
| A297-V1 | 0.021 | 18.37 |
| A297-V2 | 0.005 | 19.99 |
| A297_V3 | 0.006 | 12.84 |
| A297_V4 | 0.038 | 13.31 |
| 9E5-SELFNS | 0.009 | 16.05 |

**Example 3. Design and Preparation of Anti-FAP/CD40 Bispecific Antibodies**

3.1. Design, expression, and purification of anti-FAP/CD40 bispecific antibodies

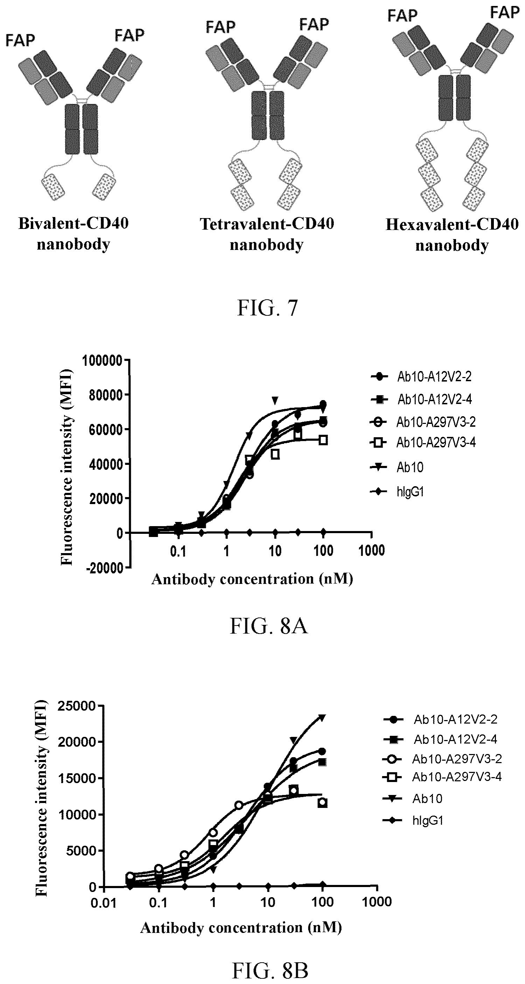

According to the humanized anti-CD40 antibody screening results, A297V3 was selected. According to the anti-FAP antibody screening results, Ab10 was selected. The selected antibodies were used to construct anti-FAP/CD40 bispecific antibodies. Ab10 was used as the IgG framework for the bispecific antibodies, and each of the C-termini of the two heavy chains of Ab10 was connected with 1 anti-CD40 nanobody, or 2 or 3 anti-CD40 nanobodies in tandem. The linkers used between the CD40 nanobodies and the linkers used for linking to the C-termini of the heavy chains of Ab10 were "GGGGSGGGGS". Each of the bispecific antibodies contains bivalent, tetravalent, and hexavalent CD40 nanobodies, as shown in FIG. 7. In the names of the antibodies, for example, Ab10-A297V3-2, Ab10 represents the anti-FAP antibody used, A297V3 represents the humanized anti-CD40 antibody used, and the "2" at the end represents that CD40 was bivalent. The other antibodies are all named in this fashion. Transient transfection and antibody expression and purification were performed using the methods in section 2.5 of Example 2. Analysis showed that the bispecific antibody molecules of interest were obtained. The amino acid sequences of the bispecific antibody molecules are as follows:

> The light chain of Ab10-A12V2-2

DIQMTQSPSSVSASVGDRVTITCRASQGISSWLAWYQQKPGKAPKLLIYAASSLQ
SGVPSRFSGSGSGTDFTLTISSLQPEDFATYYCQQANSFPPAFGQGTKVEIK<u>RTVA</u>

<u>APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC</u>    (SEQ ID NO: 20)

> The heavy chain of Ab10-A297V3-2

QVQLQQSGVEVKKPGASVTVSCRASGYSFADHFIHWVRQAPGQGFQWMGWIN PNRGVTHHAQDFQGRVAMTRDMSTDTVYMELTSLRSDDTAVYYCARDASLTA RPYYFYGFDVWGQGTLVTVSSASTKGPSVFPLAPSSKSTSGGTAALGCLVKDYF PEPVTVSWNSGALTSGVHTFPAVLQSSGLYSLSSVVTVPSSSLGTQTYICNVNHK PSNTKVDKKVEPKSCDKTHTCPPCPAPEAAGGPSVFLFPPKPKDTLMISRTPEVT CVVVDVSHEDPEVKFNWYVDGVEVHNAKTKPREEQYNSTYRVVSVLTVLHQD WLNGKEYKCKVSNKALPAPIEKTISKAKGQPREPQVYTLPPSRDELTKNQVSLT CLVKGFYPSDIAVEWESNGQPENNYKTTPPVLDSDGSFFLYSKLTVDKSRWQQG NVFSCSVMHEALHNHYTQKSLSLSPGK*GGGGSGGGGS*EVQLVESGGGLVQPGG SLRLSCAASGFTFSRYGMKWVRQAPGKGLEWVSTINSHGDTTYYADSVKGRFT ISRDNAKNSLYLQMNSLRAEDTAVYYCLRIDYSDYSSRGQGTLVTVSS (SEQ ID NO: 21)

> The heavy chain of Ab10-A297V3-4

QVQLQQSGVEVKKPGASVTVSCRASGYSFADHFIHWVRQAPGQGFQWMGWIN PNRGVTHHAQDFQGRVAMTRDMSTDTVYMELTSLRSDDTAVYYCARDASLTA RPYYFYGFDVWGQGTLVTVSSASTKGPSVFPLAPSSKSTSGGTAALGCLVKDYF PEPVTVSWNSGALTSGVHTFPAVLQSSGLYSLSSVVTVPSSSLGTQTYICNVNHK PSNTKVDKKVEPKSCDKTHTCPPCPAPEAAGGPSVFLFPPKPKDTLMISRTPEVT CVVVDVSHEDPEVKFNWYVDGVEVHNAKTKPREEQYNSTYRVVSVLTVLHQD WLNGKEYKCKVSNKALPAPIEKTISKAKGQPREPQVYTLPPSRDELTKNQVSLT CLVKGFYPSDIAVEWESNGQPENNYKTTPPVLDSDGSFFLYSKLTVDKSRWQQG NVFSCSVMHEALHNHYTQKSLSLSPGK*GGGGSGGGGS*EVQLVESGGGLVQPGG SLRLSCAASGFTFSRYGMKWVRQAPGKGLEWVSTINSHGDTTYYADSVKGRFT ISRDNAKNSLYLQMNSLRAEDTAVYYCLRIDYSDYSSRGQGTLVTVSS*GGGGSG GGGS*EVQLVESGGGLVQPGGSLRLSCAASGFTFSRYGMKWVRQAPGKGLEWV STINSHGDTTYYADSVKGRFTISRDNAKNSLYLQMNSLRAEDTAVYYCLRIDYS DYSSRGQGTLVTVSS (SEQ ID NO: 22)

> The heavy chain of Ab10-A297V3-6

QVQLQQSGVEVKKPGASVTVSCRASGYSFADHFIHWVRQAPGQGFQWMGWIN PNRGVTHHAQDFQGRVAMTRDMSTDTVYMELTSLRSDDTAVYYCARDASLTA RPYYFYGFDVWGQGTLVTVSSASTKGPSVFPLAPSSKSTSGGTAALGCLVKDYF PEPVTVSWNSGALTSGVHTFPAVLQSSGLYSLSSVVTVPSSSLGTQTYICNVNHK PSNTKVDKKVEPKSCDKTHTCPPCPAPEAAGGPSVFLFPPKPKDTLMISRTPEVT CVVVDVSHEDPEVKFNWYVDGVEVHNAKTKPREEQYNSTYRVVSVLTVLHQD WLNGKEYKCKVSNKALPAPIEKTISKAKGQPREPQVYTLPPSRDELTKNQVSLT CLVKGFYPSDIAVEWESNGQPENNYKTTPPVLDSDGSFFLYSKLTVDKSRWQQG NVFSCSVMHEALHNHYTQKSLSLSPGK*GGGGSGGGGS*EVQLVESGGGLVQPGG

SLRLSCAASGFTFSRYGMKWVRQAPGKGLEWVSTINSHGDTTYYADSVKGRFT
ISRDNAKNSLYLQMNSLRAEDTAVYYCLRIDYSDYSSRGQGTLVTVSS*GGGGSG
GGGS*EVQLVESGGGLVQPGGSLRLSCAASGFTFSRYGMKWVRQAPGKGLEWV
STINSHGDTTYYADSVKGRFTISRDNAKNSLYLQMNSLRAEDTAVYYCLRIDYS
DYSSRGQGTLVTVSS*GGGGSGGGGS*EVQLVESGGGLVQPGGSLRLSCAASGFTF
SRYGMKWVRQAPGKGLEWVSTINSHGDTTYYADSVKGRFTISRDNAKNSLYLQ
MNSLRAEDTAVYYCLRIDYSDYSSRGQGTLVTVSS (SEQ ID NO: 23)

The light chains of Ab10-A297V3-2, Ab10-A297V3-4, and Ab10-A297V3-6 are all set forth in SEQ ID NO: 20.

Note: The CH1 and Fc regions in the heavy chains are underlined; the CL regions in the light chains are underlined; the linkers are in italic type; LALA (the mutations L234A and L235A) is in bold type.

3.2. Measurement of antigen-binding affinities of anti-FAP/CD40 bispecific antibodies

The affinities of the anti-FAP/CD40 bispecific antibodies for their antigens, the human FAP protein (Acro, FAP-H5244) and the human CD40 protein (Acro, CD0-H5228), were measured by SPR (GE Healthcare; Biacore 8K). A CM5 sensor chip was used, and HBS-EP + buffer solution (10 mM HEPES, 150 mM NaCl, 3 mM EDTA, 0.05% surfactant P20) was used as the mobile phase. An anti-human IgG (Fc) antibody was immobilized by amino coupling. The test antibodies were separately diluted in HBS-EP + buffer solution as ligands and captured by the anti-human IgG (Fc) antibody on chip channels. Antigenic proteins of different species were used as analytes. The results are shown in Tables 18 and 19. The results show that the prepared bispecific antibodies can normally bind to FAP, and because their affinities for CD40 are lower, they bind preferentially to FAP, thereby playing a role.

Table 18. The affinities of the anti-FAP/CD40 bispecific antibodies for human FAP

| Antibody | Ka (1/Ms) | Kd (1/s) | KD (M) |
|---|---|---|---|
| Ab10 | 2.17E+05 | 7.39E-05 | 3.40E-10 |
| Ab10-A297V3-2 | 2.03E+05 | 8.22E-05 | 4.05E-10 |
| Ab10-A297V3-4 | 1.84E+05 | 6.96E-05 | 3.78E-10 |

Table 19. The affinities of the anti-FAP/CD40 bispecific antibodies for human CD40

| Antibody | Ka (1/Ms) | Kd (1/s) | KD (M) |
|---|---|---|---|
| 9E5-SELFNS | 6.04E+06 | 6.70E-03 | 1.11E-09 |
| Ab10-A297V3-2 | 2.09E+05 | 2.94E-02 | 1.41E-07 |
| Ab10-A297V3-4 | 5.24E+05 | 1.37E-01 | 2.62E-07 |

3.3. Measurement of affinities of anti-FAP/CD40 bispecific antibodies for cell strains highly expressing FAP

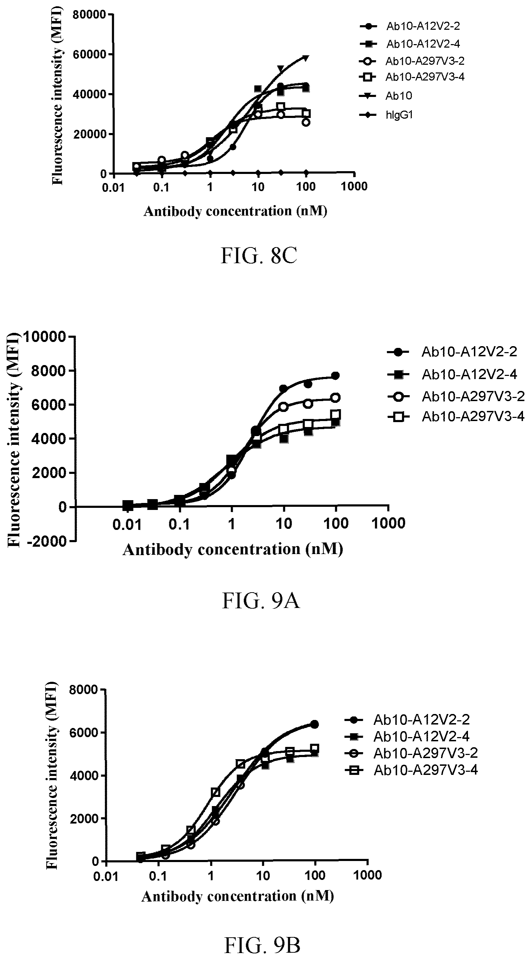

In this experiment, the binding of the anti-FAP/CD40 bispecific antibodies to stably transfected strains of CHOK1 cells highly expressing human FAP, cynomolgus monkey FAP, and mouse FAP was measured by FACS. The bispecific antibodies were shown to bind to the human, mouse, and cynomolgus monkey cell surface FAP antigens, and their binding abilities are similar to those of the mAbs. See FIGs. 8A-8C. The $EC_{50}$ values are shown in Table 20. Ab10-A12V2-2 and Ab10-A12V2-4 were another 2 anti-FAP/CD40 bispecific antibodies obtained by screening in the present application.

Table 20. The EC$_{50}$ values for the binding of the anti-FAP/CD40 bispecific antibodies to the human and mouse cell surface FAP antigens

| Antibody | Human FAP | | Mouse FAP | | Cynomolgus monkey FAP | |
|---|---|---|---|---|---|---|
| | EC$_{50}$ (nM) | Emax (MFI) | EC$_{50}$ (nM) | Emax (MFI) | EC$_{50}$ (nM) | Emax (MFI) |
| Ab10-A297V3-2 | 2.51 | 65204 | 0.79 | 12633 | 1.09 | 28178 |
| Ab10-A297V3-4 | 1.57 | 53835 | 1.65 | 12747 | 1.35 | 32480 |
| Ab10 | 1.41 | 71968 | 10.41 | 26381 | 7.34 | 64618 |

3.4. Measurement of affinities of anti-FAP/CD40 bispecific antibodies for cell strains highly expressing CD40

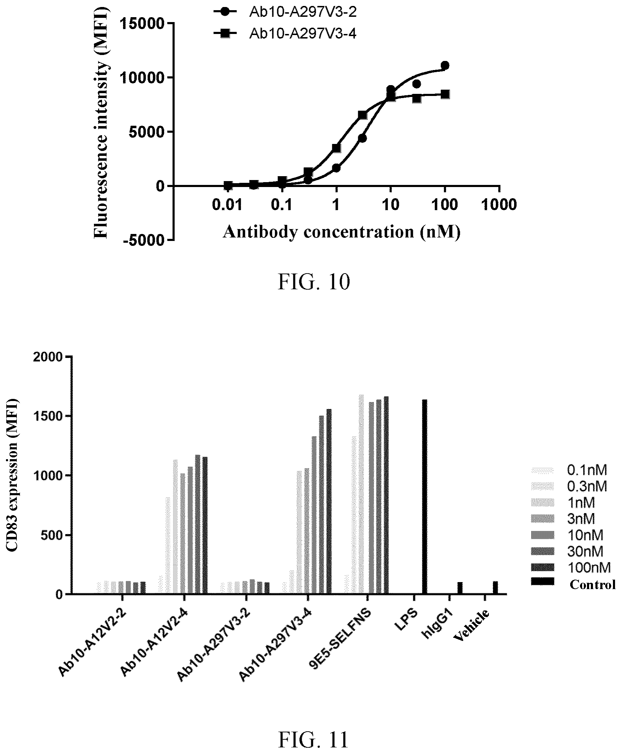

The binding of the bispecific antibodies to cell surface CD40 was measured by FACS, including HEK-BlueTMCD40L cells highly expressing human CD40 (Invivogen, Cat# hkb-cd40), and HEK293 cells that were transiently transfected with cynomolgus monkey CD40 plasmids (Sino Biological, cat# CG90970-UT) and thus made to highly express cynomolgus monkey CD40. The results show that the bispecific antibodies can bind to the CD40 on the surfaces of human and cynomolgus monkey cell membranes. See FIGs. 9A, 9B, and 11 and Table 21 for details. Ab10-A12V2-2 and Ab10-A12V2-4 were another 2 anti-FAP/CD40 bispecific antibodies obtained by screening in the present application.

Table 21. The EC$_{50}$ values for the binding of the bispecific antibodies to the human cell surface CD40 antigen

| Antibody | Human CD40 | | Cynomolgus monkey CD40 | |
|---|---|---|---|---|
| | EC$_{50}$ (nM) | Emax (MFI) | EC$_{50}$ (nM) | Emax (MFI) |
| Ab10-A297V3-2 | 1.66 | 6254 | 1.31 | 4927 |
| Ab10-A297V3-4 | 0.94 | 5085 | 0.88 | 5124 |

3.5. Measurement of FAP-dependent DC maturation-promoting activity of humanized anti-FAP/CD40 bispecific antibodies

To validate the effect of the anti-FAP/CD40 bispecific antibodies on dendritic cell maturation, monocytes were isolated from fresh PBMCs using CD14-positive magnetic beads and cultured in 1640 culture medium containing 50 ng/mL GM-CSF and 50 ng/mL hIL-4 for 5 days. A half-medium change was performed every 2-3 days. On day 5, the dendritic cells obtained by induction were added to a 96-well plate at the density of 1E5 cells/well, and CHOK1 cells in the log phase of growth and CHOK1 cells overexpressing human FAP were separately added. Meanwhile, the serially diluted test antibodies were added, with 1 $\mu$g/mL LPS as a positive control, and 100 nM hIgG1 and an antibody-free group as negative controls. After 48 h of culture, the expression of CD83 molecules on the surfaces of the dendritic cells was measured by FACS.

The results in FIG. 11 (in the absence of CHOK1/FAP) and FIG. 12 (in the presence of CHOK1/FAP) show that the bivalent molecule Ab10-A297V3-2 cannot activate DCs in the absence of CHOKI/FAP: its CD40 agonist activity is completely dependent on FAP-mediated crosslinking. However, the tetravalent molecule Ab10-A297V3-4 can induce DC cell maturation in the absence of CHOK1/FAP cells, and the activation of DC cells can be further enhanced in the presence of CHOK1/FAP cells. Thus, the FAP expressed on the surfaces of dendritic cells can provide a window of activation for the bispecific antibodies, whether bivalent or tetravalent. Ab10-A12V2-2 and Ab10-A12V2-4 were another 2 anti-FAP/CD40 bispecific antibodies obtained by screening in the present application.

## Example 4. *In Vivo* Efficacy of Anti-FAP/CD40 Bispecific Antibodies Ab10-A297V3-2/4 in Mice

B-hCD40 humanized mice were from Biocytogen Jiangsu Co., Ltd. (species: Mus musc $\mu$Lus; strain: C57BL/6; female). Mouse colorectal carcinoma MC38 cells were transfected with full-length mouse FAP plasmids to construct a stably transfected strain. Cells of the strain were subcutaneously inoculated into the right axillae of the mice at $5 \times 10^5$ cells/0.1 mL/mouse. When the mean tumor volume grew to 80-100 mm$^3$, mice with proper individual tumor volumes were selected and randomized into groups of 6. Mice were intraperitoneally injected twice a week. 48 h after the first administration, peripheral blood samples were taken and assayed for B cell counts and cell surface CD86 expression. On day 8 after the grouping, the concentrations of alanine aminotransferase (ALT) and aspartate aminotransferase (AST) were measured. The administration was stopped after 4 administrations. When there were mice with a tumor volume of more than 2000 mm$^3$ in the hIgG1 control group, the last administration was performed. 24 h after the last administration, a complete blood count was performed.

4.1. Single-dose *in vivo* efficacy and toxicity of Ab10-A297V3-2 or Ab10-A297V3-4 in mice

The anti-tumor activity of Ab10-A297V3-2 or Ab10-A297V3-4 was compared with that of the CD40 agonist mAb 9E5-mIgG1 under single-dose conditions. 9E5-mIgG1 and 9E5-SELFNS have the same variable region but use the mouse mIgG1 heavy chain constant region and the mouse kappa chain constant region as their heavy and light chain constant regions. As the activity of CD40 agonist mAbs is dependent on the FcγRIIb's crosslinking of their Fc, 9E5-mIgG1, which uses the mouse IgG1 heavy chain constant region, can better exhibit CD40 agonist activity in mice. In the experiment on *in vivo* efficacy in mice, 9E5-mIgG1 was a better control antibody than 9E5-SELFNS.
Ab10-A297V3-4 has stronger anti-tumor activity than the CD40 mAb 9E5-mIgG1 at the same molar dose in terms of whether TGI (tumor growth inhibition) or CR (complete response). The sequences of 9E5-mIgG1 are as follows:

> The heavy chain of 9E5-mIgG1

QVQLVQSGAEVKKPGASVKVSCKASGYILTTYWITWVRQAPGQGLEWMGDIH
PGSGSTKYNEKFKSRVTLTVDTSISTAYMELSRLRSEDTAVYYCARRDYWGQGT
TVTVSSAKTTPPSVYPLAPGSAAQTNSMVTLGCLVKGYFPEPVTVTWNSGSLSS
GVHTFPAVLQSDLYTLSSSVTVPSSTWPSETVTCNVAHPASSTKVDKKIVPRDCG

CKPCICTVPEVSSVFIFPPKPKDVLTITLTPKVTCVVVDISKDDPEVQFSWFVDDV
EVHTAQTQPREEQFNSTFRSVSELPIMHQDWLNGKEFKCRVNSAAFPAPIEKTIS
KTKGRPKAPQVYTIPPPKEQMAKDKVSLTCMITDFFPEDITVEWQWNGQPAEN
YKNTQPIMDTDGSYFVYSKLNVQKSNWEAGNTFTCSVLHEGLHNHHTEKSLSH
SPGK                                                      (SEQ ID NO: 30)

> The light chain of 9E5-mIgG 1

DIVMTQSPLSLPVTPGEPASISCRSSQNIVNSQGNTYLEWYLQKPGQSPQLLIYK
VTNRFSGVPDRFSGSGSGTDFTLKISRVEAEDVGVYYCFQASLVPWTFGGGTKV
EIKRADAAPTVSIFPPSSEQLTSGGASVVCFLNNFYPKDINVKWKIDGSERQNGV
LNSWTDQDSKDSTYSMSSTLTLTKDEYERHNSYTCEATHKTSTSPIVKSFNRNEC
                                                          (SEQ ID NO: 31)

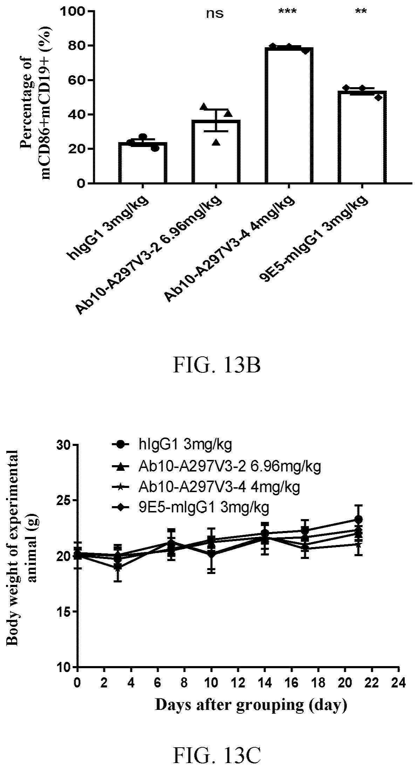

The anti-tumor activity of Ab10-A297V3-2 in a two-fold molar concentration is still weaker than that of Ab10-A297V3-4 but is similar to that of 9E5-mIgG1.
The activation of peripheral CD40 by the bispecific antibody molecules was measured by measuring the activation of mouse peripheral blood B lymphocytes. The results agree with the *in vitro* experiment. Ab10-A297V3-4 was able to partially activate peripheral B lymphocytes due to its CD40 activation activity partially independent of FAP, while Ab10-A297V3-2 did not cause detectably significant peripheral B cell activation (One-Way ANOVA) because its CD40 activation is completely dependent on FAP. Information about the tested antibodies is shown in Table 22. The *in vivo* efficacy results are shown in FIG. 13A ($P < 0.0001$ for all the antibody groups relative to the hIgG1 control group) and FIG. 13B.

Table 22. Information about the tested antibodies

| Drug | Concentration | Molecular weight (kDa) | Dose of administration |
|---|---|---|---|
| hIgG1 | 14.17 mg/mL | 150 | 3 mg/kg |
| Ab10-A297V3-2 | 2.95 mg/mL | 174 | 6.96 mg/kg |
| Ab10-A297V3-4 | 3 mg/mL | 200.03 | 4 mg/kg |

(continued)

| Drug | Concentration | Molecular weight (kDa) | Dose of administration |
|---|---|---|---|
| 9E5-mIgG1 | 4.4 mg/mL | 150 | 3 mg/kg |

In terms of mouse toxicity, neither Ab10-A297V3-2 nor Ab10-A297V3-4 caused changes in mouse body weight. See FIG. 13C.

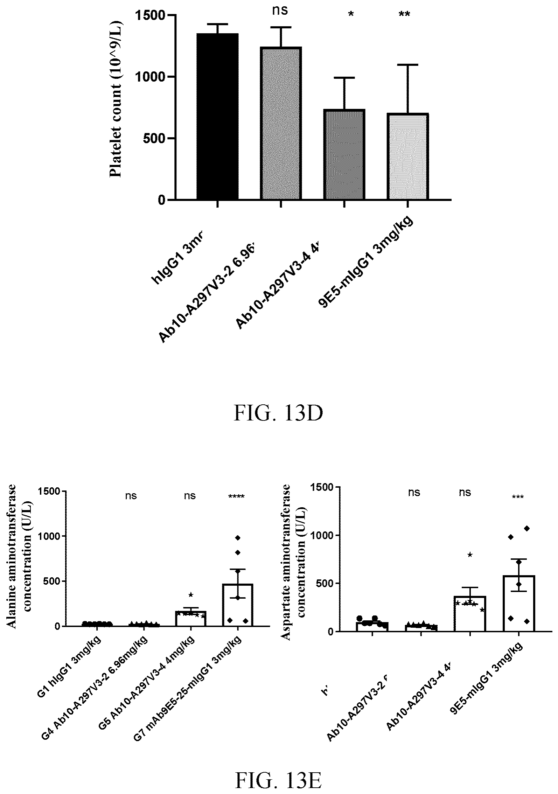

The complete blood count results show that compared to the hIgG1 control group at the same molar dose, 9E5-mIgG1 caused a certain platelet reduction, similar to hepatotoxicity; Ab10-A297V3-2 did not cause platelet reductions; Ab10-A297V3-4 caused the same platelet reduction as 9E5-mIgG1. See FIG. 13D. The dose of Ab10-A297V3-2/4 administered to mice and the statistics about tumor growth inhibition rates are detailed in Table 23.

The formula for calculating TGI is:

TGI = (day's tumor volume of blank group - day's tumor volume of treatment group) / (day's tumor volume of blank group) $\times$ 100%

Table 23. The dose of Ab10-A297V3-2 or 4 administered to mice and the tumor growth inhibition rates

| Antibody No. | Dose (mpk) | TGI |
|---|---|---|
| hIgG1 | 3 | - |
| Ab10-A297V3-2 | 6.96 (two-fold molar dose) | 54.6% |
| Ab10-A297V3-4 | 4 (equimolar dose) | 96.4% |
| 9E5-mIgG1 | 3 | 59.2% |

As can be seen from the ALL and AST results, 9E5-mIgG1 showed some hepatotoxicity on day 8 post-dose through CD32B (i.e., FcγRIIB)-mediated crosslinking. Ab10-A297V3-4 has some hepatotoxicity due to a certain level of background activation, but because its activation is independent of CD32B, the hepatotoxicity is lower than that of 9E5-mIgG1. Ab10-A297V3-2 does not have hepatotoxicity at all because its CD40 activation is dependent on FAP. See FIG. 13E.

4.2. Multiple-dose *in vivo* efficacy and toxicity of Ab10-A297V3-2 or Ab10-A297V3-4 in mice

To explore the therapeutic window of Ab10-A297V3-2 or 4, the dose of Ab10-A297V3-2 was further increased, and the dose of the molecule Ab10-A297V3-4 was decreased. See Table 24 for details. When the dose of the bivalent molecule Ab10-A297V3-2 was further increased, its anti-tumor activity was not further improved, and peripheral B lymphocyte activation was not observed either. When the dose of Ab10-A297V3-4 was reduced to 1.3 mg/kg (i.e., the 1/3 molar dose of 9E5-mIgG1), it still had a TGI of 92.6%, which was far better than that of 9E5-mIgG1.

To assess the role of the FAP antibody in the tetravalent CD40 bispecific antibody in *in vivo* experiments, the anti-tumor activity of Ab10-A297V3-4 was compared with that of another tetravalent CD40 bispecific antibody without FAP binding function (isotype-A297V3-4). Isotype-A297V3-4 is identical to Ab10-A297V3-4 in structural terms, except that it contains another isotype antibody that did not bind to any murine proteins instead of the anti-FAP moiety Ab10.

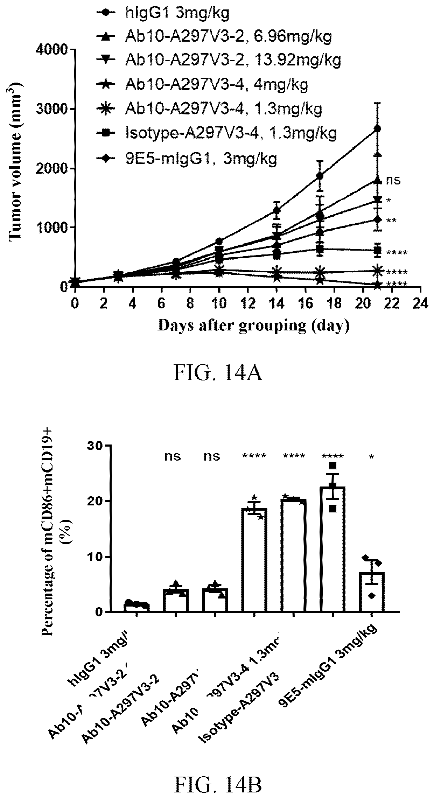

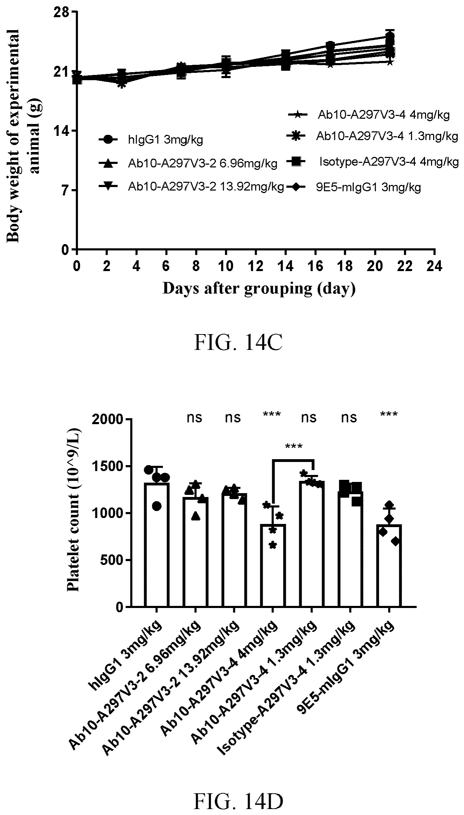

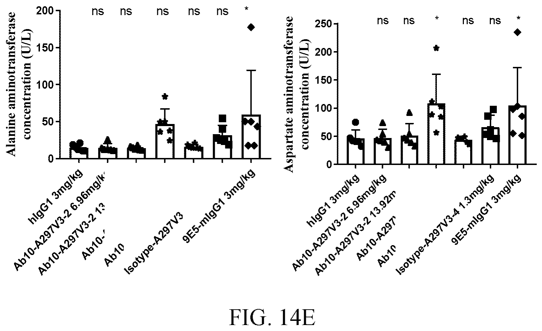

The results in FIGs. 14A-14E show that the tetravalent CD40 bispecific antibody can exhibit relatively good anti-tumor activity even without binding to FAP; furthermore, its anti-tumor activity can be stronger if it binds to FAP. As for toxicity, the increased dose of Ab10-A297V3-2 still did not cause abnormal body weight, ALT/AST levels, or platelet counts, and the reduced dose of Ab10-A297V3-4 did not cause the abnormal ALT/AST levels or platelet counts which were observed with a higher dose previously, while still maintaining a very strong anti-tumor effect (P < 0.0001). This indicates that both Ab10-A297V3-2 and Ab10-A297V3-4 have wider therapeutic windows than the CD40 mAb 9E5-mIgG1. Information about the antibodies is shown in Table 24. The statistics about the multiple-dose *in vivo* efficacy of Ab10-A297V3-2 or Ab10-A297V3-4 are shown in Table 25.

Table 24. Information about the tested antibodies

| Drug | Concentration | Molecular weight (kDa) | Dose |
|---|---|---|---|
| hIgG1 | 14.17 mg/mL | 150 | 3 mg/kg |

(continued)

| Drug | Concentration | Molecular weight (kDa) | Dose |
|---|---|---|---|
| Ab10-A297V3-2 | 2 mg/mL | 174 | 6.96 mg/kg |
| Ab10-A297V3-2 | | | 13.92 mg/kg |
| Ab10-A297V3-4 | 2 mg/mL | 200.03 | 4 mg/kg |
| Ab10-A297V3-4 | | | 1.3 mg/kg |
| Isotype-A297V3-4 | 3.44 mg/mL | 200.03 | 1.3 mg/kg |
| 9E5-mIgG1 | 4.79 mg/mL | 150 | 3 mg/kg |

Table 25. The statistics about the multiple-dose *in vivo* efficacy of Ab10-A297V3-2/4

| Drug | Dose (mg/kg) | TGI | CR ratio (<100 mm$^3$) |
|---|---|---|---|
| hIgG1 | 3 | - | 0% |
| Ab10-A297V3-2 | 6.96 (two-fold molar dose) | 32.8% | 0% |
| Ab10-A297V3-2 | 13.92 (four-fold molar dose) | 46.9% | 0% |
| Ab10-A297V3-4 | 4 (equimolar dose) | 101.6% | 100% |
| Ab10-A297V3-4 | 1.3 (1/3 molar dose) | 92.6% | 33% |
| Isotype-A297V3-4 | 1.3 (1/3 molar dose) | 79.3% | 0% |
| 9E5-mIgG1 | 3 | 59.2% | 0% |

The anti-FAP/CD40 bispecific antibody used in the following examples was the aforementioned Ab10-A297V3-4, the heavy chain and light chain amino acid sequences of which are SEQ ID NOs: 22 and 20, respectively.

**Example 5. Screening for Buffer Systems and pH Values for Anti-FAP/CD40 Bispecific Antibody Formulations**

The following buffers were prepared, and 20 mg/mL antibody formulations of the anti-FAP/CD40 bispecific antibody were prepared. A high-temperature (40 °C) stability study was performed.

1) 10 mM acetic acid-sodium acetate, pH 5.5
2) 10 mM histidine-histidine hydrochloride, pH 5.5
3) 10 mM histidine-histidine hydrochloride, pH 6.0
4) 10 mM histidine-histidine hydrochloride, pH 6.5
5) 10 mM succinic acid-sodium succinate, pH 5.0
6) 10 mM succinic acid-sodium succinate, pH 5.5
7) 10 mM succinic acid-sodium succinate, pH 6.0
8) 10 mM phosphate buffer, pH 6.5
9) 10 mM phosphate buffer, pH 7.0
10) 10 mM phosphate buffer, pH 7.5

Table 26. The results for buffer system and pH value screening-1

| No. | Appearance | | |
|---|---|---|---|
| | T0 | 40 °C-2 W | 40 °C-4 W |
| F1 | Light yellow, slightly opalescent, free of visible particles | Light yellow, slightly opalescent, a few particles | Light yellow, slightly opalescent, a large number of particles |
| F2 | Light yellow, slightly opalescent, free of visible particles | Light yellow, slightly opalescent, free of visible particles | Light yellow, slightly opalescent, free of visible particles |
| F3 | Light yellow, slightly opalescent, free of visible particles | Light yellow, slightly opalescent, free of visible particles | Light yellow, slightly opalescent, free of visible particles |

(continued)

| No. | Appearance | | |
|---|---|---|---|
| | T0 | 40 °C-2 W | 40 °C-4 W |
| F4 | Light yellow, slightly opalescent, free of visible particles | Light yellow, slightly opalescent, a large number of particles | Light yellow, slightly opalescent, a large number of particles |
| F5 | Light yellow, slightly opalescent, free of visible particles | Light yellow, slightly opalescent, free of visible particles | Light yellow, slightly opalescent, free of visible particles |
| F6, | Light yellow, slightly opalescent, free of visible particles | Light yellow, slightly opalescent, free of visible particles | Light yellow, slightly opalescent, a few particles |
| F7 | Light yellow, slightly opalescent, free of visible particles | Light yellow, slightly opalescent, free of visible particles | Light yellow, slightly opalescent, a few particles |
| F8 | Light yellow, slightly opalescent, free of visible particles | Light yellow, slightly opalescent, a few particles | Light yellow, slightly opalescent, a large number of particles |
| F9 | Light yellow, slightly opalescent, free of visible particles | Light yellow, opalescent, a large number of particles | Light yellow, opalescent, a large number of particles |
| F10 | Light yellow, slightly opalescent, free of visible particles | Light yellow, opalescent, a large number of particles | Light yellow, opalescent, a large number of particles |

Table 27. The results for buffer system and pH value screening-2

| No. | SEC-HPLC | | | CEX | | | | | | | | | NR-CE % | | |
| | T0 | 40 °C 2 W | 40 °C 4 W | T0 | | | 40 °C-2 W | | | 40 °C-4 W | | | T0 | 40 °C 2 W | 40 °C 4 W |
| | Monomer% | Monomer% | Monomer% | Main peak% | Acidic peak% | Basic peak% | Main peak% | Acidic peak% | Basic peak% | Main peak% | Acidic peak% | Basic peak% | Main peak% | Main peak% | Main peak% |
|---|---|---|---|---|---|---|---|---|---|---|---|---|---|---|---|
| F1 | 96.1 | 95.3 | 94.6 | 53.5 | 43.6 | 3.0 | 40.8 | 55.0 | 4.3 | 33.8 | 63.6 | 2.6 | 95.7 | 94.4 | 91.0 |
| F2 | 96.1 | 96.7 | 95.3 | 53.5 | 43.6 | 2.9 | 38.6 | 55.4 | 6.1 | 29.4 | 67.1 | 3.5 | 96.0 | 94.3 | 90.2 |
| F3 | 96.1 | 96.7 | 95.3 | 53.4 | 43.6 | 3.0 | 37.7 | 56.0 | 6.3 | 32.5 | 67.5 | 0.0 | 95.9 | 95.5 | 88.3 |
| F4 | 95.9 | 95.9 | 94.2 | 53.2 | 43.9 | 2.9 | 31.9 | 63.6 | 4.5 | 25.8 | 74.2 | 0.0 | 96.6 | 94.0 | 83.7 |
| F5 | 96.0 | 89.3 | 87.0 | 53.7 | 43.3 | 3.0 | 28.7 | 66.9 | 4.4 | 14.6 | 82.0 | 3.4 | 96.5 | 93.1 | 90.5 |
| F6 | 95.9 | 85.6 | 81.1 | 53.6 | 43.4 | 3.0 | 32.3 | 59.2 | 8.5 | 22.2 | 73.8 | 4.0 | 96.5 | 94.2 | 90.9 |
| F7 | 95.5 | 85.3 | 80.6 | 53.4 | 43.4 | 3.2 | 36.0 | 57.1 | 7.0 | 27.5 | 69.1 | 3.4 | 96.4 | 94.9 | 87.5 |
| F8 | 95.6 | 88.8 | 84.3 | 53.5 | 43.4 | 3.1 | 33.6 | 61.5 | 4.9 | 20.4 | 74.6 | 5.0 | 96.5 | 94.7 | 83.0 |
| F9 | 95.3 | 82.5 | 75.8 | 53.0 | 44.0 | 3.0 | 26.2 | 69.2 | 4.6 | 18.3 | 81.7 | 0.0 | 96.4 | 94.1 | 75.6 |
| F10 | 94.6 | 78.6 | 75.4 | 52.8 | 44.1 | 3.1 | 17.3 | 79.0 | 3.7 | 8.9 | 91.1 | 0.0 | 96.2 | 91.4 | 64.1 |

Experimental results:

The appearance results show that: after formulations F1-F10 were stored at 40 °C for 4 weeks, no significant particles were observed in formulations F2 (histidine salt buffer, pH 5.5), F3 (histidine salt buffer, pH 6.0), and F5 (succinate buffer, pH 5.0); visible particles were observed in all the other formulations. The results indicate that the histidine salt buffers (pH 5.5, pH 6.0) and the succinate buffer (pH 5.0) were superior to other buffer systems.

The SEC results show that: after storage at a high temperature of 40 °C for 4 weeks, all the formulations showed decreases in SEC purity, the decreases in the histidine salt systems (F2-F4) and the acetate system (F1) were less than those in the succinate systems (F5-F7) and the phosphate buffer systems (F8-F10), and formulations F2 and F3 showed no significant change in monomer purity.

The CEX results show that: after storage at 40 °C for 4 weeks, the main peaks of formulations F1 and F3 decreased by about 20%, the main peak of formulation F2 decreased by about 24%, which was second to those of formulations F1 and F3, and the main peaks of other formulations decreased more significantly.

The NR-CE results show that: after storage at 40 °C for 4 weeks, formulations F1, F2, F5, and F6 showed the least purity decrease of about 6% compared with T0, formulation F3 showed the second smallest purity decrease, and the other formulations showed more significant purity decreases.

In summary, in terms of the appearance, SEC, CEX, and NR-CE detection results of the formulations, formulations F2 and F3 are better than the other formulations and exhibit better stability. Therefore, the histidine salt buffer system was selected as the final buffer system. F2 (10 mM histidine salt buffer, pH 5.5) and F3 (10 mM histidine salt buffer, pH 6.0) were selected as the buffer systems used for the next round of further pH screening.

**Example 6. Screening for Surfactant in Anti-FAP/CD40 Bispecific Antibody Formulations**

The 10 mM histidine-histidine hydrochloride pH 5.0 and pH 5.5 buffer systems were selected, and formulations containing 25 mg/mL anti-FAP/CD40 bispecific antibody, 80 mg/mL sucrose, and surfactants of different types and having different concentrations were prepared to investigate the stability after repeated freeze-thaw and shaking:

15) 10 mM histidine-histidine hydrochloride pH 5.0, 0.2 mg/mL polysorbate 80;
16) 10 mM histidine-histidine hydrochloride pH 5.5, 0.4 mg/mL polysorbate 80;
17) 10 mM histidine-histidine hydrochloride pH 5.5, 0.8 mg/mL polysorbate 80;
18) 10 mM histidine-histidine hydrochloride pH 5.5, 2 mg/mL poloxamer 188.

Table 28. The results for surfactant type and concentration screening

| Test item | Condition | | F15 | F16 | F17 | F18 |
|---|---|---|---|---|---|---|
| Appearance | T0 | | LY,SO,PF | LY,SO,PF | LY,SO,PF | LY,SO,PF |
| | Freeze-thaw-3 rounds | | LY,SO,1* | LY,SO,1* | LY,SO,PF | LY,SO,AFP |
| Test item | Condition | | F15 | F16 | F17 | F18 |
| | Freeze-thaw-5 rounds | | LY,SO,AFP | LY,SO,1* | LY,SO,AFP | LY,SO,AFP |
| | Shaking-1 day | | LY,SO,PF | LY,SO,PF | LY,SO,PF | LY,SO,PF |
| | Shaking-3 days | | LY,SO,PF | LY,SO,PF | LY,SO,PF | LY,SO,AFP |
| Insoluble microparticle (MFI) | T0 | ≥2 μm | 305 | 305 | 189 | 359 |
| | | ≥10 μm | 19 | 19 | 19 | 27 |
| | | ≥25 μm | 7 | 7 | 5 | 4 |
| | Freeze-thaw-5 rounds | ≥2 μm | 596 | 610 | 510 | 763 |
| | | ≥10 μm | 23 | 12 | 71 | 61 |
| | | ≥25 μm | 4 | 4 | 17 | 9 |
| | Shaking-3 days | ≥2 μm | 303 | 724 | 700 | 740 |
| | | ≥10 μm | 17 | 50 | 32 | 41 |
| | | ≥25 μm | 2 | 5 | 0 | 4 |

(continued)

| Test item | Condition | | F15 | F16 | F17 | F18 |
|---|---|---|---|---|---|---|
| SEC | T0 | Aggregate% | 2.2 | 2.1 | 2.1 | 2.1 |
| | | Monomer% | 97.8 | 97.9 | 97.9 | 97.9 |
| | | Fragment% | 0.0 | 0.0 | 0.0 | 0.0 |
| | Freeze-thaw-5 rounds | Aggregate% | 2.2 | 2.1 | 2.1 | 2.1 |
| | | Monomer% | 97.8 | 97.9 | 97.9 | 97.9 |
| | | Fragment% | 0.0 | 0.0 | 0.0 | 0.0 |
| | Shaking-3 days | Aggregate% | 2.3 | 2.1 | 2.1 | 2.2 |
| | | Monomer% | 97.7 | 97.9 | 97.9 | 97.9 |
| | | Fragment% | 0.0 | 0.0 | 0.0 | 0.0 |
| CEX | T0 | Acidic peak% | 37.4 | 37.4 | 37.3 | 37.4 |
| | | Main peak% | 58.2 | 56.9 | 58.4 | 58.4 |
| | | Basic peak% | 4.4 | 5.7 | 4.3 | 4.2 |
| | Freeze-thaw-5 rounds | Acidic peak% | 37.0 | 37.2 | 36.5 | 37.3 |
| | | Main peak% | 58.8 | 58.8 | 59.7 | 58.7 |
| | | Basic peak% | 4.2 | 4.0 | 3.8 | 4.0 |
| | Shaking-3 days | Acidic peak% | 37.2 | 36.6 | 37.3 | 37.5 |
| | | Main peak% | 58.6 | 59.7 | 58.6 | 58.3 |
| | | Basic peak% | 4.2 | 3.7 | 4.0 | 4.1 |
| NR-CE % | T0 | | 95.6 | 95.6 | 95.4 | 95.5 |
| | Freeze-thaw-5 rounds | | 95.6 | 95.2 | 95.3 | 95.6 |
| | Shaking-3 days | | 95.4 | 95.3 | 95.3 | 95.5 |
| NOTE: LY = LIGHT YELLOW; SO = SLIGHTLY OPALESCENT; PF = FREE OF VISIBLE PARTICLES; AFP = A FEW PARTICLES. * : 1 DENOTES 1 PARTICLE. | | | | | | |

Experimental results

The appearance results show that: after 5 rounds of freeze-thaw, 1 particle was observed in formulation F16, and a few visible particles were observed in formulations F15, F17, and F18. After 3 days of shaking, no visible particles were observed in formulations F15-F17, and a few visible particles were observed in formulation F18.

The insoluble microparticle results show that: after 3 days of shaking and 5 rounds of freeze-thaw, the particle counts of formulations F15-F18 did not change significantly, and the difference among the formulations was not large.

The SEC results show that: after 5 rounds of freeze-thaw and 3 days of shaking, the SEC monomer contents of formulations F15-F18 did not change significantly, and the difference among the formulations was not large.

The CEX results show that: after 3 days of shaking and 5 rounds of freeze-thaw, the main peaks, acidic peaks, and basic peaks of formulations F15-F18 did not change significantly, and the difference among the formulations was not large.

The NR-CE results show that: after 5 rounds of freeze-thaw and 3 days of shaking, formulations F15-F18 showed no significant change in purity, and the difference among the formulations was not large.

In summary, in the histidine salt buffer systems at pH 5.0-5.5, as can be seen from the appearance results, after 5 rounds of freeze-thaw, visible particles were observed in formulations F15-F18, and only 1 particle was observed in formulation F16, which is better than the other formulations; after 3 days of shaking, a few particles were observed in formulation F18, and no visible particles were observed in the other formulations. There was no significant difference in insoluble microparticle and purity results of different formulations. Therefore, polysorbate 80 was selected as the surfactant with a content of 0.04% (w/v).

**Example 7. Screening for Auxiliary Materials in Anti-FAP/CD40 Bispecific Antibody Formulations**

The 10 mM histidine-histidine hydrochloride buffer systems were selected, and anti-FAP/CD40 bispecific antibody formulations containing 0.4 mg/mL polysorbate 80 and different auxiliary materials and having different antibody concentrations were prepared: 19) 10 mM histidine-histidine hydrochloride pH 5.0, 8% sucrose, 25 mg/mL anti-FAP/CD40 bispecific antibody;

20) 10 mM histidine-histidine hydrochloride pH 5.5, 8% sucrose, 25 mg/mL anti-FAP/CD40 bispecific antibody;
21) 10 mM histidine-histidine hydrochloride pH 5.5, 1% sucrose + 4% mannitol, 25 mg/mL anti-FAP/CD40 bispecific antibody;
22) 10 mM arginine-glutamic acid pH 5.0, 8% sucrose, 25 mg/mL anti-FAP/CD40 bispecific antibody;
23) 10 mM histidine-histidine hydrochloride pH 5.5, 8% sucrose + 0.01% EDTA, 25 mg/mL anti-FAP/CD40 bispecific antibody;
24) 10 mM histidine-histidine hydrochloride pH 5.5, 8% sucrose + 0.01% EDTA, 5 mg/mL anti-FAP/CD40 bispecific antibody;
25) 10 mM histidine-histidine hydrochloride pH 5.5, 8% sucrose, 5 mg/mL anti-FAP/CD40 bispecific antibody.

The formulations described above were left to stand at 2-8 °C, 25 °C, and 40 °C and subjected to stability tests and repeated freeze-thaw stability tests.

Table 29. The results for anti-FAP/CD40 bispecific antibody auxiliary material screening

| Test item | Condition | | F19 | F20 | F21 | F22 | F23 | F24 | F25 |
|---|---|---|---|---|---|---|---|---|---|
| Appearance | T0 | | LY,SO,PF | LY,SO,PF | LY,SO,PF | LY,SO,PF | LY,SO,PF | LY,SO,PF | LY,SO,PF |
| | 40 °C-2 W | | LY,SO,PF | LY,SO,PF | LY,SO,PF | LY,SO,PF | LY,SO,PF | LY,SO,PF | LY,SO,PF |
| | 40 °C-4 W | | LY,SO,AFP | LY,SO,PF | LY,SO,AFP | LY,SO,PF | LY,SO,PF | LY,SO,PF | LY,SO,PF |
| | 25 °C-4 W | | N/A | LY,SO,PF | N/A | LY,SO,PF | N/A | N/A | LY,SO,PF |
| | 2~8 °C-4 W | | N/A | LY,SO,PF | N/A | LY,SO,PF | N/A | N/A | LY,SO,PF |
| | Freeze-thaw-3 rounds | | LY,SO,PF | LY,SO,PF | LY,SO,AFP | LY,SO,PF | LY,SO,PF | LY,SO,PF | LY,SO,PF |
| | Freeze-thaw-5 rounds | | LY,SO,PF | LY,SO,PF | LY,O, AFP | LY,SO,PF | LY,SO,PF | LY,SO,PF | LY,SO,PF |
| SEC | T0 | Aggregate% | 2.23 | 2.12 | 2.10 | 2.23 | 2.14 | 2.07 | 2.22 |
| | | Monomer% | 97.77 | 97.88 | 97.90 | 97.77 | 97.86 | 97.93 | 97.78 |
| | | Fragment% | 0.00 | 0.00 | 0.00 | 0.00 | 0.00 | 0.00 | 0.00 |
| | 40 °C-2 W | Aggregate% | 3.71 | 3.56 | 3.62 | 2.26 | 3.66 | 2.25 | 2.02 |
| | | Monomer% | 96.29 | 96.44 | 96.38 | 97.74 | 96.34 | 97.75 | 97.98 |
| | | Fragment% | 0.00 | 0.00 | 0.00 | 0.00 | 0.00 | 0.00 | 0.00 |
| | 40 °C-4 W | Aggregate% | 2.98 | 3.15 | 2.93 | 1.58 | 3.06 | 1.32 | 1.25 |
| | | Monomer% | 96.98 | 96.85 | 97.04 | 98.30 | 96.91 | 98.68 | 98.75 |
| | | Fragment% | 0.04 | 0.00 | 0.03 | 0.12 | 0.03 | 0.00 | 0.00 |
| | 25 °C-4 W | Aggregate% | N/A | 1.53 | N/A | 1.54 | N/A | N/A | 1.71 |
| | | Monomer% | N/A | 98.47 | N/A | 98.46 | N/A | N/A | 98.29 |
| | | Fragment% | N/A | 0.00 | N/A | 0.00 | N/A | N/A | 0.00 |
| | 2~8 °C -4 W | Aggregate% | N/A | 1.49 | N/A | 1.54 | N/A | N/A | 1.40 |
| | | Monomer% | N/A | 98.51 | N/A | 98.46 | N/A | N/A | 98.60 |
| | | Fragment% | N/A | 0.00 | N/A | 0.00 | N/A | N/A | 0.00 |
| | 5 rounds of freeze-thaw | Aggregate% | 2.18 | 2.09 | 3.72 | 2.20 | N/A | N/A | 2.18 |
| | | Monomer% | 97.82 | 97.91 | 96.28 | 97.80 | N/A | N/A | 97.82 |
| | | Fragment% | 0.00 | 0.00 | 0.00 | 0.00 | N/A | N/A | 0.00 |

(continued)

| Test item | Condition | | F19 | F20 | F21 | F22 | F23 | F24 | F25 |
|---|---|---|---|---|---|---|---|---|---|
| CEX | T0 | Acidic peak% | 35.92 | 36.37 | 36.44 | 35.36 | 36.62 | 35.77 | 36.56 |
| | | Main peak% | 61.04 | 59.09 | 59.40 | 61.67 | 59.21 | 61.29 | 59.15 |
| | | Basic peak% | 3.04 | 4.54 | 4.16 | 2.97 | 4.17 | 2.94 | 4.29 |
| | 40 °C-2 W | Acidic peak% | 50.89 | 48.24 | 49.00 | 46.16 | 47.48 | 47.70 | 47.04 |
| | | Main peak% | 47.15 | 50.00 | 48.99 | 52.31 | 50.40 | 50.45 | 51.14 |
| | | Basic peak% | 1.96 | 1.75 | 2.01 | 1.53 | 2.13 | 1.84 | 1.82 |
| | 40 °C-4 W | Acidic peak% | 63.82 | 60.35 | 60.25 | 57.22 | 59.14 | 57.71 | 61.73 |
| | | Main peak% | 33.83 | 37.62 | 37.61 | 41.33 | 38.80 | 40.77 | 35.90 |
| | | Basic peak% | 2.36 | 2.03 | 2.14 | 1.45 | 2.06 | 1.52 | 2.36 |
| | 25 °C-4 W | Acidic peak% | N/A | 40.16 | N/A | 40.05 | N/A | N/A | 38.01 |
| | | Main peak% | N/A | 56.82 | N/A | 57.26 | N/A | N/A | 59.64 |
| | | Basic peak% | N/A | 3.01 | N/A | 2.69 | N/A | N/A | 2.35 |
| | 2~8 °C -4 W | Acidic peak% | N/A | 37.50 | N/A | 36.56 | N/A | N/A | 36.43 |
| | | Main peak% | N/A | 59.84 | N/A | 61.18 | N/A | N/A | 61.19 |
| | | Basic peak% | N/A | 2.65 | N/A | 2.26 | N/A | N/A | 2.38 |
| | 5 rounds of freeze-thaw | Acidic peak% | 38.58 | 38.59 | 38.18 | 38.61 | N/A | N/A | 38.63 |
| | | Main peak% | 59.12 | 59.19 | 58.89 | 59.11 | N/A | N/A | 59.11 |
| | | Basic peak% | 2.30 | 2.22 | 2.92 | 2.28 | N/A | N/A | 2.25 |
| NR-CE % | T0 | | 95.19 | 95.32 | 95.14 | 94.96 | 95.56 | 94.67 | 95.51 |
| | 40 °C-2 W | | 94.69 | 94.79 | 93.72 | 94.37 | 95.00 | 94.89 | 94.66 |
| | 40 °C-4 W | | 93.04 | 93.85 | 93.95 | 94.15 | 94.16 | 93.93 | 93.58 |
| | 25 °C-4 W | | N/A | 95.57 | N/A | 95.45 | N/A | N/A | 95.52 |
| | 2~8 °C-4 W | | N/A | 95.68 | N/A | 95.67 | N/A | N/A | 95.67 |
| | Freeze-thaw-5 rounds | | 95.39 | 95.18 | 95.36 | 95.35 | N/A | N/A | 95.24 |

Note: LY = light yellow; SO = slightly opalescent; O = opalescent; PF = free of visible particles; AFP = a few particles. N/A: no detection.

Experimental results

The appearance results show that: after freeze-thaw, visible particles were observed in formulation F21, the degree of opalescence gradually increased with the increase in the number of freeze-thaw rounds, and no visible particles were observed in the other formulations. After storage at 40 °C for 4 weeks, a few visible particles were observed in formulations F19 and F21, and no visible particles were observed in the other formulations. After storage at 2-8 °C and 25 °C for 4 weeks, all the formulations showed no visible particles.

The SEC results show that: after storage at 40 °C for 4 weeks, all the formulations showed no significant change in monomer contents. After 5 rounds of freeze-thaw, the SEC monomer content showed no significant change.

The CEX results show that: after storage at 40 °C for 4 weeks, the main peaks of formulations F22-F24 decreased slightly, and the main peaks of the other formulations decreased more significantly. After 5 rounds of freeze-thaw, the CEX main peak, acidic peak, and basic peak contents did not change significantly.

The NR-CE results show that: after storage at 40 °C for 4 weeks, formulation F19 showed the greatest decrease in purity, and the other formulations showed slight decreases in purity, but the decreases were comparable. After 5 rounds of freeze-thaw, all the formulations showed no significant change in purity, and the difference among the formulations was not large.

In summary, as can be seen from the appearance results, after 5 rounds of freeze-thaw, a few particles were observed and the degree of opalescence was enhanced in formulation F21 as compared to the other formulations; after incubation at 40 °C for 4 weeks, a few particles were observed in formulations F19 and F21, and no significant change was observed in the other formulations. The SEC monomer purity of each formulation showed no significant change. As can be seen from the CEX results, after the samples were incubated at 40 °C for 4 weeks, the main peak contents of formulations F20-F24 decreased less than those of the other formulations. As can be seen from the NR-CE results, after incubation at 40 °C for 4 W, formulation F19 showed the greatest decrease in purity, and the other formulations showed slight decreases in purity. Therefore, formulation F20 (10 mM histidine buffer, 8% sucrose, 0.04% PS80, pH 5.5, with a protein concentration of 25 mg/mL) was selected.

**Example 8. Screening for Protein Concentration in Anti-FAP/CD40 Bispecific Antibody Formulations**

The 10 mM acetic acid-histidine pH 5.0 and pH 5.5 buffer systems were selected, and anti-FAP/CD40 bispecific antibody formulations containing 8% sucrose and 0.8 mg/mL polysorbate 80 and having different antibody concentrations were prepared:

26) 10 mM acetic acid-histidine pH 5.0, 50 mg/mL anti-FAP/CD40 bispecific antibody;
27) 10 mM acetic acid-histidine pH 5.0, 80 mg/mL anti-FAP/CD40 bispecific antibody;
28) 10 mM acetic acid-histidine pH 5.0, 100 mg/mL anti-FAP/CD40 bispecific antibody;
29) 10 mM acetic acid-histidine pH 5.5, 50 mg/mL anti-FAP/CD40 bispecific antibody. The stability of each formulation at 5 °C and 25 °C was investigated.

Table 30. The results for anti-FAP/CD40 bispecific antibody concentration screening

| Test item | Condition | F26 | F27 | F28 | F29 |
|---|---|---|---|---|---|
| Appearance | T0 | Light yellow, nearly colorless, clear, free of particles | Light yellow, nearly colorless, clear, free of particles | Light yellow, nearly colorless, clear, free of particles | Light yellow, nearly colorless, clear, free of particles |
| | 5 °C-3 M | Light yellow, nearly colorless, clear, free of particles | Light yellow, nearly colorless, clear, free of particles | Light yellow, nearly colorless, clear, free of particles | Light yellow, nearly colorless, clear, free of particles |
| | 25 °C-3 M | Light yellow, nearly colorless, clear, free of particles | Light yellow, nearly colorless, clear, free of particles | Light yellow, nearly colorless, clear, free of particles | Light yellow, nearly colorless, clear, free of particles |

(continued)

| Test item | Condition | | F26 | F27 | F28 | F29 |
|---|---|---|---|---|---|---|
| SEC | T0 | Aggregate% | 0.66 | 0.72 | 0.76 | 0.72 |
| | | Monomer% | 99.29 | 99.23 | 99.2 | 99.23 |
| | | Fragment% | 0.05 | 0.05 | 0.04 | 0.05 |
| | 5 °C-3 M | Aggregate% | 0.85 | 1.02 | 1.12 | 0.97 |
| | | Monomer% | 99.04 | 98.89 | 98.7 | 98.9 |
| | | Fragment% | 0.11 | 0.09 | 0.18 | 0.13 |
| | 25 °C -3 M | Aggregate% | 1.48 | 3.57 | 5.15 | 1.5 |
| | | Monomer% | 98.27 | 96.21 | 94.73 | 98.34 |
| | | Fragment% | 0.25 | 0.22 | 0.12 | 0.16 |
| CEX | T0 | Acidic peak% | 34.01 | 33.43 | 33.82 | 34.18 |
| | | Main peak% | 63.87 | 64.31 | 64.03 | 63.66 |
| | | Basic peak% | 2.12 | 2.26 | 2.15 | 2.15 |
| | 5 °C-3 M | Acidic peak% | 37.59 | 37.52 | 37.34 | 37.69 |
| | | Main peak% | 59.93 | 59.93 | 60.57 | 60.33 |
| | | Basic peak% | 2.47 | 2.55 | 2.09 | 1.98 |
| | 25 °C -3 M | Acidic peak% | 44.21 | 44.65 | 44.83 | 44.81 |
| | | Main peak% | 54.24 | 53.39 | 51.79 | 54.38 |
| | | Basic peak% | 1.56 | 1.96 | 3.38 | 0.8 |
| NR-CE main peak% | T0 | | 97.98 | 98.01 | 98.11 | 98.05 |
| | 5 °C-3 M | | 98.98 | 98.98 | 98.35 | 98.95 |
| | 25 °C-3 M | | 97.92 | 97.72 | 97.64 | 93.61 |

The appearance results show that: after being left to stand at 5 °C and 25 °C for 3 months, the formulations with different antibody concentrations showed no significant change and demonstrated relatively good stability.

The SEC results show that: the high-concentration formulations F27 and F28 showed a descending trend in monomer contents after being left to stand at 25 °C for 3 months, and the higher the concentration, the more significantly the monomer content decreased; formulation F28 (100 mg/mL) showed a monomer purity decrease to about 95% after being left to stand at 25 °C for 3 months. After being left to stand at 5 °C for 3 months, the formulations showed no significant change.

The CEX results show that: the formulations all showed slight decreases in main peak contents after being left to stand at 5 °C for 3 months or at 25 °C for 3 months, and the difference among the formulations was not large.

The NR-CE results show that: after being left to stand at 25 °C for 3 months, formulation F29 showed a main peak decrease by about 5%, and the other formulations showed no significant decreasing trend.

In summary, the stability was relatively good when the protein concentration was up to 100 mg/mL and the pH was 5.0.

## Example 9. Other Alternative Formulation Formulas

In addition, the present disclosure also provides anti-FAP/CD40 bispecific antibody pharmaceutical formulations of other formulation formulas, wherein the anti-FAP/CD40 bispecific antibody is Ab10-A297V3-4 of the present application, and its heavy and light chain sequences are SEQ ID NOs: 22 and 20, respectively. The formulas include, but are not limited to:

(1) 25 mg/mL FAP/CD40 bispecific antibody, 80 mg/mL sucrose, 0.6 mg/mL polysorbate 80, and 10 mM histidine-histidine hydrochloride buffer pH 5.5;
(2) 25 mg/mL FAP/CD40 bispecific antibody, 80 mg/mL sucrose, 0.6 mg/mL polysorbate 80, and 10 mM histidine-histidine hydrochloride buffer pH 5.3;

(3) 25 mg/mL FAP/CD40 bispecific antibody, 80 mg/mL sucrose, 0.6 mg/mL polysorbate 80, and 10 mM histidine-histidine hydrochloride buffer pH 5.2;

(4) 25 mg/mL FAP/CD40 bispecific antibody, 80 mg/mL sucrose, 0.6 mg/mL polysorbate 80, and 10 mM histidine-histidine hydrochloride buffer pH 5.0;

(5) 25 mg/mL FAP/CD40 bispecific antibody, 80 mg/mL sucrose, 0.4 mg/mL polysorbate 80, and 10 mM histidine-histidine hydrochloride buffer pH 5.5;

(6) 25 mg/mL FAP/CD40 bispecific antibody, 80 mg/mL sucrose, 0.4 mg/mL polysorbate 80, and 10 mM histidine-histidine hydrochloride buffer pH 5.3;

(7) 25 mg/mL FAP/CD40 bispecific antibody, 80 mg/mL sucrose, 0.4 mg/mL polysorbate 80, and 10 mM histidine-histidine hydrochloride buffer pH 5.2;

(8) 25 mg/mL FAP/CD40 bispecific antibody, 80 mg/mL sucrose, 0.4 mg/mL polysorbate 80, and 10 mM histidine-histidine hydrochloride buffer pH 5.0;

(9) 25 mg/mL FAP/CD40 bispecific antibody, 80 mg/mL sucrose, 0.4 mg/mL polysorbate 80, and 10 mM histidine-acetic acid buffer pH 5.0;

(10) 25 mg/mL FAP/CD40 bispecific antibody, 80 mg/mL sucrose, 0.4 mg/mL polysorbate 80, and 10 mM histidine-acetic acid buffer pH 5.5;

(11) 25 mg/mL FAP/CD40 bispecific antibody, 80 mg/mL sucrose, 0.4 mg/mL polysorbate 80, and 10 mM histidine-acetic acid buffer pH 5.3;

(12) 25 mg/mL FAP/CD40 bispecific antibody, 80 mg/mL sucrose, 0.4 mg/mL polysorbate 80, and 10 mM histidine-acetic acid buffer pH 5.2;

(13) 25 mg/mL FAP/CD40 bispecific antibody, 80 mg/mL sucrose, 0.6 mg/mL polysorbate 80, and 10 mM histidine-acetic acid buffer pH 5.0;

(14) 25 mg/mL FAP/CD40 bispecific antibody, 80 mg/mL sucrose, 0.6 mg/mL polysorbate 80, and 10 mM histidine-acetic acid buffer pH 5.5;

(15) 25 mg/mL FAP/CD40 bispecific antibody, 80 mg/mL sucrose, 0.6 mg/mL polysorbate 80, and 10 mM histidine-acetic acid buffer pH 5.3;

(16) 25 mg/mL FAP/CD40 bispecific antibody, 80 mg/mL sucrose, 0.6 mg/mL polysorbate 80, and 10 mM histidine-acetic acid buffer pH 5.2;

(17) 10 mg/mL FAP/CD40 bispecific antibody, 80 mg/mL sucrose, 0.4 mg/mL polysorbate 80, and 10 mM histidine-histidine hydrochloride buffer pH 5.5.

The experimental results show that the FAP/CD40 bispecific antibody formulations of the formulas described above have good stability; the formulas can be applied to the preparation of FAP/CD40 bispecific antibody medicaments.

**Claims**

1. A pharmaceutical composition, comprising a FAP/CD40-binding molecule and a buffer, wherein the FAP/CD40-binding molecule comprises a first antigen-binding domain that specifically binds to FAP and a second antigen-binding domain that specifically binds to CD40; the first antigen-binding domain that specifically binds to FAP comprises a heavy chain variable region and a light chain variable region; the heavy chain variable region comprises a HCDR1, a HCDR2, and a HCDR3, and the light chain variable region comprises a LCDR1, a LCDR2, and a LCDR3, wherein:

   1) the HCDR1, HCDR2, and HCDR3 comprise the amino acid sequences set forth in SEQ ID NOs: 3, 4, and 5, respectively; the LCDR1, LCDR2, and LCDR3 comprise the amino acid sequences set forth in SEQ ID NOs: 6, 7, and 8, respectively;
   or 2) the HCDR1, HCDR2, and HCDR3 comprise the amino acid sequences set forth in SEQ ID NOs: 3, 4, and 5, respectively; the LCDR1, LCDR2, and LCDR3 comprise the amino acid sequences set forth in SEQ ID NOs: 6, 32, and 8, respectively;
   the buffer is selected from the group consisting of an acetate buffer, a succinate buffer, a histidine salt buffer, and a phosphate buffer; preferably, the buffer is a histidine salt buffer; more preferably, the buffer is a histidine-hydrochloride buffer or a histidine-acetate buffer; most preferably, the buffer is a histidine-histidine hydrochloride buffer or a histidine-acetic acid buffer.

2. The pharmaceutical composition according to claim 1, wherein the second antigen-binding domain that specifically binds to CD40 in the FAP/CD40-binding molecule comprises at least one immunoglobulin single variable domain, and the immunoglobulin single variable domain comprises three complementarity-determining regions CDR1, CDR2, and CDR3, wherein the CDR1, CDR2, and CDR3 comprise the amino acid sequences set forth in SEQ ID NOs: 12, 13, and 14, respectively.

3. The pharmaceutical composition according to claim 1 or 2, wherein in the first antigen-binding domain that specifically binds to FAP in the FAP/CD40-binding molecule,
the heavy chain variable region comprises the amino acid sequence set forth in SEQ ID NO: 1 or an amino acid sequence having at least 90% identity thereto, and the light chain variable region comprises the amino acid sequence set forth in SEQ ID NO: 2 or an amino acid sequence having at least 90% identity thereto.

4. The pharmaceutical composition according to claim 2 or 3, wherein the immunoglobulin single variable domain in the second antigen-binding domain that specifically binds to CD40 in the FAP/CD40-binding molecule comprises any one of the amino acid sequences set forth in SEQ ID NOs: 11 and 16 to 19 or an amino acid sequence having at least 90% sequence identity to any one of SEQ ID NOs: 11 and 16 to 19.

5. The pharmaceutical composition according to any one of claims 2 to 4, wherein the second antigen-binding domain that specifically binds to CD40 in the FAP/CD40-binding molecule comprises 2, 3, 4, 5, or 6 said immunoglobulin single variable domains.

6. The pharmaceutical composition according to any one of claims 2 to 5, wherein the first antigen-binding domain that specifically binds to FAP in the FAP/CD40-binding molecule comprises a heavy chain variable region and a light chain variable region, wherein:

the immunoglobulin single variable domain of the second antigen-binding domain that specifically binds to CD40 is located at the N-terminus of the heavy chain variable region of the first antigen-binding domain that specifically binds to FAP;
the immunoglobulin single variable domain of the second antigen-binding domain that specifically binds to CD40 is located at the C-terminus of the heavy chain variable region of the first antigen-binding domain that specifically binds to FAP;
the immunoglobulin single variable domain of the second antigen-binding domain that specifically binds to CD40 is located at the N-terminus of the light chain variable region of the first antigen-binding domain that specifically binds to FAP; and/or
the immunoglobulin single variable domain of the second antigen-binding domain that specifically binds to CD40 is located at the C-terminus of the light chain variable region of the first antigen-binding domain that specifically binds to FAP.

7. The pharmaceutical composition according to any one of claims 2 to 6, wherein the immunoglobulin single variable domain of the second antigen-binding domain that specifically binds to CD40 in the FAP/CD40-binding molecule is linked, either directly or by a linker, to the first antigen-binding domain that specifically binds to FAP;

preferably, the linker is an amino acid sequence represented by $(G_4S)_x$, wherein x is independently selected from the group consisting of integers of 1-20;
more preferably, the linker is an amino acid sequence represented by $(G_4S)_2$, $(G_4S)_3$, or $(G_4S)_4$.

8. The pharmaceutical composition according to any one of claims 1 to 7, wherein the FAP/CD40-binding molecule further comprises a human immunoglobulin Fc region, wherein:

preferably, the Fc region is an Fc region of human IgG1 or IgG4;
more preferably, the human IgG1 comprises a mutation that removes or reduces Fc effector function;
most preferably, the human IgG1 comprises a mutation selected from the group consisting of N297A, D265A/N297A, L234A/L235A, L234A/L235A/P329G, L234E, L234F, L234E/L235F, and L234E/L235F/P329G.

9. The pharmaceutical composition according to any one of claims 1 to 8, wherein the first antigen-binding domain that specifically binds to FAP in the FAP/CD40-binding molecule comprises a heavy chain and a light chain, wherein:

preferably, the heavy chain is of the IgG1 or IgG4 isotype, and the light chain is of the Kappa isotype;
more preferably, the heavy chain is the amino acid sequence set forth in SEQ ID NO: 24 or an amino acid sequence having at least 90% identity thereto, and the light chain is the amino acid sequence set forth in SEQ ID NO: 25 or an amino acid sequence having at least 90% identity thereto.

10. The pharmaceutical composition according to any one of claims 1 to 9, wherein the FAP/CD40-binding molecule comprises a first polypeptide chain and a second polypeptide chain, wherein:

the first polypeptide chain comprises the amino acid sequence set forth in any one of SEQ ID NOs: 21-23 or an amino acid sequence having at least 90% identity thereto, and the second polypeptide chain comprises the amino acid sequence set forth in SEQ ID NO: 20 or an amino acid sequence having at least 90% identity thereto; preferably, the FAP/CD40-binding molecule comprises two identical first polypeptide chains and two identical second polypeptide chains.

11. The pharmaceutical composition according to any one of claims 1 to 10, wherein the FAP/CD40-binding molecule is an anti-FAP/CD40 bispecific antibody.

12. The pharmaceutical composition according to any one of claims 1 to 11, further comprising a surfactant, wherein preferably, the surfactant is one or more surfactants selected from the group consisting of a polysorbate and a poloxamer; more preferably, the surfactant is a polysorbate; most preferably, the surfactant is polysorbate 80.

13. The pharmaceutical composition according to any one of claims 1 to 12, further comprising a sugar, wherein preferably, the sugar is one or more sugars selected from the group consisting of sucrose, glucose, trehalose, and maltose; more preferably, the sugar is sucrose.

14. The pharmaceutical composition according to any one of claims 1 to 13, further comprising one or more auxiliary materials selected from the group consisting of a polyol and a metal chelating agent, wherein

preferably, the polyol is one or more polyols selected from the group consisting of glycerol, mannitol, and sorbitol; the metal chelating agent is selected from the group consisting of ethylenediaminetetraacetic acid and a pharmaceutically acceptable salt thereof; more preferably, the polyol is mannitol; the metal chelating agent is ethylenediaminetetraacetic acid.

15. The pharmaceutical composition according to any one of claims 1 to 14, wherein the pharmaceutical composition has a pH of 3.5-7, preferably 4-6.5, more preferably 4.2-6.2, and most preferably 4.5-6.

16. The pharmaceutical composition according to any one of claims 1 to 15, wherein the FAP/CD40-binding molecule is at a concentration of 0.01 mg/mL-500 mg/mL, preferably 0.1 mg/mL-400 mg/mL, more preferably 0.5 mg/mL-200 mg/mL, and most preferably 1 mg/mL-150 mg/mL.

17. The pharmaceutical composition according to any one of claims 1 to 16, wherein the buffer is at a concentration of 0.1 mM-50 mM, preferably 0.5 mM-40 mM, more preferably 1 mM-30 mM, and most preferably 5 mM-20 mM.

18. The pharmaceutical composition according to any one of claims 12 to 17, wherein the surfactant is at a concentration of 0.01 mg/mL-10 mg/mL, preferably 0.05 mg/mL-5 mg/mL, more preferably 0.1 mg/mL-3 mg/mL, and most preferably 0.2 mg/mL-2 mg/mL.

19. The pharmaceutical composition according to any one of claims 13 to 18, wherein the sugar is at a concentration of 1 mg/mL-200 mg/mL, preferably 10 mg/mL-150 mg/mL, more preferably 30 mg/mL-120 mg/mL, and most preferably 50 mg/mL-100 mg/mL.

20. The pharmaceutical composition according to any one of claims 14 to 19, wherein the auxiliary material is at a concentration of 0.001% w/v-20% w/v, preferably 0.005% w/v-15% w/v, more preferably 0.008% w/v-10% w/v, and most preferably 0.01% w/v-8% w/v.

21. A pharmaceutical composition, comprising:

the FAP/CD40-binding molecule according to any one of claims 1 to 11; a histidine salt buffer, preferably a histidine hydrochloride buffer or a histidine acetate buffer, and more preferably a histidine-histidine hydrochloride buffer or a histidine-acetic acid buffer; a polysorbate; sucrose; wherein optionally, the composition further comprises mannitol or ethylenediaminetetraacetic acid.

22. The pharmaceutical composition according to claim 21, comprising any one of the following groups 1)-4):

1) 0.01 mg/mL-500 mg/mL said FAP/CD40-binding molecule according to any one of claims 1 to 11;

0.1 mM-50 mM histidine salt buffer, preferably a histidine hydrochloride buffer or a histidine acetate buffer, and more preferably a histidine-histidine hydrochloride buffer or a histidine-acetic acid buffer;
0.01 mg/mL-10 mg/mL polysorbate;
1 mg/mL-200 mg/mL sucrose;
wherein optionally, the pharmaceutical composition further comprises 0.1% w/v-15% w/v mannitol or 0.001% w/v-5% w/v ethylenediaminetetraacetic acid;
and the pharmaceutical composition has a pH of 3.5-7;

2) 0.1 mg/mL-400 mg/mL said FAP/CD40-binding molecule according to any one of claims 1 to 11;

0.5 mM-40 mM histidine salt buffer, preferably a histidine hydrochloride buffer or a histidine acetate buffer, and more preferably a histidine-histidine hydrochloride buffer or a histidine-acetic acid buffer;
0.05 mg/mL-5 mg/mL polysorbate;
10 mg/mL-150 mg/mL sucrose;
wherein optionally, the pharmaceutical composition further comprises 0.5% w/v-12% w/v mannitol or 0.001% w/v-1% w/v ethylenediaminetetraacetic acid;
and the pharmaceutical composition has a pH of 4-6.5;

3) 0.5 mg/mL-200 mg/mL said FAP/CD40-binding molecule according to any one of claims 1 to 11;

1 mM-30 mM histidine salt buffer, preferably a histidine hydrochloride buffer or a histidine acetate buffer, and more preferably a histidine-histidine hydrochloride buffer or a histidine-acetic acid buffer;
0.1 mg/mL-3 mg/mL polysorbate;
30 mg/mL-120 mg/mL sucrose;
wherein optionally, the pharmaceutical composition further comprises 1% w/v-10% w/v mannitol or 0.005% w/v-0.1% w/v ethylenediaminetetraacetic acid;
and the pharmaceutical composition has a pH of 4.2-6.2;

4) 1 mg/mL-150 mg/mL said FAP/CD40-binding molecule according to any one of claims 1 to 11;

5 mM-20 mM histidine salt buffer, preferably a histidine hydrochloride buffer or a histidine acetate buffer, and more preferably a histidine-histidine hydrochloride buffer or a histidine-acetic acid buffer;
0.2 mg/mL-2 mg/mL polysorbate;
50 mg/mL-100 mg/mL sucrose;
wherein optionally, the pharmaceutical composition further comprises 1% w/w-10% w/w mannitol or 0.005% w/w-0.1% w/w ethylenediaminetetraacetic acid;
and the pharmaceutical composition has a pH of 4.5-6.

23. The pharmaceutical composition according to claim 21, comprising any one of the following groups 1)-21):

1) 1-150 mg/mL said FAP/CD40-binding molecule according to any one of claims 1 to 11;

about 10 mM histidine hydrochloride buffer or histidine acetate buffer, preferably a histidine-histidine hydrochloride buffer or a histidine-acetic acid buffer;
0.2 mg/mL-1 mg/mL polysorbate 80;
about 80 mg/mL sucrose;
wherein the pharmaceutical composition has a pH of 4.5-6;

2) about 5 mg/mL, about 10 mg/mL, about 20 mg/mL, about 25 mg/mL, about 50 mg/mL, about 80 mg/mL, or about 100 mg/mL, preferably about 5 mg/mL, about 10 mg/mL, about 20 mg/mL, or about 25 mg/mL, said FAP/CD40-binding molecule according to any one of claims 1 to 11;

about 10 mM histidine hydrochloride buffer, preferably a histidine-histidine hydrochloride buffer;
about 0.4 mg/mL polysorbate 80;

about 80 mg/mL sucrose;
wherein the pharmaceutical composition has a pH of about 5.5;

3) about 5 mg/mL, about 10 mg/mL, about 20 mg/mL, about 25 mg/mL, about 50 mg/mL, about 80 mg/mL, or about 100 mg/mL, preferably about 5 mg/mL, about 10 mg/mL, about 20 mg/mL, or about 25 mg/mL, said FAP/CD40-binding molecule according to any one of claims 1 to 11;

about 10 mM histidine hydrochloride buffer, preferably a histidine-histidine hydrochloride buffer;
about 0.4 mg/mL polysorbate 80;
about 80 mg/mL sucrose;
wherein the pharmaceutical composition has a pH of about 5.3;

4) about 5 mg/mL, about 10 mg/mL, about 20 mg/mL, about 25 mg/mL, about 50 mg/mL, about 80 mg/mL, or about 100 mg/mL, preferably about 5 mg/mL, about 10 mg/mL, about 20 mg/mL, or about 25 mg/mL, said FAP/CD40-binding molecule according to any one of claims 1 to 11;

about 10 mM histidine hydrochloride buffer, preferably a histidine-histidine hydrochloride buffer;
about 0.4 mg/mL polysorbate 80;
about 80 mg/mL sucrose;
wherein the pharmaceutical composition has a pH of about 5.2;

5) about 5 mg/mL, about 10 mg/mL, about 20 mg/mL, about 25 mg/mL, about 50 mg/mL, about 80 mg/mL, or about 100 mg/mL, preferably about 5 mg/mL, about 10 mg/mL, about 20 mg/mL, or about 25 mg/mL, said FAP/CD40-binding molecule according to any one of claims 1 to 11;

about 10 mM histidine hydrochloride buffer, preferably a histidine-histidine hydrochloride buffer;
about 0.4 mg/mL polysorbate 80;
about 80 mg/mL sucrose;
wherein the pharmaceutical composition has a pH of about 5;

6) about 5 mg/mL, about 10 mg/mL, about 20 mg/mL, about 25 mg/mL, about 50 mg/mL, about 80 mg/mL, or about 100 mg/mL, preferably about 5 mg/mL, about 10 mg/mL, about 20 mg/mL, or about 25 mg/mL, said FAP/CD40-binding molecule according to any one of claims 1 to 11;

about 10 mM histidine hydrochloride buffer, preferably a histidine-histidine hydrochloride buffer;
about 0.6 mg/mL polysorbate 80;
about 80 mg/mL sucrose;
wherein the pharmaceutical composition has a pH of about 5.5;

7) about 5 mg/mL, about 10 mg/mL, about 20 mg/mL, about 25 mg/mL, about 50 mg/mL, about 80 mg/mL, or about 100 mg/mL, preferably about 5 mg/mL, about 10 mg/mL, about 20 mg/mL, or about 25 mg/mL, said FAP/CD40-binding molecule according to any one of claims 1 to 11;

about 10 mM histidine hydrochloride buffer, preferably a histidine-histidine hydrochloride buffer;
about 0.6 mg/mL polysorbate 80;
about 80 mg/mL sucrose;
wherein the pharmaceutical composition has a pH of about 5.3;

8) about 5 mg/mL, about 10 mg/mL, about 20 mg/mL, about 25 mg/mL, about 50 mg/mL, about 80 mg/mL, or about 100 mg/mL, preferably about 5 mg/mL, about 10 mg/mL, about 20 mg/mL, or about 25 mg/mL, said FAP/CD40-binding molecule according to any one of claims 1 to 11;

about 10 mM histidine hydrochloride buffer, preferably a histidine-histidine hydrochloride buffer;
about 0.6 mg/mL polysorbate 80;
about 80 mg/mL sucrose;
wherein the pharmaceutical composition has a pH of about 5.2;

9) about 5 mg/mL, about 10 mg/mL, about 20 mg/mL, about 25 mg/mL, about 50 mg/mL, about 80 mg/mL, or about 100 mg/mL, preferably about 5 mg/mL, about 10 mg/mL, about 20 mg/mL, or about 25 mg/mL, said FAP/CD40-binding molecule according to any one of claims 1 to 11;

about 10 mM histidine hydrochloride buffer, preferably a histidine-histidine hydrochloride buffer;
about 0.6 mg/mL polysorbate 80;
about 80 mg/mL sucrose;
wherein the pharmaceutical composition has a pH of about 5;

10) about 5 mg/mL, about 10 mg/mL, about 20 mg/mL, about 25 mg/mL, about 50 mg/mL, about 80 mg/mL, or about 100 mg/mL said FAP/CD40-binding molecule according to any one of claims 1 to 11;

about 10 mM histidine-acetic acid buffer;
about 0.4 mg/mL polysorbate 80;
about 80 mg/mL sucrose;
wherein the pharmaceutical composition has a pH of about 5;

11) about 5 mg/mL, about 10 mg/mL, about 20 mg/mL, about 25 mg/mL, about 50 mg/mL, about 80 mg/mL, or about 100 mg/mL said FAP/CD40-binding molecule according to any one of claims 1 to 11;

about 10 mM histidine-acetic acid buffer;
about 0.4 mg/mL polysorbate 80;
about 80 mg/mL sucrose;
wherein the pharmaceutical composition has a pH of about 5.2;

12) about 5 mg/mL, about 10 mg/mL, about 20 mg/mL, about 25 mg/mL, about 50 mg/mL, about 80 mg/mL, or about 100 mg/mL said FAP/CD40-binding molecule according to any one of claims 1 to 11;

about 10 mM histidine-acetic acid buffer;
about 0.4 mg/mL polysorbate 80;
about 80 mg/mL sucrose;
wherein the pharmaceutical composition has a pH of about 5.3;

13) about 5 mg/mL, about 10 mg/mL, about 20 mg/mL, about 25 mg/mL, about 50 mg/mL, about 80 mg/mL, or about 100 mg/mL said FAP/CD40-binding molecule according to any one of claims 1 to 11;

about 10 mM histidine-acetic acid buffer;
about 0.4 mg/mL polysorbate 80;
about 80 mg/mL sucrose;
wherein the pharmaceutical composition has a pH of about 5.5;

14) about 5 mg/mL, about 10 mg/mL, about 20 mg/mL, about 25 mg/mL, about 50 mg/mL, about 80 mg/mL, or about 100 mg/mL said FAP/CD40-binding molecule according to any one of claims 1 to 11;

about 10 mM histidine-acetic acid buffer;
about 0.6 mg/mL polysorbate 80;
about 80 mg/mL sucrose;
wherein the pharmaceutical composition has a pH of about 5;

15) about 5 mg/mL, about 10 mg/mL, about 20 mg/mL, about 25 mg/mL, about 50 mg/mL, about 80 mg/mL, or about 100 mg/mL said FAP/CD40-binding molecule according to any one of claims 1 to 11;

about 10 mM histidine-acetic acid buffer;
about 0.6 mg/mL polysorbate 80;
about 80 mg/mL sucrose;
wherein the pharmaceutical composition has a pH of about 5.2;

16) about 5 mg/mL, about 10 mg/mL, about 20 mg/mL, about 25 mg/mL, about 50 mg/mL, about 80 mg/mL, or about 100 mg/mL said FAP/CD40-binding molecule according to any one of claims 1 to 11;

about 10 mM histidine-acetic acid buffer;
about 0.6 mg/mL polysorbate 80;
about 80 mg/mL sucrose;
wherein the pharmaceutical composition has a pH of about 5.3;

17) about 5 mg/mL, about 10 mg/mL, about 20 mg/mL, about 25 mg/mL, about 50 mg/mL, about 80 mg/mL, or about 100 mg/mL said FAP/CD40-binding molecule according to any one of claims 1 to 11;

about 10 mM histidine-acetic acid buffer;
about 0.6 mg/mL polysorbate 80;
about 80 mg/mL sucrose;
wherein the pharmaceutical composition has a pH of about 5.5;

18) about 5 mg/mL, about 10 mg/mL, about 20 mg/mL, about 25 mg/mL, about 50 mg/mL, about 80 mg/mL, or about 100 mg/mL said FAP/CD40-binding molecule according to any one of claims 1 to 11;

about 10 mM histidine-acetic acid buffer;
about 0.8 mg/mL polysorbate 80;
about 80 mg/mL sucrose;
wherein the pharmaceutical composition has a pH of about 5;

19) about 5 mg/mL, about 10 mg/mL, about 20 mg/mL, about 25 mg/mL, about 50 mg/mL, about 80 mg/mL, or about 100 mg/mL said FAP/CD40-binding molecule according to any one of claims 1 to 11;

about 10 mM histidine-acetic acid buffer;
about 0.8 mg/mL polysorbate 80;
about 80 mg/mL sucrose;
wherein the pharmaceutical composition has a pH of about 5.2;

20) about 5 mg/mL, about 10 mg/mL, about 20 mg/mL, about 25 mg/mL, about 50 mg/mL, about 80 mg/mL, or about 100 mg/mL said FAP/CD40-binding molecule according to any one of claims 1 to 11;

about 10 mM histidine-acetic acid buffer;
about 0.8 mg/mL polysorbate 80;
about 80 mg/mL sucrose;
wherein the pharmaceutical composition has a pH of about 5.3;

21) about 5 mg/mL, about 10 mg/mL, about 20 mg/mL, about 25 mg/mL, about 50 mg/mL, about 80 mg/mL, or about 100 mg/mL said FAP/CD40-binding molecule according to any one of claims 1 to 11;

about 10 mM histidine-acetic acid buffer;
about 0.8 mg/mL polysorbate 80;
about 80 mg/mL sucrose;
wherein the pharmaceutical composition has a pH of about 5.5.

24. A freeze-dried formulation, wherein the freeze-dried formulation is capable of forming the pharmaceutical composition according to any one of claims 1 to 23 upon reconstitution, or the freeze-dried formulation is obtained by lyophilizing the pharmaceutical composition according to any one of claims 1 to 23.

25. A reconstituted solution, wherein the reconstituted solution is prepared by reconstituting the freeze-dried formulation according to claim 24.

26. An article of manufacture, comprising a container, wherein the container contains the pharmaceutical composition according to any one of claims 1 to 23, the freeze-dried formulation according to claim 24, or the reconstituted solution according to claim 25.

27. Use of the pharmaceutical composition according to any one of claims 1 to 23, the freeze-dried formulation according to claim 24, or the reconstituted solution according to claim 25 in the manufacture of a medicament for treating or ameliorating a disease or disorder, wherein

preferably, the disease or disorder is a tumor or cancer;
more preferably, the disease or disorder is lung cancer, prostate cancer, breast cancer, head and neck cancer, esophageal cancer, gastric cancer, colorectal cancer, bladder cancer, cervical cancer, uterine cancer, ovarian cancer, liver cancer, melanoma, renal cancer, squamous cell carcinoma, or hematological cancer.

28. A method for treating or ameliorating a tumor or cancer, comprising:

administering to a subject in need thereof a therapeutically effective amount of the pharmaceutical composition according to any one of claims 1 to 23, the freeze-dried formulation according to claim 24, or the reconstituted solution according to claim 25, wherein
preferably, the tumor or cancer is lung cancer, prostate cancer, breast cancer, head and neck cancer, esophageal cancer, gastric cancer, colorectal cancer, bladder cancer, cervical cancer, uterine cancer, ovarian cancer, liver cancer, melanoma, renal cancer, squamous cell carcinoma, or hematological cancer.

FIG. 1

FIG. 2

FIG. 3

FIG. 4

FIG. 5

FIG. 6

Bivalent-CD40
nanobody

Tetravalent-CD40
nanobody

Hexavalent-CD40
nanobody

FIG. 7

FIG. 8A

FIG. 8B

FIG. 8C

FIG. 9A

FIG. 9B

FIG. 10

FIG. 11

FIG. 12

Days after grouping (day)

FIG. 13A

FIG. 13B

FIG. 13C

FIG. 13D

FIG. 13E

FIG. 14A

FIG. 14B

FIG. 14C

FIG. 14D

FIG. 14E

## INTERNATIONAL SEARCH REPORT

| International application No. |
|---|
| **PCT/CN2024/078357** |

### A. CLASSIFICATION OF SUBJECT MATTER

C07K 16/28(2006.01)i; C07K 16/40(2006.01)i; C07K 16/46(2006.01)i; A61P 35/00(2006.01)i

According to International Patent Classification (IPC) or to both national classification and IPC

### B. FIELDS SEARCHED

Minimum documentation searched (classification system followed by classification symbols)

　　IPC: C07K, A61P

Documentation searched other than minimum documentation to the extent that such documents are included in the fields searched

Electronic data base consulted during the international search (name of data base and, where practicable, search terms used)

　　CNABS, VCN, CNKI, CNTXT, USTXT, EPTXT, PUBMED, ISI WEB OF SCIENCE, GENBANK, EMBL-EBI, STNext, 申请人/发明人, applicant/inventor, 双特异性抗体, 抗成纤维细胞活化蛋白, FAP/CD40, CD40, bispecific antibody, BiAb, 肿瘤, 癌症, tumor, cancer, SEQ ID NOs: 1-32

### C. DOCUMENTS CONSIDERED TO BE RELEVANT

| Category* | Citation of document, with indication, where appropriate, of the relevant passages | Relevant to claim No. |
|---|---|---|
| PX | WO 2023025194 A1 (JIANGSU HENGRUI PHARMACEUTICALS CO., LTD. et al.) 02 March 2023 (2023-03-02)<br>claims 1-29 | 1-28 |
| A | CN 110402255 A (F. HOFFMANN-LA ROCHE AG) 01 November 2019 (2019-11-01)<br>abstract, and claims 1-32 | 1-28 |
| A | WO 2020070041 A1 (F. HOFFMANN-LA ROCHE AG.) 09 April 2020 (2020-04-09)<br>abstract, and claims 1-34 | 1-28 |
| A | WO 2020230899 A1 (KYOWA KIRIN CO., LTD.) 19 November 2020 (2020-11-19)<br>abstract, and claims 1-32 | 1-28 |
| A | WO 2022101458 A1 (F. HOFFMANN-LA ROCHE AG.) 19 May 2022 (2022-05-19)<br>abstract, and claims 1-27 | 1-28 |
| A | SUM, E. et al. "Fibroblast Activation Protein a-Targeted CD40 Agonism Abrogates Systemic Toxicity and Enables Administration of High Doses to Induce Effective Antitumor Immunity"<br>*Clinical Cancer Research*. Vol. 27, No. (14), 15 July 2021 (2021-07-15), pp. 4036-4053<br>abstract | 1-28 |

☐ Further documents are listed in the continuation of Box C.　　☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **24 May 2024** | **29 May 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/078357** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

International application No.

**PCT/CN2024/078357**

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **28**
   because they relate to subject matter not required to be searched by this Authority, namely:

   Claim 28 relates to a method for treating a disease, which falls within the scope of PCT Rule 39.1(iv) for which a search is not performed. However, a search is still performed on the basis of the pharmaceutical use thereof.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
|---|
| **PCT/CN2024/078357** |

| Patent document cited in search report | | | Publication date (day/month/year) | Patent family member(s) | | | Publication date (day/month/year) |
|---|---|---|---|---|---|---|---|
| WO | 2023025194 | A1 | 02 March 2023 | TW | 202330600 | A | 01 August 2023 |
| | | | | CA | 3228641 | A1 | 02 March 2023 |
| | | | | AU | 2022332499 | A1 | 21 March 2024 |
| CN | 110402255 | A | 01 November 2019 | RU | 2019134352 | A | 05 May 2021 |
| | | | | RU | 2019134352 | A3 | 21 July 2021 |
| | | | | RU | 2766234 | C2 | 10 February 2022 |
| | | | | MX | 2019011907 | A | 09 January 2020 |
| | | | | IL | 269396 | A | 28 November 2019 |
| | | | | SG | 11201908787 | WA | 30 October 2019 |
| | | | | PE | 20200006 | A1 | 06 January 2020 |
| | | | | PH | 12019502294 | A1 | 06 July 2020 |
| | | | | JP | 2022017275 | A | 25 January 2022 |
| | | | | JP | 7288943 | B2 | 08 June 2023 |
| | | | | US | 2021188992 | A1 | 24 June 2021 |
| | | | | CR | 20190427 | A | 01 November 2019 |
| | | | | CL | 2019002542 | A1 | 31 January 2020 |
| | | | | US | 2018340030 | A1 | 29 November 2018 |
| | | | | JP | 2020515276 | A | 28 May 2020 |
| | | | | JP | 6997209 | B2 | 04 February 2022 |
| | | | | KR | 20190137125 | A | 10 December 2019 |
| | | | | KR | 102364599 | B1 | 21 February 2022 |
| | | | | BR | 112019017753 | A2 | 07 April 2020 |
| | | | | AU | 2018247796 | A1 | 29 August 2019 |
| | | | | WO | 2018185045 | A1 | 11 October 2018 |
| | | | | CA | 3053358 | A1 | 11 October 2018 |
| | | | | CO | 2019009432 | A2 | 18 September 2019 |
| | | | | TW | 201841939 | A | 01 December 2018 |
| | | | | TWI | 704158 | B | 11 September 2020 |
| | | | | EP | 3606956 | A1 | 12 February 2020 |
| | | | | MA | 49039 | A | 12 February 2020 |
| WO | 2020070041 | A1 | 09 April 2020 | BR | 112021005822 | A2 | 27 July 2021 |
| | | | | TW | 202028240 | A | 01 August 2020 |
| | | | | EP | 3861026 | A1 | 11 August 2021 |
| | | | | CR | 20210154 | A | 21 April 2021 |
| | | | | AU | 2019355252 | A1 | 01 April 2021 |
| | | | | US | 2020190207 | A1 | 18 June 2020 |
| | | | | US | 11242396 | B2 | 08 February 2022 |
| | | | | UA | 126188 | C2 | 25 August 2022 |
| | | | | MX | 2021003548 | A | 27 May 2021 |
| | | | | IL | 281739 | A | 31 May 2021 |
| | | | | CL | 2021000751 | A1 | 05 November 2021 |
| | | | | PE | 20211863 | A1 | 21 September 2021 |
| | | | | CO | 2021003900 | A2 | 19 April 2021 |
| | | | | CA | 3113548 | A1 | 09 April 2020 |
| | | | | SG | 11202102477 | QA | 29 April 2021 |
| | | | | AR | 116565 | A1 | 19 May 2021 |
| | | | | JP | 2023065370 | A | 12 May 2023 |
| | | | | US | 2022227878 | A1 | 21 July 2022 |
| | | | | MA | 53806 | A | 05 January 2022 |
| | | | | JP | 2022511382 | A | 31 January 2022 |

Form PCT/ISA/210 (patent family annex) (July 2022)

**INTERNATIONAL SEARCH REPORT**
**Information on patent family members**

International application No.

**PCT/CN2024/078357**

| Patent document cited in search report | | | Publication date (day/month/year) | Patent family member(s) | | | Publication date (day/month/year) |
|---|---|---|---|---|---|---|---|
| | | | | JP | 7221379 | B2 | 13 February 2023 |
| | | | | KR | 20210069675 | A | 11 June 2021 |
| WO | 2020230899 | A1 | 19 November 2020 | EP | 3970743 | A1 | 23 March 2022 |
| | | | | EP | 3970743 | A4 | 15 February 2023 |
| | | | | AU | 2020276931 | A1 | 16 December 2021 |
| | | | | CA | 3140385 | A1 | 19 November 2020 |
| | | | | US | 2022259328 | A1 | 18 August 2022 |
| | | | | JPWO | 2020230899 | A1 | 19 November 2020 |
| | | | | TW | 202108626 | A | 01 March 2021 |
| | | | | KR | 20220008820 | A | 21 January 2022 |
| WO | 2022101458 | A1 | 19 May 2022 | JP | 2023549316 | A | 24 November 2023 |
| | | | | US | 2023340154 | A1 | 26 October 2023 |
| | | | | EP | 4244254 | A1 | 20 September 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310174524
- WO 2023025194 A
- WO 9404678 A
- WO 0348731 A
- US 9266938 B2
- WO 2020108611 A1

**Non-patent literature cited in the description**

- **PYPE S et al.** *J Biol Chem.*, 16 June 2000, vol. 275 (24), 1858693
- **KEHRY M R.** *J Immunol*, 1996, vol. 156, 2345-2348
- *J. biol. chem.*, 1968, vol. 243, 3558
- **LEDBETTER et al.** *J. Immunol.*, 1987, vol. 138, 788-785
- **STAMENKOVIC et al.** *EMBO J.*, 1989, vol. 8, 1403
- **ARAN F. LABRIJN et al.** *Nature Reviews Drug Discovery*, 2019, vol. 18, 585-608
- **CHEN S1 et al.** *J Immunol Res.*, 11 February 2019, vol. 2019, 4516041
- **JOHNSON** ; **WU**. *Nucleic Acids Res.*, 2000, vol. 28, 214-8
- **CHOTHIA et al.** *J. Mol. Biol.*, 1986, vol. 196, 901-17
- **CHOTHIA et al.** *Nature*, 1989, vol. 342, 877-83
- **MARTIN et al.** *Proc Natl Acad Sci (USA)*, 1989, vol. 86, 9268-9272
- AbMTM, A Computer Program for Modeling Variable Regions of Antibodies. Oxford Molecular, Ltd.
- **SAMUDRALA et al.** Ab Initio Protein Structure Prediction Using a Combined Hierarchical Approach. *PROTEINS, Structure, Function and Genetics Suppl.*, 1999, vol. 3, 194-198
- **MACCALLUM et al.** *J. Mol. Biol.*, 1996, vol. 5, 732-45
- **MAKABE et al.** *Journal of Biological Chemistry*, 2008, vol. 283, 1156-1166
- **HAMERS-CASTERMAN C** ; **ATARHOUCH T** ; **MUYLDERMANS S** ; **ROBINSON G** ; **HAMERS C** ; **SONGA EB** ; **BENDAHMAN N** ; **HAMERS R**. Naturally occurring antibodies devoid of light chains. *Nature*, 1993, vol. 363, 446-448
- **R. VAN DER LINDEN et al.** *Journal of Immunological Methods*, 2000, vol. 240, 185-195
- **LI et al.** *J Biol Chem.*, 2012, vol. 287, 13713-13721
- **DEFFAR et al.** *African Journal of Biotechnology*, 17 June 2009, vol. 8 (12), 2645-2652
- **STAHLI et al.** *Methods in Enzymology*, 1983, vol. 9, 242-253
- **KIRKLAND et al.** *J. Immunol.*, 1986, vol. 137, 3614-3619
- **HARLOW** ; **LANE**. Antibodies: A Laboratory Manual. Cold Spring Harbor Press, 1988
- **MOREL et al.** *Molec. Immunol.*, 1988, vol. 25, 7-15
- **CHEUNG et al.** *Virology*, 1990, vol. 176, 546-552
- **MOLDENHAUER et al.** *Scand. J. Immunol.*, 1990, vol. 32, 77-82
- **CACECI et al.** *Byte*, 1984, vol. 9, 340-362
- **WONG** ; **LOHMAN**. *Proc. Natl. Acad. Sci. USA*, 1993, vol. 90, 5428-5432